# EUROPEAN PATENT SPECIFICATION

(11) **EP 1 810 971 B1**
(45) Date of publication and mention of the grant of the patent: **25.12.2013**
(21) Application number: 07009479.2
(22) Date of filing: 03.03.2004
(51) Int. Cl.: C07D 405/12, A61K 31/397, A61P 3/06

(54) **Substituted azetidinone compounds, formulations and uses thereof for the treatment of hypercholesterolemia**
Substituierte Azetidinon-Derivate, deren pharmazeutische Formulierungen und deren Verwendung zur Behandlung von Hypercholesterolemia
Composés d'azetidinone substitués, formulations et utilisations de ceux-ci pour traiter l'hypercholestérolémie

(30) Priority: 07.03.2003 US 452722 P
(43) Date of publication of application: 25.07.2007
(62) Divisional of application: 04716968.5
(73) Proprietor: Merck Sharp & Dohme Corp., Rahway, NJ 07065-0907 (US)
(72) Inventor: Burnett, Duane A., Bernardsville, NJ 07924 (US); Clader, John W., Cranford, NJ 07016 (US)
(74) Representative: Hussain, Deeba

(56) References cited:
- US-A- 5 661 145

## Description

### BACKGROUND OF THE INVENTION

### Field of the Invention

The present invention relates to substituted azetidinone compounds useful for treating vascular and lipidemic conditions, and formulations and processes related thereto.

Atherosclerotic coronary heart disease (CHD) represents the major cause for death and vascular morbidity in the western world. Risk factors for atherosclerotic coronary heart disease include hypertension, diabetes mellitus, family history, male gender, cigarette smoke and high serum cholesterol. A total cholesterol level in excess of 225-250 mg/dl is associated with significant elevation of risk of CHD. The newly revised NCEP ATP III low density lipoprotein (LDL-C) goal for patients with CHD or CHD risk equivalent is <100 mg/dL (2.59 mmol/L), for individuals with two or more risk factors is <130 mg/dL (3.37 mmol/L) and for individuals with fewer than two risk factors is <160 mg/dL (4.14 mmol/L).

The regulation of whole-body cholesterol homeostasis in mammals and animals involves the regulation of dietary cholesterol and modulation of cholesterol biosynthesis, bile acid biosynthesis and the catabolism of the cholesterol-containing plasma lipoproteins. The liver is the major organ responsible for cholesterol biosynthesis and catabolism and, for this reason, it is a prime determinant of plasma cholesterol levels. The liver is the site of synthesis and secretion of very low density lipoproteins (VLDL) which are subsequently metabolized to low density lipoproteins (LDL) in the circulation. LDL are the predominant cholesterol-carrying lipoproteins in the plasma and an increase in their concentration is correlated with increased atherosclerosis. When intestinal cholesterol absorption is reduced, by whatever means, less cholesterol is delivered to the liver. The consequence of this action is decreased hepatic lipoprotein (VLDL) production and an increase in the hepatic clearance of plasma cholesterol, mostly as LDL. Thus, the net effect of inhibiting intestinal cholesterol absorption is a decrease in plasma cholesterol levels and progression of atherosclerotic lesion formation.

U.S. Patents Nos. 5,767,115, 5,624,920, 5,668,990, 5,656,624 and 5,688,787, respectively, disclose hydroxy-substituted azetidinone compounds and substituted β -lactam compounds useful for lowering cholesterol and/or in inhibiting the formation of cholesterol-containing lesions in mammalian arterial walls. U.S. Patent No. 5,756,470, U.S. Patent Application No. 2002/0137690, U.S. Patent Application No. 2002/0137689 and PCT Patent Application No. WO 2002/066464 disclose sugar-substituted azetidinones and amino acid substituted azetidinones useful for preventing or treating atherosclerosis and reducing plasma cholesterol levels.

U.S. Patents Nos. 5,846,966 and 5,661,145, respectively, disclose treatments for inhibiting atherosclerosis and reducing plasma cholesterol levels using such hydroxy-substituted azetidinone compounds or substituted β-lactam compounds in combination with HMG CoA reductase inhibitor compounds, which act by blocking hydroxymethylglutaryl coenzyme A (HMG-CoA) reductase (the rate-limiting enzyme in hepatic cholesterol synthesis).

Despite recent improvements in the treatment of vascular disease, there remains a need for improved compounds and compositions for treating hyperlipidaemia, atherosclerosis and other vascular conditions that provide more efficient delivery of treatment.

### SUMMARY OF THE INVENTION

Reference is made to a compound represented by the structural formula (1): 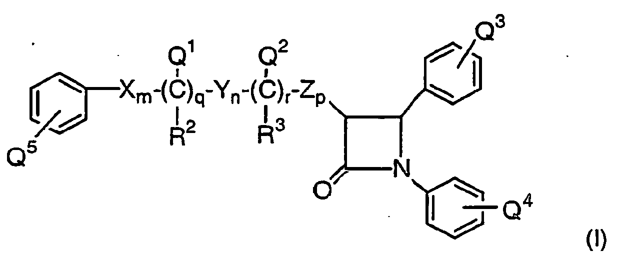 or pharmaceutically acceptable isomers, salts, solvates or esters of the compound of Formula (I),
wherein in Formula (I) above:
X, Y and Z can be the same or different and each is independently selected from the group consisting of -CH₂-, -CH(alkyl)- and -C(alkyl)₂-;
Q¹ and Q² can be the same or different and each is independently selected from the group consisting of H, -(C₀-C₃₀ alkylene)-G, -OR⁶, -OC(O)R⁶, -OC(O)OR⁹, -OC(O)NR⁶R⁷, and -L-M;
Q³ is 1 to 5 substituents independently selected from the group consisting of alkyl, alkenyl, alkynyl, -(C₀-C₃₀ alkylene)-G, -(C₀-C₁₀ alkylene)-OR⁶, -(C₀-C₁₀ alkylene)-C(O)R⁶, -(C₀-C₁₀ alkylene)-C(O)OR⁶, -(C₀-C₁₀ alkylene)-OC(O)R⁶, -(C₀-C₁₀ alkylene)-OC(O)OR⁹, -CH=CH-C(O)R⁶, -CH=CH-C(O)OR⁶, -C≡C-C(O)OR⁶, -C≡C-C(O)R⁶, -O-(C₁-C₁₀ alkylene)-OR⁶, -O-(C₁-C₁₀ alkylene)-C(O)R⁶, -O-(C₁-C₁₀ alkylene)-C(O)OR⁶, -CN, -O-(C₁-C₁₀ alkylene)-C(O)NR⁶R⁷, -O-(C₀-C₁₀ alkylene)-C(O)NR⁶NR⁷C(O)OR⁶, -O-(C₁-C₁₀ alkylene)-C(O)(aryl)-N-N=N⁻, -OC(O)-(C₁-C₁₀ alkylene)-C(O)OR⁶, -(C₀-C₁₀ alkylene)-C(O)NR⁶R⁷ -(C₀-C₁₀ alkylene)-OC(O)NR⁶R⁷, -NO₂, -(C₀-C₁₀ alkylene)-NR⁶R⁷, -O-(C₂-C₁₀ alkylene)-NR⁶R⁷, -NR⁶C(O)R⁷, -NR⁶C(O)OR⁹, -NR⁶C(O)NR⁷R⁸, -NR⁶S(O)₀₋₂R⁹, -N(S(O)₀₋₂R⁹)₂, -CHNOR⁶, -C(O)NR⁶R⁷, -C(O)NR⁶NR⁶R⁷, -S(O)₀₋₂NR⁶R⁷, -S(O)₀₋₂R⁹, -O-C(O)-(C₁-C₁₀ alkylene)-C(O)NR⁶R⁷, -OC(O)-(C₁-C₁₀ alkylene)-NR⁶C(O)O-(alkylaryl), -P(O)(OR¹⁰)₂, -(C₁-C₁₀ alkylene)-OSi(alkyl)₃ , -CF₃, -OCF₃, halo, alkoxyalkoxy, alkoxyalkoxyalkoxy, alkoxycarbonylalkoxy, alkoxyarylalkoxy, alkoxyiminoalkyl, alkyldioyl, allyloxy, aryl, arylalkyl, aryloxy, arylalkoxy, aroyl, aroyloxy, aroylaroyloxy, arylalkoxycarbonyl, benzoylbenzoyloxy, heteroaryl, heteroarylalkyl, heteroarylalkoxy, dioxolanyl, heterocyclyl, heterocyclylalkyl, heterocyclylcarbonyl, heterocyclylcarbonylalkoxy and -L-M;
Q⁴ is 1 to 5 substituents independently selected from the group consisting of alkyl, alkenyl, alkynyl, -(C₀-C₃₀ alkylene)-G, -(C₀-C₁₀ alkylene)-OR⁶, -(C₀-C₁₀ alkylene)-C(O)R⁶, -(C₀-C₁₀ alkylene)-C(O)OR⁶, -(C₀-C₁₀ alkylene)-OC(O)R⁶, -(C₀-C₁₀ alkylene)-OC(O)OR⁹, -CH=CH-C(O)R⁶, -CH=CH-C(O)OR⁶, -C≡C-C(O)OR⁶, -C≡C-C(O)R⁶, -O-(C₁-C₁₀ alkylene)-OR⁶, -O-(C₁-C₁₀ alkylene)-C(O)R⁶, -O-(C₁-C₁₀ alkylene)-C(O)OR⁶, -CN, -O-(C₁-C₁₀ alkylene)-C(O)NR⁶R⁷, -O-(C₀-C₁₀ alkylene)-C(O)NR⁶NR⁷C(O)OR⁶, -O-(C₁-C₁₀ alkylene)-C(O)(aryl)-N-N=N⁻, -OC(O)-(C₁-C₁₀alkylene)-C(O)OR⁶, -(C₀-C₁₀ alkylene)-C(O)NR⁶R⁷, -(C₀-C₁₀ alkylene)-OC(O)NR⁶R⁷, -NO₂, -(C₀-C₁₀ alkylene)-NR⁶R⁷, -O-(C₂-C₁₀ alkylene)-NR⁶R⁷, -NR⁶C(O)R⁷, -NR⁶C(O)OR⁹, -NR⁶C(O)NR⁷R⁸, -NR⁶S(O)₀₋₂R⁹, -N(S(O)₀₋₂R⁹)₂, -CHNOR⁶, -C(O)NR⁶R⁷, -C(O)NR⁶NR⁶R⁷, -S(O)₀₋₂NR⁶R⁷, -S(O)₀₋₂R⁹, -O-C(O)-(C₁-C₁₀ alkylene)-C(O)NR⁶R⁷, -OC(O)-(C₁-C₁₀ alkylene)-NR⁶C(O)O-(alkylaryl), -P(O)(OR¹⁰)₂, -(C₁-C₁₀ alkylene)-OSi(alkyl)₃, -CF₃, -OCF₃, halo, alkoxyalkoxy, alkoxyalkoxyalkoxy, alkoxycarbonylalkoxy, alkoxyarylalkoxy, alkoxyiminoalkyl, alkyldioyl, allyloxy, aryl, arylalkyl, aryloxy, arylalkoxy, aroyl, aroyloxy, aroylaroyloxy, arylalkoxycarbonyl, benzoylbenzoyloxy, heteroaryl, heteroarylalkyl, heteroarylalkoxy, dioxolanyl, heterocyclyl, heterocyclylalkyl, heterocyclylcarbonyl, heterocyclylcarbonylalkoxy and -L-M;
Q⁵ is 1 to 5 substituents independently selected from the group consisting of alkyl, alkenyl, alkynyl, -(C₀-C₃₀ alkylene)-G, -(C₀-C₁₀ alkylene)-OR⁶, -(C₀-C₁₀ alkylene)-C(O)R ⁶, -(C₀-C₁₀ alkylene)-C(O)OR⁶, -(C₀-C₁₀ alkylene)-OC(O)R⁶, -(C₀-C₁₀ alkylene)-OC(O)OR⁹, -CH=CH-C(O)R⁶ ,-CH=CH-C(O)OR⁶ -C≡C-C(O)OR⁶ -C≡C-C(O)R⁶ -O-(C₁-C₁₀ alkylene)-OR⁶, -O-(C₁-C₁₀ alkylene)-C(O)R⁶ , -O-(C₁-C₁₀ alkylene)-C(O)OR⁶, -CN, -O-(C₁-C₁₀ alkylene)-C(O)NR⁶R⁷,-O-(C₀-C₁₀ alkylene)-C(O)NR⁶NR⁷C(O)OR⁶, -O-(C₁-C₁₀ alkylene)-C(O)(aryl)-N-N=N⁻, -OC(O)-(C₁-C₁₀ alkylene)-C(O)OR⁶, -(C₀-C₁₀ alkylene)-C(O)NR⁶R⁷, -(C₀-C₁₀ alkylene)-OC(O)NR⁶R⁷, -NO₂, -(C₀-C₁₀ alkylene)-NR⁶R⁷, -O-(C₂-C₁₀ alkylene)-NR⁶R⁷, -NR⁶C(O)R⁷, -NR⁶C(O)OR⁹, -NR⁶C(O)NR⁷R⁸, NR⁶S(O)₀₋₂R⁹, -N(S(O)₀₋₂R⁹)₂, -CHNOR⁶, -C(O)NR⁶R⁷, -C(O)NR⁶NR⁶R⁷, -S(O)₀₋₂NR⁶R⁷, -S(O)₀₋₂R⁹, -O-C(O)-(C₁-C₁₀ alkylene)-C(O)NR⁶R⁷, -OC(O)-(C₁-C₁₀ alkylene)-NR⁶C(O)O-(alkylaryl), -P(O)(OR¹⁰)₂, -(C₁-C₁₀ alkylene)-OSi(alkyl)₃, -CF₃, -OCF₃, halo, alkoxyalkoxy, alkoxyalkoxyalkoxy, alkoxycarbonylalkoxy, alkoxyarylalkoxy, alkoxyiminoalkyl, alkyldioyl, allyloxy, aryl, arylalkyl, aryloxy, arylalkoxy, aroyl, aroyloxy, aroylaroyloxy, arylalkoxycarbonyl, benzoylbenzoyloxy, heteroaryl, heteroarylalkyl, heteroarylalkoxy, dioxolanyl, heterocyclyl, heterocyclylalkyl, heterocyclylcarbonyl, heterocyclylcarbonylalkoxy and -L-M;
wherein optionally one or more carbon atoms of the -(C₀-C₃₀ alkylene)- radical of Q¹, Q², Q³, Q⁴ and Q⁵ is independently replaced by -O-, -C(O)-, -CH=CH-, -C≡C-, -N(alkyl)-, -N(alkylaryl)- or -NH-;
G is selected from the group consisting of a sugar residue, disugar residue, trisugar residue, tetrasugar residue, sugar acid, amino sugar, amino acid residue, oligopeptide residue comprising 2 to 9 amino acids, trialkylammoniumalkyl radical and -S(O)₂-OH, wherein optionally the sugar residue, disugar residue, trisugar residue, tetrasugar residue, sugar acid, amino sugar, amino acid residue or oligopeptide residue of G is substituted with -L-M;
L is selected from the group consisting of 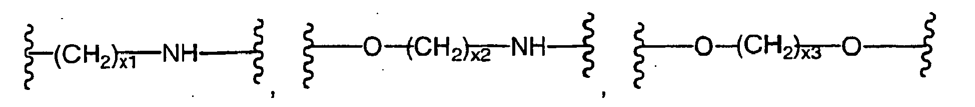 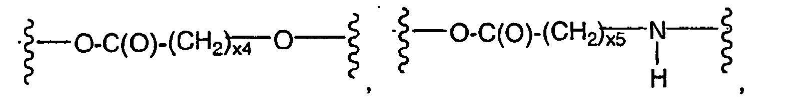 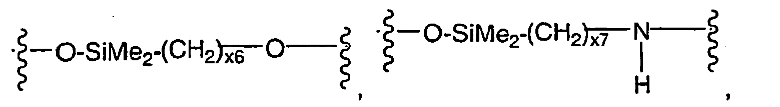 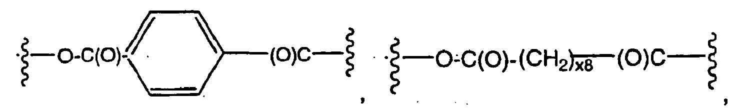 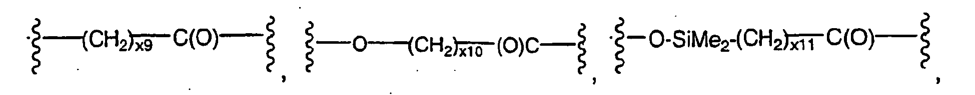 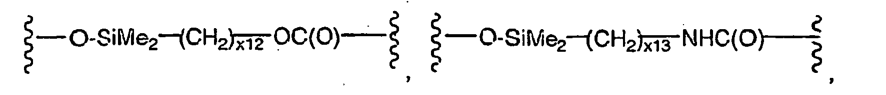 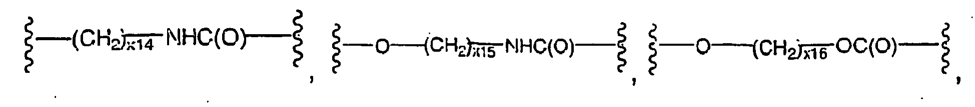 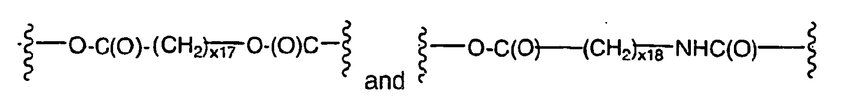 wherein Me is methyl;
M is selected from the group of moieties consisting of 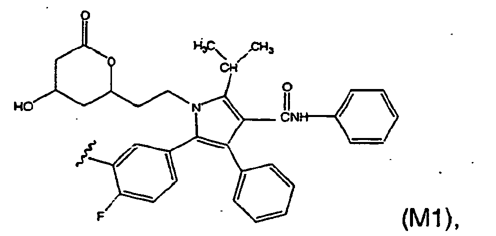 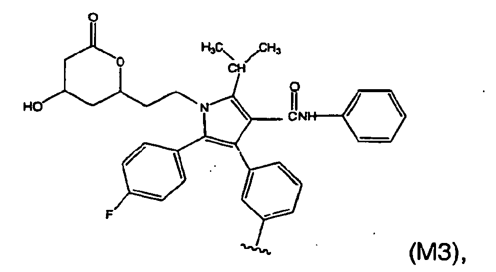 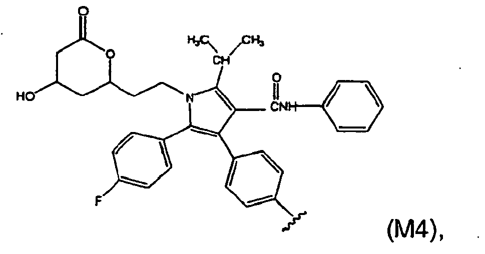 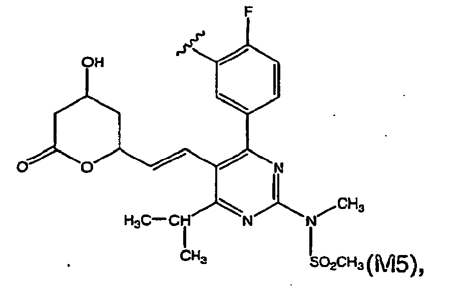 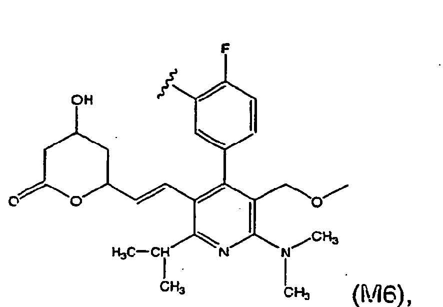 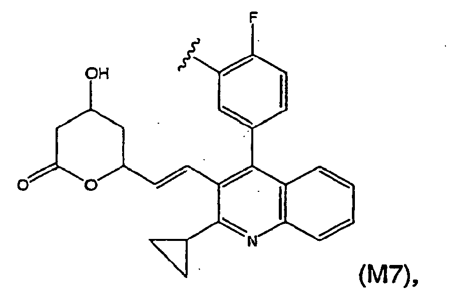 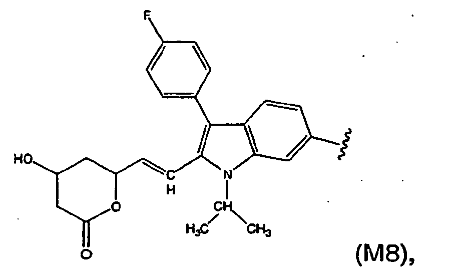 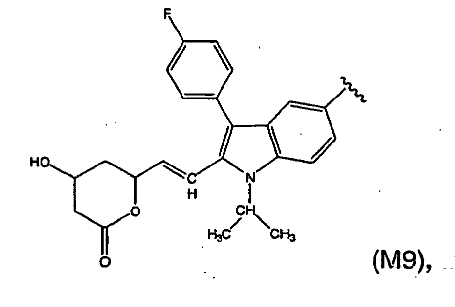 and 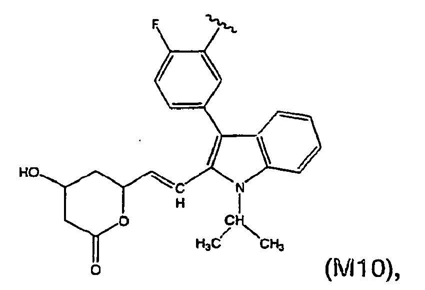 pharmaceutically ac:cepta.ble salts of the moieties (M1) and (M3) to (M10) and free acids of the moieties (M1) and (M3) to (M10);
R² and R³ can be the same or different and each is independently selected from the group consisting of hydrogen, alkyl and aryl;
R⁶, R⁷ and R⁸ can be the same or different and each is independently selected from the group consisting of hydrogen, alkyl, aryl and arylalkyl; and
each R⁹ is independently alkyl, aryl or arylalkyl.
each R¹⁰ is independently H or alkyl;
q is 0 or 1;
r is 0 or 1;
m, n and p are independently selected from 0, 1, 2, 3 or 4; provided that at least one of q and r is 1, and the sum of m, n, p, q and r is 1, 2, 3, 4, 5 or 6; and provided that when p is 0 and r is 1, the sum of m, q and n is 1, 2, 3, 4 or 5;
x1 is 1 to 10;
x2 is 1 to 10;
x3 is 1 to 10;
x4 is 1 to 10;
x5 is 1 to 10;
x6 is 1 to 10;
x7 is 1 to 10;
x8 is 1 to 10;
x9 is 1 to 10;
x10 is 1 to 10;
x11 is 1 to 10;
x12 is 1 to 10;
x13 is 1 to 10;
x14 is 1 to 10;
x15 is 1 to 10; and
x16 is 1 to 10;
x17 is 1 to 10; and
x18 is 1 to 10;
with the proviso that at least one of Q¹, Q², Q³, Q⁴ and Q⁵ is -L-M or the sugar residue, disugar residue, trisugar residue, tetrasugar residue, sugar acid, amino sugar, amino acid residue or oligopeptide residue of G is substituted with -L-M.

In one embodiment, the present invention provides a compound represented by the structural formula (IA): 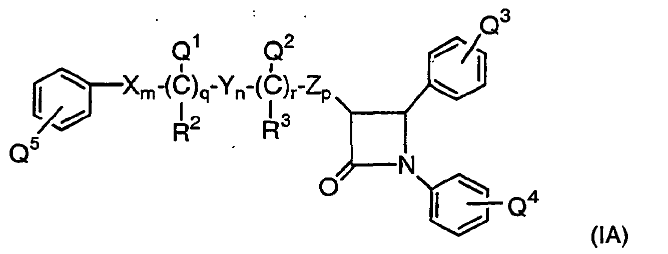 or pharmaceutically acceptable isomers, salts, solvates or esters of the compound of Formula (IA),
wherein in Formula (IA) above:
X, Y and Z can be the same or different and each is independently selected from the group consisting of -CH₂-, -CH(alkyl)- and -C(alkyl)₂-;
Q¹ and Q² can be the same or different and each is independently selected from the group consisting of H, -(C₀-C₃₀ alkylene)-G, -OR⁶, -OC(O)R⁶, -OC(O)OR⁹, -OC(O)NR⁶R⁷, and -L-M;
Q³ is 1 to 5 substituents independently selected from the group consisting of alkyl, alkenyl, alkynyl, -(C₀-C₃₀ alkylene)-G, -(C₀-C₁₀ alkylene)-OR⁶, -(C₀-C₁₀ alkylene)-C(O)R⁶, -(C₀-C₁₀ alkylene)-C(O)OR⁶, -(C₀-C₁₀ alkylene)-OC(O)R⁶, -(C₀-C₁₀ alkylene)-OC(O)OR ⁹, -CH=CH-C(O)R⁶, -CH=CH-C(O)OR⁶, -O≡C-C(O)OR⁶ -C≡C-C(O)R⁶, -O-(C₁-C₁₀ alkylene)-OR⁶, -O-(C₁-C₁₀ alkylene)-C(O)R⁶, -O-(C₁-C₁₀ alkylene)-C(O)OR⁶, -CN, -O-(C₁-C₁₀ alkylene)-C(O)NR⁶R⁷, -O-(C₀-C₁₀ alkylene)-C(O)NR⁶NR⁷C(O)OR⁶, -O-(C₁-C₁₀ alkylene)-C(O)(aryl)-N-N=N⁻, -OC(O)-(C₁-C₁₀ alkylene)-C(O)OR⁶, -(C₀-C₁₀ alkylene)-C(O)NR⁶NR⁷, -(C₀-C₁₀ alkylene)-OC(O)NR⁶R⁷, -NO₂, -(C₀-C₁₀ alkylene)-NR⁶R⁷, -O-(C₂-C₁₀ alkylene)-NR⁶R⁷, -NR⁶C(O)R⁷, -NR⁶C(O)OR⁹, -NR⁶C(O)NR⁷R⁸, -NR⁶S(O)₀₋₂R⁹, -N(S(O)₀₋₂R⁹)₂, -CHNOR⁶, -C(O)NR⁶R⁷, -C(O)NR⁶NR⁶R⁷, -S(O)₀₋₂NR⁶R⁷, -S(O)₀₋₂R⁹, -O-C(O)-(C₁-C₁₀ alkylene)-C(O)NR⁶R⁷, -OC(O)-(C₁-C₁₀ alkylene)-NR⁶C(O)O-(alkylaryl), -P(O)(OR¹⁰)₂, -(C₁-C₁₀ alkylene)-OSi(alkyl)₃, -CF₃, -OCF₃, halo, alkoxyalkoxy, alkoxyalkoxyalkoxy, alkoxycarbonylalkoxy, alkoxyarylalkoxy, alkoxyiminoalkyl, alkyldioyl, allyloxy, aryl, arylalkyl, aryloxy, arylalkoxy, aroyl, aroyloxy, aroylaroyloxy, arylalkoxycarbonyl, benzoylbenzoyloxy, heteroaryl, heteroarylalkyl, heteroarylalkoxy, dioxolanyl, heterocyclyl, heterocyclylalkyl, heterocyclylcarbonyl, heterocyclylcarbonylalkoxy and -L-M;
Q⁴ is 1 to 5 substituents independently selected from the group consisting of alkyl, alkenyl, alkynyl, -(C₀-C₃₀ alkylene)-G, -(C₀-C₁₀ alkylene)-OR⁶, -(C₀-C₁₀ alkylene)-C(O)R⁶, -(C₀-C₁₀ alkylene)-C(O)OR⁶, -(C₀-C₁₀ alkylene)-OC(O)R⁶, -(C₀-C₁₀ alkylene)-OC(O)OR⁹, -CH=CH-C(O)R⁶, -CH=CH-C(O)OR⁶, -C≡C-C(O)OR⁶, -C≡C-C(O)R⁶, -O-(C₁-C₁₀ alkylene)-OR⁶, -O-(C₁-G₁₀ alkylene)-C(O)R⁶, -O-(C₁-C₁₀ alkylene)-C(O)OR⁶, -CN, -O-(C₁-C₁₀ alkylene)-C(O)NR⁶R⁷, -O-(C₀-C₁₀ alkylene)-C(O)NR⁶NR⁷C(O)OR⁶, -O-(C₁-C₁₀ alkylene)-C(O)(aryl)-N-N=N⁻, -OC(O)-(C₁-C₁₀ alkylene)-C(O)OR⁶, -(C₀-C₁₀ alkylene)-C(O)NR⁶R⁷, -(C₀-C₁₀ alkylene)-OC(O)NR⁶R⁷, -NO₂, -(C₀-C₁₀ alkylene)-NR⁶R⁷, -O-(C₂-C₁₀ alkylene)-NR⁶R⁷, -NR⁶C(O)R⁷, -NR⁶C(O)OR⁹, -NR⁶C(O)NR⁷R⁸, NR⁶S(O)₀₋₂R⁹, -N(S(O)₀₋₂R⁹)₂, -CHNOR⁶, -C(O)NR⁶R⁷, -C(O)NR⁶NR⁶R⁷, -S(O)₀₋₂NR⁶R⁷, -S(O)₀₋₂R⁹, -O-C(O)-(C₁-C₁₀ alkylene)-C(O)NR⁶R⁷, -OC(O)-(C₁-C₁₀ alkylene)-NR⁶C(O)O-(alkylaryl), -P(O)(OR¹⁰)₂, -(C₁-C₁₀ alkylene)-OSi(alkyl)₃ , -CF₃, -OCF₃, halo, alkoxyalkoxy, alkoxyalkoxyalkoxy, alkoxycarbonylalkoxy, alkoxyarylalkoxy, alkoxyiminoalkyl, alkyldioyl, allyloxy, aryl, arylalkyl, aryloxy, arylalkoxy, aroyl, aroyloxy, aroylaroyloxy, arylalkoxycarbonyl, benzoylbenzoyloxy, heteroaryl, heteroarylalkyl, heteroarylalkoxy, dioxolanyl, heterocyclyl), heterocyclylalkyl, heterocyclylcarbonyl, heterocyclylcarbonylalkoxy and -L-M;
Q⁵ is 1 to 5 substituents independently selected from the group consisting of alkyl, alkenyl, alkynyl, -(C₀-C₃₀ allcylene)-G, -(C₀-C₁₀ alkylene)-OR⁶, -(C₀-C₁₀ alkylene)-C(O)R⁶, -(C₀-C₁₀ alkylene)-C(O)OR⁶, -(C₀-C₁₀ alkylene)-OC(O)R⁶, -(C₀-C₁₀ alkylene)-OC(O)OR⁹, -CH=CH-C(O)R⁶, -CH=CH-C(O)OR⁶, -C≡C-C(O)OR⁶, -C≡C-C(O)R⁶, -O-(C₁-C₁₀ alkylene)-OR⁶, -O-(C₁-C₁₀ alkylene)-C(O)R⁶, -O-(C₁-C₁₀ alkylene)-C(O)OR⁶, -CN, -O-(C₁-C₁₀ alkylene)-C(O)NR⁶R⁷, -O-(C₀-C₁₀ alkylene)-C(O)NR⁶NR⁷C(O)OR⁶, -O-(C₁-C₁₀ alkylene)-C(O)(aryl)-N-N=N⁻, -OC(O)-(C₁-C₁₀ alkylene)-C(O)OR⁶, -(C₀-C₁₀ alkylene)-C(O)NR⁶R⁷, -(C₀-C₁₀ alkylene)-OC(O)NR⁶R⁷, -NO₂, -(C₀-C₁₀ alkylene)-NR⁶R⁷, -O-(C₂-C₁₀ alkylene)-NR⁶R⁷, -NR⁶C(O)R⁷, -NR⁶C(O)OR⁹, -NR⁶C(O)NR⁷R⁸, -NR⁶S(O)₀₋₂R⁹, -N(S(O)₀₋₂R⁹)₂, -CHNOR⁶, -C(O)NR⁶R⁷, -C(O)NR⁶NR⁶R⁷, -S(O)₀₋₂NR⁶R⁷, -S(O)₀₋₂R⁹, -O-C(O)-(C₁-C₁₀ alkylene)-C(O)NR⁶R⁷, -OC(O)-(C₁-C₁₀ alkylene)-NR⁶C(O)O-(alkylaryl), -P(O)(OR¹⁰)₂, -(C₁-C₁₀ alkylene)-OSi(alkyl)₃, -CF₃, -OCF₃, halo, alkoxyalkoxy, alkoxyalkoxyalkoxy, alkoxycarbonylalkoxy, alkoxyarylalkoxy, alkoxyiminoalkyl, alkyldioyl, allyloxy, aryl, arylalkyl, aryloxy, arylalkoxy, aroyl, aroyloxy, aroylaroyloxy, arylalkoxycarbonyl, benzoylbenzoyloxy, heteroaryl, heteroarylalkyl, heteroarylalkoxy, dioxolanyl, heterocyclyl, heterocyclylalkyl, heterocyclylcarbonyl, heterocyclylcarbonylalkoxy and -L-M;
wherein optionally one or more carbon atoms of the -(C₀-C₃₀ alkylene)- radical of Q¹, Q², Q³, Q⁴ and Q⁵ is independently replaced by -O-, -C(O)-, -CH=CH-, -C≡C-, -N(alkyl)-, -N(alkylaryl)- or-NH-;
G is selected from the group consisting of a sugar residue, disugar residue, trisugar residue, tetrasugar residue, sugar acid, amino sugar, amino acid residue, oligopeptide residue comprising 2 to 9 amino acids, trialkylammoniumalkyl radical and -S(O)₂-OH, wherein optionally the sugar residue, disugar residue, trisugar residue, tetrasugar residue, sugar acid, amino sugar, amino acid residue or oligopeptide residue of G is substituted with -L-M;
L is selected from the group consisting of 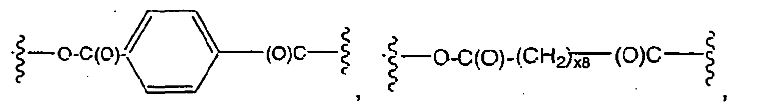 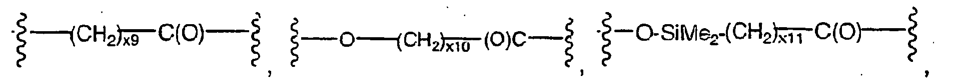 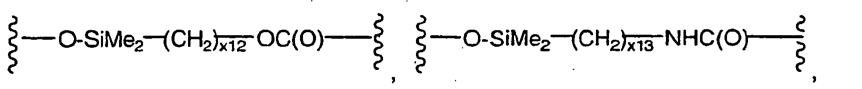 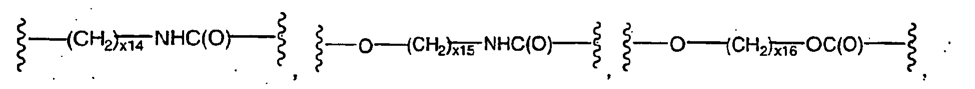 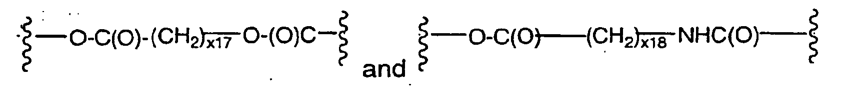 wherein Me is methyl;
M is selected from the group of moieties consisting of 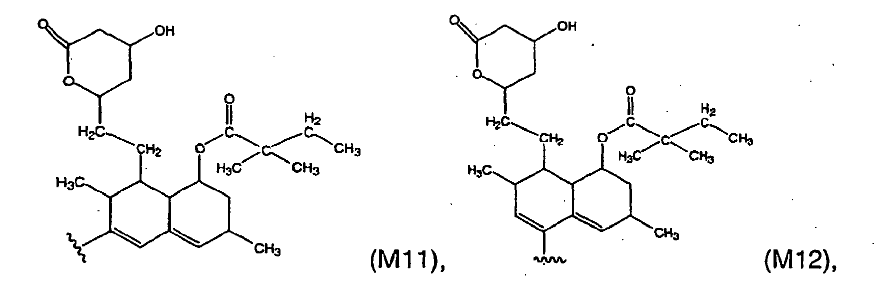 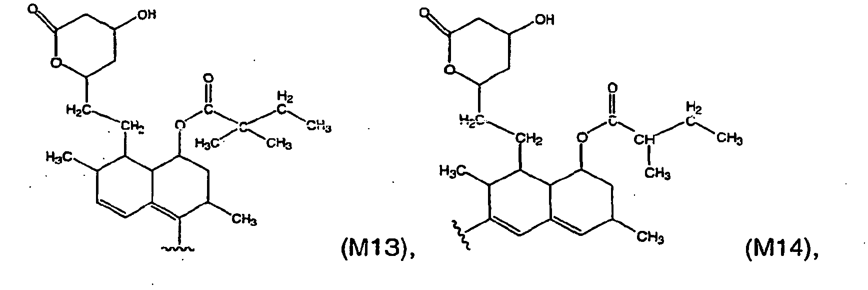 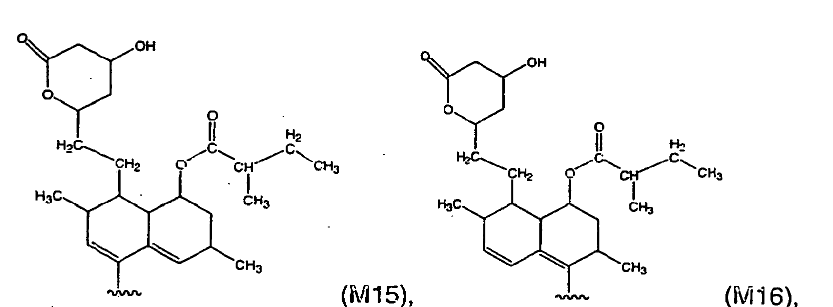 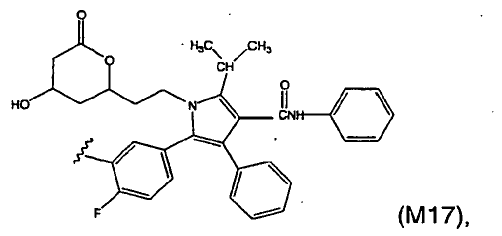 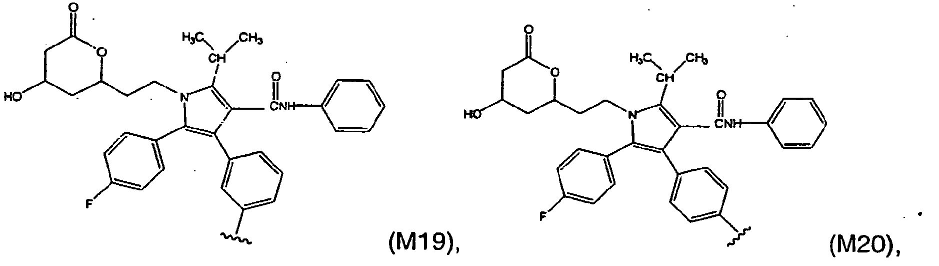 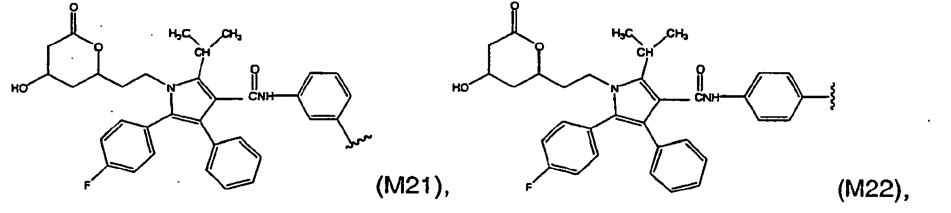 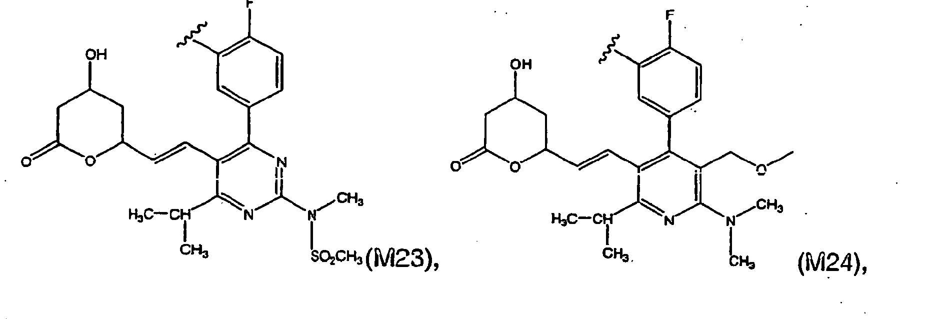 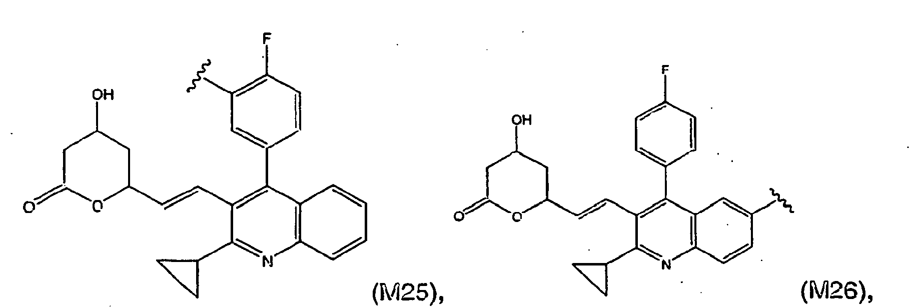 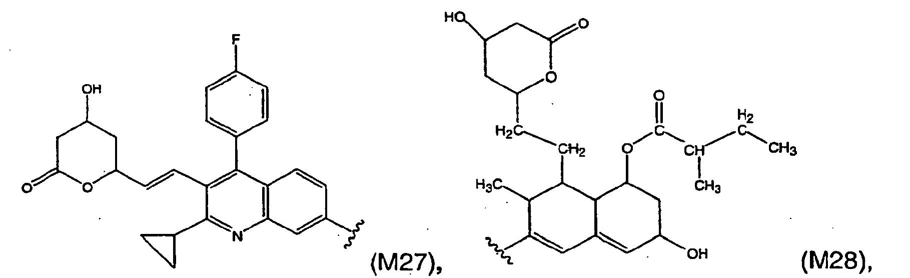 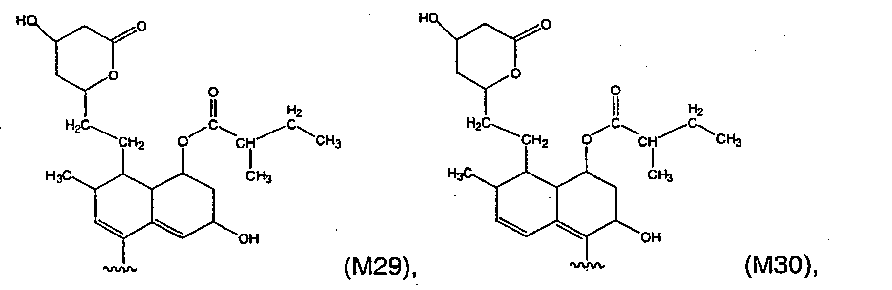 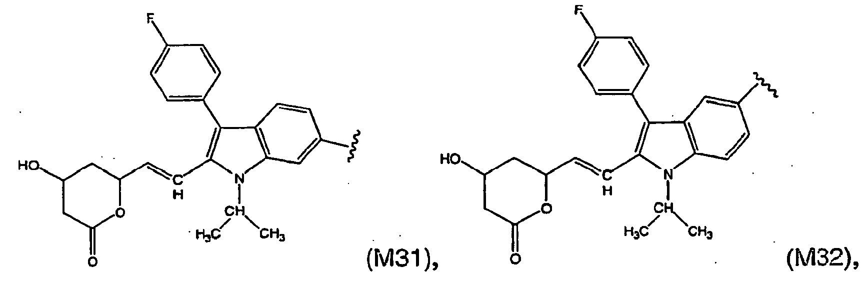 and 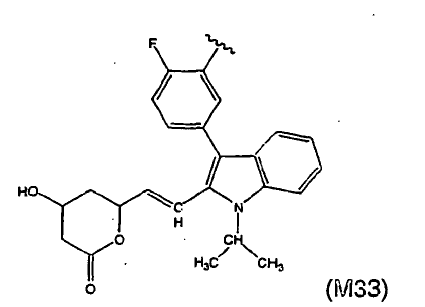 and pharmaceutical acceptable salts of moieties (M1) to (M33);
R² and R³ can be the same or different and each is independently selected from the group consisting of hydrogen, alkyl and aryl;
R⁶, R⁷ and R⁸ can be the same or different and each is independently selected from the group consisting of hydrogen, alkyl, aryl and arylalkyl; and
each R⁹ is independently alkyl, aryl or arylalkyl.
each R¹⁰ is independently H or alkyl;
q is 0 or 1;
r is 0 or 1;
m, n and p are independently selected from 0, 1, 2, 3 or 4; provided that at least one of q and r is 1, and the sum of m, n, p, q and r is 1, 2, 3, 4, 5 or 6; and provided that when p is 0 and r is 1, the sum of m, q and n is 1, 2, 3, 4 or 5;
x8 is 1 to 10;
x9 is 1 to 10;
x10 is 1 to 10;
x11 is 1 to 10;
x12 is 1 to 10;
x13 is 1 to 10;
x14 is 1 to 10;
x15 is 1 to 10; and
x16 is 1 to 10;
x17 is 1 to 10; and
x18 is 1 to 10;
with the proviso that at least one of Q¹, Q², Q³, Q⁴ and Q⁵ is -L-M or the sugar residue, disugar residue, trisugar residue, tetrasugar residue, sugar acid, amino sugar, amino acid residue or oligopeptide residue of G is substituted with -L-M.

Pharmaceutical formulations or compositions for the treatment or prevention of a vascular condition, diabetes, obesity, stroke, lowering a concentration of a sterol or stanol in plasma of a mammal, preventing demyelination or treating Alzheimer's disease and/or regulating levels of amyloid β peptides in a subject comprising a therapeutically effective amount of the above compounds and a pharmaceutically acceptable carrier also are provided.

Methods of treating or preventing a vascular condition, diabetes, obesity, stroke, lowering a concentration of a sterol or stanol in plasma of a mammal, preventing demyelination or treating Alzheimer's disease and/or regulating levels of amyloid β peptides in a subject comprising the step of administering to a subject in need of such treatment an effective amount of the above compounds of Formula (I) or (IA) also are provided.

Other than in the operating examples, or where otherwise indicated, all numbers expressing quantities of ingredients, reaction conditions, and so forth used in the specification and claims are to be understood as being modified in all instances by the term "about."

### DETAILED DESCRIPTION

In its many embodiments, the present invention provides a novel class of compounds of Formula (IA) above, processes for producing such compounds, pharmaceutical formulations or compositions comprising one or more of such compounds, methods of preparing the same, and compounds for use in the treatment, prevention, inhibition or amelioration of one or more conditions or diseases associated with vascular conditions or other conditions such as are discussed in detail below.

The compounds of Formula (IA) are capable of being metabolized in vivo to form a sterol and/or stanol absorption inhibitor compound and a sterol biosynthesis inhibitor compound. As used herein, "sterol absorption inhibitor" means a compound capable of inhibiting the absorption of one or more sterols, including but not limited to cholesterol and phytosterols (such as sitosterol, campesterol, stigmasterol and avenosterol) when administered in a therapeutically effective (sterol absorption inhibiting) amount to a subject or human. "Stanol absorption inhibitor" means a compound capable of inhibiting the absorption of one or more 5α-stanols (such as cholestanol, 5α-campestanol, 5α-sitostanol) when administered in a therapeutically effective (stanol absorption inhibiting) amount to a subject or human. The sterol or stanol absorption inhibitor can inhibit the absorption of cholesterol from the intestinal lumen into enterocytes, leading to a decrease in the delivery of intestinal sterol or stanol, respectively, to the liver. "Sterol biosynthesis inhibitor" means a compound, such as a 3-hydroxy-3-methylglutaryl coenzyme A (HMG CoA) reductase inhibitor, that blocks hydroxymethylglutaryl coenzyme A (HMG-CoA) reductase, which is the rate-limiting enzyme in hepatic cholesterol synthesis.

In an alternative embodiment, compounds of Formula (IA) can have dual functionality, i.e., can exhibit sterol and/or stanol absorption inhibiting properties and also block hydroxymethylglutaryl coenzyme A (HMG-GoA) reductase.

Referring now to Formula (IA), in one embodiment of the present invention, X, Y and Z are each -CH₂-.

The sum of m, n, p, q and r is preferably 2, 3 or 4, more preferably 3. Also preferred are compounds of Formula (IA) in which p, q and n are each zero, r is 1 and m is 2 or 3.

in one embodiment, m, n and r are each zero, q is 1, p is 2, and Z is -CH₂-. Also preferred are compounds wherein m, n and r are each zero, q is 1, p is 2, and Z is -CH₂-, Q¹ is -OR⁶, wherein R⁶ is hydrogen and Q⁵ is fluorine.

R² and R³ are each preferably hydrogen.

In one embodiment, Q¹ and Q² can be -OR⁶ wherein R⁶ is hydrogen, or a group readily metabolizable to a hydroxyl (such as -O(CO)R ⁶, -O(CO)OR⁹ and -O(CO)NR⁶R⁷, defined above).

In another embodiment Q⁴ is halo or -OR⁶.

In another embodiment, Q¹ is -OR⁶ wherein R⁶ is H.

In yet another embodiment, Q¹ is -L-M.

In another embodiment, Q² is -L-M.

In another embodiment, Q³ is -L-M.

In another embodiment, Q⁴ is -L-M.

In another embodiment, Q⁵ is -L-M.

In another embodiment, Q⁵ is halo.

In another embodiment, Q¹, Q², Q³, Q⁴ or Q⁵ is independently -(C₀-C₃₀ alkylene)-G. In another embodiment, Q¹, Q² or Q³ is independently -(C₀-C₃₀ alkylene)-G. In another embodiment, Q¹ or Q³ is independently -(C₀-C₃₀ alkylene)-G.

In one embodiment, G is selected from the group consisting of: 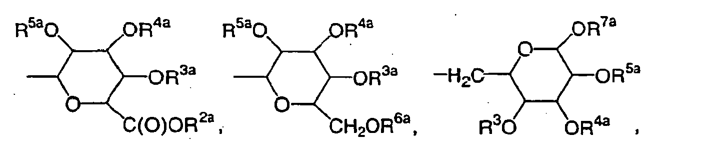 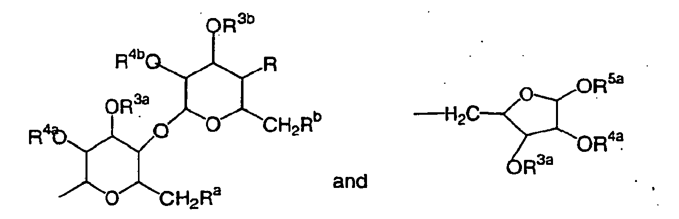 (sugar residues)
wherein
R, R^{a} and R^{b} can be the same or different and each is independently selected from the group consisting of H, -OH, halo, -NH₂, azido, alkoxyalkoxy or -W-R³⁰;
W is independently selected from the group consisting of -NH-C(O)-, -O-C(O)-, -O-C(O)-N(R³¹)-, -NH-C(O)-N(R³¹)- and -O-C(S)-N(R³¹)-;
R2a and R^{6a} can be the same or different and each is independently selected from the group consisting of H, alkyl, acetyl, aryl and arylalkyl;
R^{3a}, R^{4a}, R^{5a}, R^{7a}, R^{3b} and R^{4b} can be the same or different and each is independently selected from the group consisting of H, alkyl, acetyl, arylalkyl, - C(O)alkyl and -C(O)aryl;
R³⁰ is independently selected from the group consisting of R³²-substituted T, R³²-substituted-T-alkyl, R³²-substituted-alkenyl, R³²-substitufed-alkyl, R³²-substituted-cycloalkyl and R³²-substituted-cycloalkylalkyl;
R³¹ is independently selected from the group consisting of H and alkyl;
T is independently selected from the group consisting of phenyl, furyl, thienyl, pyrrolyl, oxazolyl, isoxazolyl, thiazolyl, isothiazolyl, benzothiazolyl, thiadiazolyl, pyrazolyl, imidazolyl and pyridyl;
R³² is 1 to 3 substituents which are each independently selected from the group consisting of H, halo, alkyl, -OH, phenoxy, -CF₃, -NO₂, alkoxy, methylenedioxy, oxo, alkylsulfanyl, alkylsulfinyl, alkylsulfonyl, -N(CH₃)₂, -C(O)-NHalkyl, -C(O)-N(alkyl)₂,
-C(O)-alkyl, -C(O)-alkoxy and pyrrolidinylcarbonyl; or R³² is a covalent bond and R³¹, the nitrogen to which it is attached and R³² form a pyrrolidinyl, piperidinyl, N-methyl-piperazinyl, indolinyl or morpholinyl group, or a alkoxycarbonyl-substituted pyrrolidinyl, piperidinyl, N-methylpiperazinyl, indolinyl or morpholinyl group.

In another embodiment, G is selected from: 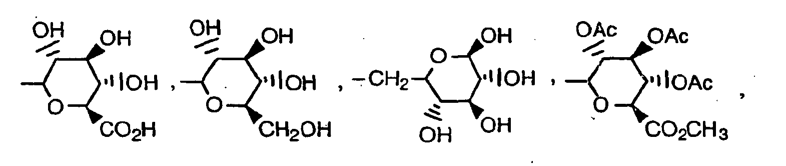 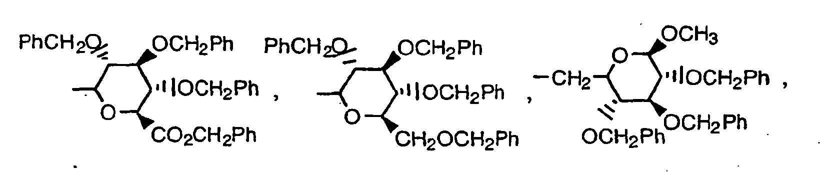 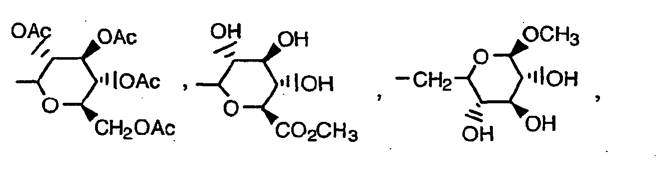 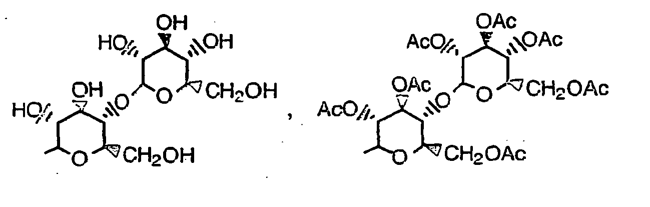 and 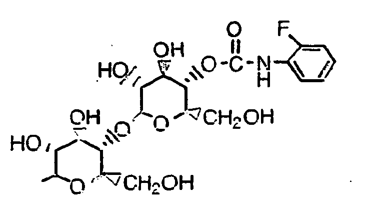 wherein Ac is acetyl and Ph is phenyl.

In another embodiment, optionally one or more carbon atoms of the -(C₀-C₃₀ alkylene)- radical of Q¹, Q², Q³, Q⁴ and Q⁵ is independently replaced by -O-, -C(O)-, - CH=CH-, -C≡C-, -N(alkyl)-, -N(alkylaryl)- or -NH-, preferably -O-.

The -(C₀-C₃₀ alkylene)-G substituent is preferably in the 4-position of the phenyl ring to which it is attached. In one embodiment, L is 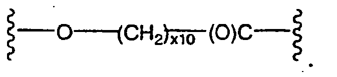 in another embodiment, L is 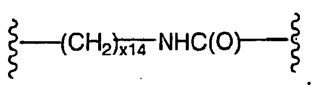 In another embodiment, L is 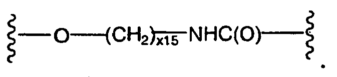 In another embodiment, L is 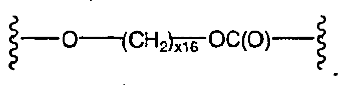 In another embodiment, L is 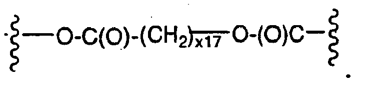 In another embodiment, L is 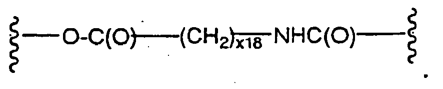 In one embodiment, M is 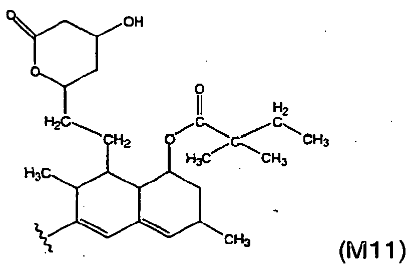 or pharmaceutically acceptable salts thereof.
In another embodiment, M is 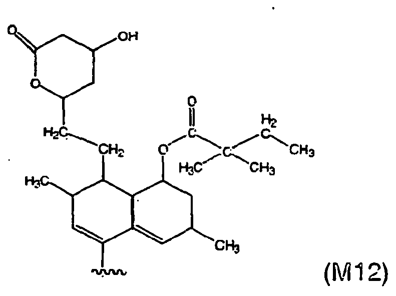 or pharmaceutically acceptable salts thereof.
In another embodiment, M is 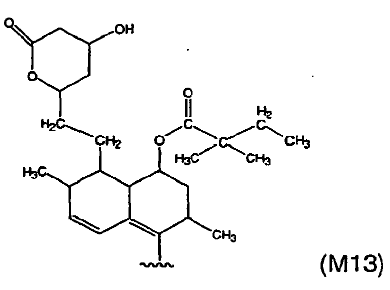 or pharmaceutically acceptable salts thereof.

One embodiment of the present invention is a compound of Formula (II)

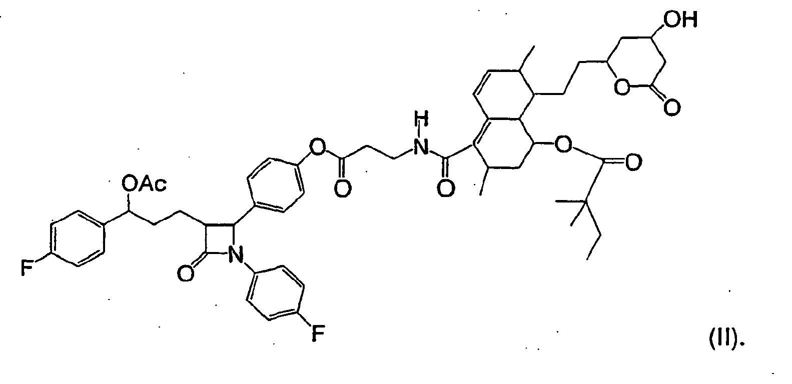

When the compound of Formula (II) is metabolized, one of the compounds (sterol and/or stanol absorption inhibitor) which can be formed is represented by Formula (III) (ezetimibe) below: 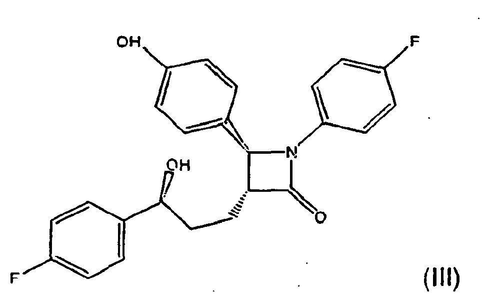 or pharmaceutically acceptable salts, esters or solvates of the compound of Formula (III).

Alternatively or additionally, when the compound of Formula (II) is metabolized, compounds (sterol biosynthesis inhibitor) which can be formed are represented by Formulae (IV) (carboxylated open acid analog of simvastatin) and (V) (carboxylated analog of simvastatin) below:

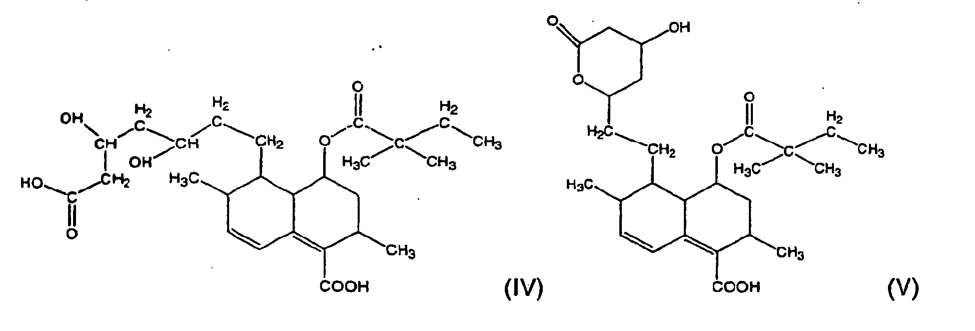

As used above, and throughout the specification, the following terms, unless otherwise indicated, shall be understood to have the following meanings:
"Subject" includes both mammals and non-mammalian animals.
"Mammal" includes humans and other mammalian animals.

The above statements, wherein, for example, Q¹ and Q² are said to be independently selected from a group of substituents, means that Q¹ and Q² are independently selected, but also that where an Q¹ or Q² variable occurs more than once in a molecule, those occurrences are independently selected (e.g., if Q¹ is -OR⁶ wherein R⁶ is hydrogen, Q² can be -OR⁶ wherein R⁶ is alkyl). Those skilled in the art will recognize that the size and nature of the substituent(s) will affect the number of substituents that can be present.

The term "optionally substituted" means optional substitution with the specified groups, radicals or moieties. It should be noted that any atom with unsatisfied valences in the text, schemes, examples and tables herein is assumed to have the hydrogen atom(s) to satisfy the valences.

The following definitions apply regardless of whether a term is used by itself or in combination with other terms, unless otherwise indicated. Therefore, the definition of "alkyl" applies to "alkyl" as well as the "alkyl" portions of "hydroxyalkyl", "haloalkyl", "alkoxy", etc.

As used herein, the term "alkyl" means an aliphatic hydrocarbon group that can be straight or branched and comprises 1 to about 20 carbon atoms in the chain. Preferred alkyl groups comprise 1 to about 12 carbon atoms in the chain. More preferred alkyl groups comprise 1 to about 6 carbon atoms in the chain. "Branched" means that one or more lower alkyl groups such as methyl, ethyl or propyl, are attached to a linear alkyl chain. "Lower alkyl" means a group having about 1 to about 6 carbon atoms in a chain that may be straight or branched. The alkyl can be substituted by one or more substituents independently selected from the group consisting of halo, aryl, cycloalkyl, cyano, hydroxy, alkoxy, alkylthio, amino, -NH(alkyl), -NH(cycloalkyl), -N(alkyl)₂ (which alkyls can be the same or different), carboxy and-C(O)O-alkyl. Non-limiting examples of suitable alkyl groups include methyl, ethyl, n-propyl, isopropyl, n-butyl, t-butyl, n-pentyl, heptyl, nonyl, decyl, fluoromethyl, trifluoromethyl and cyclopropylmethyl.

"Alkenyl" means an aliphatic hydrocarbon group (straight or branched carbon chain) comprising one or more double bonds in the chain and which can be conjugated or unconjugated. Useful alkenyl groups can comprise 2 to about 15 carbon atoms in the chain, preferably 2 to about 12 carbon atoms in the chain, and more preferably 2 to about 6 carbon atoms in the chain. "Lower alkenyl" means 2 to about 6 carbon atoms in the chain that can be straight or branched. The alkenyl group can be substituted by one or more substituents independently selected from the group consisting of halo, alkyl, aryl, cycloalkyl, cyano and alkoxy. Non-iimiting examples of suitable alkenyl groups include ethenyl, propenyl, n-butenyl, 3-methylbutenyl and n-pentenyl.

Where an alkyl or alkenyl chain joins two other variables and is therefore bivalent, the terms alkylene and alkenylene, respectively, are used.

"Alkoxy" means an alkyl-O- group in which the alkyl group is as previously described. Useful alkoxy groups can comprise 1 to about 12 carbon atoms, preferably 1 to about 6 carbon atoms. Non-limiting examples of suitable alkoxy groups include methoxy, ethoxy and isopropoxy. The alkyl group of the alkoxy is linked to an adjacent moiety through the ether oxygen.

"Alkoxyarylalkoxy" means an alkyl-O-aryl-alkylene-O- group in which the alkyl, alkylene and aryl groups are as previously described. Useful alkoxyarylalkoxy groups can comprise 7 to about 26 carbon atoms, preferably 7 to about 12 carbon atoms. A non-limiting example of a suitable alkoxyarylalkoxy group is methoxybenzyloxy. The alkoxyarylalkoxy is linked to an adjacent moiety through the ether oxygen.

"Alkoxycarbonylalkoxy" means an alkyl-O-C(O)-alkylene-O- group in which the alkyl and alkylene groups are as previously described. Useful alkoxycarbonylalkoxy groups can comprise 3 to about 12 carbon atoms, preferably 3 to about 8 carbon atoms. A non-limiting example of a suitable alkoxycarbonylalkoxy group is CH₃CH₂-O-C(O)-CH₂-O-. The alkoxycarbonylalkoxy is linked to an adjacent moiety through the ether oxygen.

"Alkoxyiminoalkyl" means an alkyl-O-N=CH-alkylene- group in which the alkyl and alkylene groups are as previously described. Useful alkoxyiminoalkyl groups can comprise 2 to about 12 carbon atoms, preferably 2 to about 8 carbon atoms. The alkoxyiminoalkyl is linked to an adjacent moiety through the alkylene group.

"Alkyldioyl" means an ROC(O)-alkylene-C(O)-O- group in which R is alkyl or H and the alkylene group is as previously described. Useful alkyldioyl groups can comprise 2 to about 12 carbon atoms, preferably 2 to about 8 carbon atoms. Non-limiting examples of suitable alkyldioyl groups include 1,3-propanediol. The alkyldioyl is linked to an adjacent moiety through the ester oxygen.

"Alkynyl" means an aliphatic hydrocarbon group comprising at least one carbon-carbon triple bond and which may be straight or branched and comprising about 2 to about 15 carbon atoms in the chain. Preferred alkynyl groups have about 2 to about 12 carbon atoms in the chain; and more preferably about 2 to about 4 carbon atoms in the chain. Branched means that one or more lower alkyl groups such as methyl, ethyl or propyl, are attached to a linear alkynyl chain. "Lower alkynyl" means about 2 to about 6 carbon atoms in the chain which may be straight or branched. Non-limiting examples of suitable alkynyl groups include ethynyl, propynyl, 2-butynyl, 3-methylbutynyl, n-pentynyl, and decynyl. The alkynyl group may be substituted by one or more substituents which may be the same or different, each substituent being independently selected from the group consisting of alkyl, aryl and cycloalkyl.

"Allyloxy" means H₂C=CH-O-. The allyloxy is linked to an adjacent moiety through the ether oxygen.

"Aryl" means an aromatic monocyclic or multicyclic ring system comprising about 5 to about 14 carbon atoms, preferably about 6 to about 10 carbon atoms. The aryl group can be substituted with one or more "ring system substituents" which may be the same or different, and are as defined herein. Non-limiting examples of suitable aryl groups include phenyl, naphthyl, indenyl, tetrahydronaphthyl and indanyl. "Arylene" means a bivalent phenyl group, including ortho, meta and para-substitution.

"Aralkyl" or "arylalkyl" means an aryl-alkylene- group in which the aryl and alkylene are as previously described. Preferred aralkyls comprise a lower alkyl group. Non-limiting examples of suitable aralkyl groups include benzyl, phenethyl and naphthlenylmethyl. The aralkyl is linked to an adjacent moiety through the alkylene group.

"Aryloxy" means an aryl-O- group in which the aryl group is as previously described. Non-limiting examples of suitable aryloxy groups include phenoxy and naphthoxy. The bond to the parent moiety is through the ether oxygen.

"Aralkoxy" or "arylalkyloxy" means an aralkyl-O- group in which the aralkyl group is as previously described. Non-limiting examples of suitable aralkoxy groups include benzyloxy and 1- or 2-naphthalenemethoxy. The bond to the parent moiety is through the ether oxygen. "Aralkoxycarbonyl" means an aralkoxy-C(O)- group in which the aralkoxy group is as previously described.

"Aroyl" means an aryl-C(O)- group in which the aryl group is as previously described. The bond to the parent moiety is through the carbonyl. Non-limiting examples of suitable groups include benzoyl and 1- and 2-naphthoyl.

"Aroyloxy" means an aroyl-O- group in which the aroyl group is as previously described. The bond to the parent moiety is through the ether oxygen. Non-limiting examples of suitable groups include benzoyloxy and 1- and 2-naphthoyloxy.

"Cycloalkyl" means a non-aromatic mono- or multicyclic ring system comprising about 3 to about 10 carbon atoms, preferably about 5 to about 10 carbon atoms. Preferred cycloalkyl rings contain about 5 to about 7 ring atoms. The cycloalkyl can be substituted with one or more "ring system substituents" which may be the same or different, and are as defined below. Non-limiting examples of suitable monocyclic cycloalkyls include cyclopropyl, cyctobutyl, cyclopentyl, cyclohexyl and the like. Non-limiting examples of suitable multicyclic cycloalkyls include 1-decalinyl, norbornyl, adamantyl and the like. "Cycloalkylene" refers to a corresponding bivalent ring, wherein the points of attachment to other groups include all positional isomers.

"Dioxolanyl" means

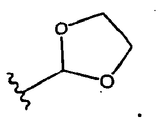

"Halo" refers to fluorine, chlorine, bromine or iodine radicals. Preferred are fluoro, chloro or bromo, and more preferred are fluoro and chloro.

"Heteroaryl" means a monocyclic or multicyclic aromatic ring system of about 5 to about 14 ring atoms, preferably about 5 to about 10 ring atoms, in which one or more of the atoms in the ring system is/are atoms other than carbon, for example nitrogen, oxygen or sulfur. The heteroatom(s) interrupt a carbocyclic ring structure and have a sufficient number of delocalized pi electrons to provide aromatic character, provided that the rings do not contain adjacent oxygen and/or sulfur atoms. Preferred heteroaryls contain about 5 to about 6 ring atoms. The "heteroaryl" can be optionally substituted by one or more "ring system substituents" which may be the same or different, and are as defined herein. The prefix aza, oxa or thia before the heteroaryl root name means that at least a nitrogen, oxygen or sulfur atom respectively, is present as a ring atom. A nitrogen atom of a heteroaryl can be oxidized to form the corresponding N-oxide. All regioisomers are contemplated, e.g., 2-pyridyl, 3-pyridyl and 4-pyridyl. Examples of useful 6-membered heteroaryl groups include pyridyl, pyrimidinyl, pyrazinyl, pyridazinyl and the like and the N-oxides thereof. Examples of useful 5-membered heteroaryl rings include furyl, thienyl, pyrrolyl, thiazolyl, isothiazolyl, imidazolyl, pyrazolyl and isoxazolyl. Useful bicyclic groups are benzo-fused ring systems derived from the heteroaryl groups named above, e.g., quinolyl, phthalazinyl, quinazolinyl, benzofuranyl, benzothienyl and indolyl.

"Heteroarylalkyl" or "heteroaralkyl" means a heteroaryl-alkylene- group in which the heteroaryl and alkyl are as previously described. Preferred heteroaralkyls contain a lower alkyl group. Non-limiting examples of suitable heteroaralkyl groups include pyridylmethyl, 2-(furan-3-yl)ethyl and quinolin-3-ylmethyl. The bond to the parent moiety is through the alkylene. "Heteroarylalkoxy" means a heteroaryl-alkylene-O-group in which the heteroaryl and alkylene are as previously described.

"Heterocyclyl" means a non-aromatic saturated monocyclic or multicyclic ring system comprising about 3 to about 10 ring atoms, preferably about 5 to about 10 ring atoms, in which one or more of the atoms in the ring system is an element other than carbon, for example nitrogen, oxygen or sulfur, alone or in combination. There are no adjacent oxygen and/or sulfur atoms present in the ring system. Preferred heterocyclyls contain about 5 to about 6 ring atoms. The prefix aza, oxa or thia before the heterocyclyl root name means that at least a nitrogen, oxygen or sulfur atom respectively is present as a ring atom. The heterocyclyl can be optionally substituted by one or more "ring system substituents" which may be the same or different, and are as defined herein. The nitrogen or sulfur atom of the heterocyclyl can be optionally oxidized to the corresponding N-oxide, S-oxide or S,S-dioxide. Non-limiting examples of suitable monocyclic heterocyclyl rings include piperidyl, pyrrolidinyl, piperazinyl, morpholinyl, thiomorpholinyl, thiazolidinyl, 1,3-dioxolanyl, 1,4-dioxanyl, tetrahydrofuranyl, tetrahydrothiophenyl, tetrahydrothiopyranyl, and the like.

"Heterocyclylalkyl" means a heterocyclyl-alkylene- group in which the heterocyclyl and alkylene groups are as previously described. Preferred heterocyclylalkyls contain a lower alkylene group. The bond to the parent moiety is through the alkylene. "Heterocyclylcarbonyl" means a heterocyclyl-C(O)- group in which the heterocyclyl is as previously described. Preferred heterocyclylcarbonyls contain a lower alkyl group. The bond to the parent moiety is through the carbonyl. "Heterocyclylcarbonylalkoxy" means a heterocyclyl-C(O)-alkoxy- group in which the heterocyclyl and alkoxy are as previously described.

"Ring system substituent" means a substituent attached to an aromatic or non-aromatic ring system that, for example, replaces an available hydrogen on the ring system. Ring system substituents may be the same or different, each being independently selected from the group consisting of aryl, heteroaryl, aralkyl, alkylaryl, aralkenyl, heteroaralkyl, alkylheteroaryl, heteroaralkenyl, hydroxy, hydroxyalkyl, alkoxy, aryloxy, aralkoxy, acyl, aroyl, halo, nitro, cyano, carboxy, alkoxycarbonyl, aryloxycarbonyl, aralkoxycarbonyl, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio, cycloalkyl, cycloalkenyl, heterocyclyl, heterocyclenyl, Y₁Y₂N-; Y₁Y₂N-alkyl-, Y₁Y₂NC(O)- and Y₁Y₂NSO₂-, wherein Y₁ and Y₂ may be the same or different and are independently selected from the group consisting of hydrogen, alkyl, aryl, and aralkyl.

"Sugar residue" means a moiety derived from an aldose or ketose that has 3 to 7 carbon atoms and may belong to the D or L series. Non-limiting examples of suitable aldoses from which the sugar residue can be formed include glucose, mannose, galactose, ribose, erythrose and glyceraldehydes. A non-limiting example of a suitable ketose from which the sugar residue can be formed is fructose.

"Disugar residue" means a moiety derived from a sugar that can be hydrolyzed to two monosaccharide molecules. Non-limiting examples of suitable compounds from which the disugar residue can be formed include maltose, lactose, cellobiose and sucrose.

Examples of sugar residues and disugar residues include those moieties G listed in detail above.

' Di-, tri- or tetrasaccharides are formed by acetal-like binding of two or more sugars. The bonds may be in α or β form. "Trisugar residue" means a moiety derived from a sugar that can be hydrolyzed to three monosaccharide molecules. "Tetrasugar residue" means a moiety derived from a sugar that can be hydrolyzed to four monosaccharide molecules.

If the sugar is substituted, the substitution is preferably at the hydrogen atom of an OH group of the sugar.

"Sugar acid" means an sugar residue, such as can be formed from glucuronic acid, galacturonic acid, gluconic acid, galactonic acid, mannonic acid, glucaric acid and galactaric acid.

"Amino sugar" means an amino-substituted sugar residue such as can be formed from glucosamine, galactosamine, glucamine or 3-amino-1,2-propanediol.

Suitable protective groups for the hydroxyl groups of the sugars include benzyl, acetyl, benzoyl, pivaloyl, trityl, tert-butyldimethylsilyl, benzilidene, cyclohexidene or isopropylidene protective groups.

"Amino acid residue" means a moiety derived from an amino acid. The amino acid moiety can be prepared from the D or L forms of the amino acid. Non-limiting examples of suitable amino acids from which the amino acid residue can be prepared include alanine, arginine, asparagine, aspartic acid, cysteine, cystine, glutamic acid, glutamine, glycine, histidine, hydroxylysine, hydroxyproline, isoleucine, leucine, lysine, methionine, phenylanine, proline, serine, threonine, tryptophane, tyrosine, valine, 2-aminoadipic acid, 3-aminoadipic acid, beta-alanine, 2-aminobutyric acid, 4-aminobutyric acid, piperidino carboxylic acid, 6-aminocaproic acid, 2-aminoheptanoic acid, 2-(2-thienyl)glycine, penicillamine, N-ethylasparagine, 2-aminoisobutyric acid, 2-aminoisobutyric acid, 2-aminopimelic acid, 2,4-diaminobutyric acid, desmosine, 2,2-diaminopimelic acid, 2,3-diaminopropioninc acid, N-ethylglycine, 3-(2-thienyl)alanine, sarcosine, N-methylisoleucine, 6-N-methyllysine, N-methylvaline, norvaline, norleucine, ornithine and N-methylglycine.

"Oligopeptide residue" means the residue of a peptide constructed of 2 to 9 of the amino acids mentioned above.

"Trialkylammonium alkyl radical" means the group 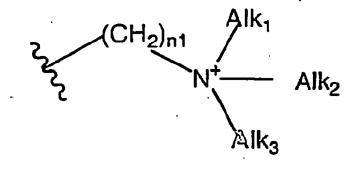 wherein n1 is 0 to 10 and Alk₁, Alk₂ and Alk₃ can be the same or different and each is a straight or branched alkyl radical having 1 to 20 carbon atoms.

Compounds of the invention have at least one asymmetrical carbon atom and therefore all enantiomers, stereoisomers, rotamers, tautomers and racemates of the compounds of Formula (IA) (where they exist) are contemplated as being part of this invention. The invention includes d and I isomers in both pure form and in admixture, including racemic mixtures, Isomers can be prepared using conventional techniques, either by reacting optically pure or optically enriched starting materials or by separating isomers of a compound of the Formula (IA). Polymorphous forms of the compounds of Formula (IA), whether crystalline or amorphous, also are contemplated as being part of this invention.

Those skilled in the art will appreciate that for some of the compounds of the Formula (IA), one isomer will show greater pharmacological activity than other isomers.

Compounds of the invention with an amino group can form pharmaceutically acceptable salts with organic and inorganic acids. Examples of suitable acids for salt formation are hydrochloric, sulfuric, phosphoric, acetic, citric, oxalic, malonic, salicylic, malic, fumaric, succinic, ascorbic, maleic, methanesulfonic and other mineral and carboxylic acids well known to those in the art. The salt is prepared by contacting the free base form with a sufficient amount of the desired acid to produce a salt. The free base form may be regenerated by treating the salt with a suitable dilute aqueous base solution such as dilute aqueous sodium bicarbonate. The free base form differs from its respective salt form somewhat in certain physical properties, such as solubility in polar solvents, but the salt is otherwise equivalent to its respective free base forms for purposes of the invention.

Certain compounds of the invention are acidic (e.g., those compounds which possess a carboxyl group). These compounds form pharmaceutically acceptable salts with inorganic and organic bases. Examples of such salts are the sodium, potassium, calcium, aluminum, gold and silver salts. Also included are salts formed with pharmaceutically acceptable amines such as ammonia, alkyl amines, hydroxyalkylamines, N-methylglucamine and the like.

Compounds of the invention with a carboxylic acid group can form pharmaceutically acceptable esters with an alcohol. Examples of suitable alcohols include methanol and ethanol.

Solvates of the compounds of the invention are also contemplated herein.

"Solvate" means a physical association of a compound of this invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolatable solvates. Non-limiting examples of suitable solvates include ethanolates, methanolates, and the like. "Hydrate" is a solvate wherein the solvent molecule is H₂O.

Generally, the azetidinone portion of the compounds of Formula (I) can be prepared by a variety of methods well known to those skilled in the art, for example such as are disclosed in U.S. Patents Nos. 5,631,365, 5,767,115, 5,846,966, 6,207,822, PCT Patent Application No. 02/079174 and PCT Patent Application WO 93/02048, and in the Example below. Preferably the azetidinone is prepared from ezetimibe, such as can be prepared by routine separation methods from ZETIA® ezetimibe formulation that is commercially available from Schering-Plough Corporation.

The statin compound for preparing the -M portion of the molecule can be prepared by a variety of methods, for example the statin compound for preparing M11, M12 or M13 can be prepared by methods such as are disclosed in PCT WO 98/12188, U.S. Patents Nos. 5763653, 5763646, 4444784., 4582915, 4820850, or by routine separation methods from ZOCOR® simvastatin formulation which is commercially available from Merck & Co. Inc. The compound for preparing M14 or M15 can be prepared by methods such as are disclosed in U.S. Patents Nos. 4231938, 4294926, US 5763653, 4323648, 4916239, 5763646 or by routine separation methods from MEVACOR® lovastatin formulation which is commercially available from Merck & Co. Inc. The compound for preparing M1, M3, M4, M17, M19, M20, M21 or M22 can be prepared by methods such as are disclosed in U.S. Patents Nos. 5273995, 4681893, 5969156 or by routine separation methods from LIPITOR® atorvastatin formulation which is commercially available from Pfizer. The compound for preparing M6 or M23 can be prepared by methods such as are disclosed in U.S. Patent No. 5260440 or by routine separation methods from CRESTOR® rosuvastatin formulation that is commercially available from AstraZeneca. The compound for preparing M24 can be prepared by methods such as are disclosed in U.S. Patents Nos. 5006530 and 5177080. The compound for preparing M7, M25, M26, or M27 can be prepared by methods such as are disclosed in U.S. Patents Nos. 5872130, 5856336, 5011930 and 5854259. The compound for preparing M28, M29 or M30 can be prepared by methods such as are disclosed in U.S. Patents Nos. 4346227, 4537859, 4410629 or by routine separation methods from PRAVACHOL® pravastatin formulation which is commercially available from Bristol-Myers Squibb. The compound for preparing M8, M9, M10, M31, M32 or M33 can be prepared by methods such as are disclosed in U.S. Patents Nos. 5354772 and 4739073 or by routine separation methods from LESCOL® fluvastatin formulation that is commercially available from Novartis.

In general, the compounds of Formula (IA) can be prepared through the general routes described below in Scheme 1. The azetidinone portion of the molecule and -M portion of the molecule can be linked by linker -L- as shown for example in Scheme 1 below. Non-limiting examples of suitable compounds for preparing linker 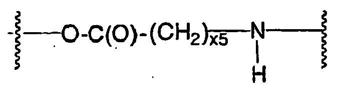 are from N-Boc-β-alanine, N-Boc glycine and N-Boc-6-aminocaproic acid, respectively, in a manner as shown below:

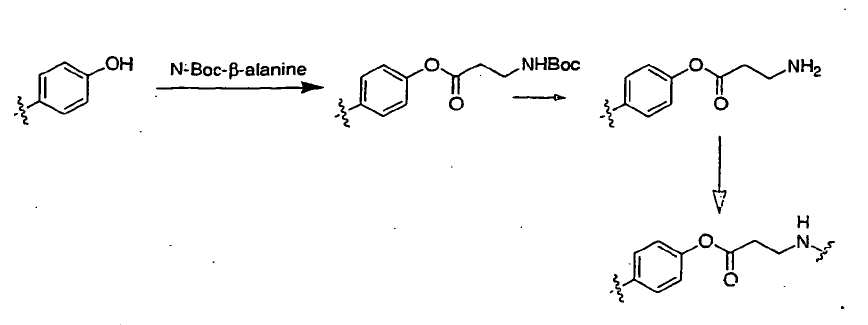

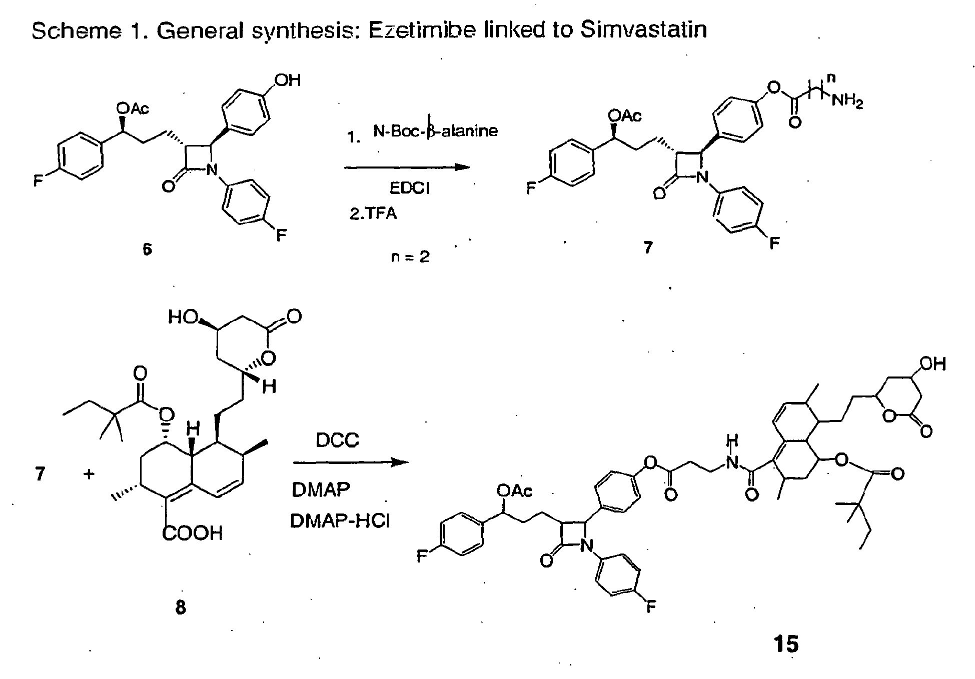

Generally, in Scheme 1, treatment of the starting azetidinone (for example acetoxy-protected ezetimibe 6) with an amino acid in which the amine functionality can be blocked with a suitable protective group such as a butoxycarbonyl (Boc) and an amide coupling reagent such as 1-(3-Dimethylaminopropyl)-3-ethylcarbodiimide gives rise to the protected amino ester which upon treatment with mild acid such as trifluoroacetic acid gives the desired amine-substituted azetidinone 7. Selection of solvents and additives for the amide coupling reaction may vary and would be obvious to one skilled in the art. Amine-substituted azetidinone 7 is reacted with carboxy-substituted statin 8 (for example simvastatin) and pyridine to form compound 9 of the present invention.

The daily dose of the compound of Formula (IA) can range from about 0.1 to about 1000 mg per day, preferably about 0.25 to about 100 mg/day, and more preferably about 5, 10, 20, 30, 40, 50 , 60 ,70, 80 , 90 or 100 mg per day, given in a single dose or 2-4 divided doses. The exact dose, however, is determined by the attending clinician and is dependent on the potency of the compound administered, the age, weight, condition and response of the patient. The phrases "effective amount" and "therapeutically effective amount" mean that amount of a compound of Formula I, and other pharmacological or therapeutic agents described below, that will elicit a biological or medical response of a tissue, system, animal or mammal that is being sought by the administrator (such as a researcher, doctor or veterinarian) which includes alleviation of the symptoms of the condition or disease being treated and the prevention, slowing or halting of progression of one or more conditions, for example vascular conditions, such as hyperlipidaemia (for example atherosclerosis, hypercholesterolemia or sitosterolemia), vascular inflammation, stroke, diabetes, obesity and/or to reduce the level of sterol(s) (such as cholesterol) or stanol(s) in the plasma of a subject. As used herein, "vascular" comprises cardiovascular, cerebrovascular, peripheral vascular and combinations thereof. The formulations or compositions, combinations and treatments of the present invention can be administered by any suitable means which produce contact of these compounds with the site of action in the body, for example in the plasma, liver or small intestine of a mammal or human.

For administration of pharmaceutically acceptable salts of the above compounds, the weights indicated above refer to the weight of the acid equivalent or the base equivalent of the therapeutic compound derived from the salt.

In one embodiment of the present invention, the compositions or therapeutic combinations can further comprise one or more pharmacological or therapeutic agents or drugs such as lipid-lowering agents discussed below. As used herein, "combination therapy" or "therapeutic combination" means the administration of two or more therapeutic agents, such as a compound of Formula (I) and a lipid-lowering or antihypertensive agent, to prevent or treat a condition as described above. Such administration includes coadministration of these therapeutic agents in a substantially simultaneous manner, such as in a single tablet or capsule having a fixed ratio of active ingredients or in multiple, separate capsules for each therapeutic agent. Also, such administration includes use of each type of therapeutic agent in a sequential manner. In either case, the treatment using the combination therapy will provide beneficial effects in treating the condition. A potential advantage of the combination therapy disclosed herein may be a reduction in the required amount of an individual therapeutic compound or the overall total amount of therapeutic compounds that are effective in treating the condition. By using a combination of therapeutic agents, the side effects of the individual compounds can be reduced as compared to a monotherapy, which can improve patient compliance. Also, therapeutic agents can be selected to provide a broader range of complimentary effects or complimentary modes of action.

Non-limiting examples of additional cholesterol biosynthesis inhibitors for use in the compositions, therapeutic combinations and methods of the present invention include squalene synthase inhibitors, squalene epoxidase inhibitors and mixtures thereof. Non-limiting examples of suitable HMG CoA synthetase inhibitors include L-659,699 ((E,E)-11 -[3'R-(hydroxy-methyl)-4'-oxo-2'R-oxetanyl]-3,5,7R-trimethyl-2,4-undecadienoic acid); squalene synthesis inhibitors, for example squalestatin 1; and squalene epoxidase inhibitors, for example, NB-598 ((E)-N-ethyl-N-(6,6-dimethyl-2-hepten-4-ynyl)-3-[(3,3'-bithiophen-5-yl)methoxy]benzene-methanamine hydrochloride) and other sterol biosynthesis inhibitors such as DMP-565. Generally, a total daily dosage of additional cholesterol biosynthesis inhibitor(s) can range from about 0.1 to about 160 mg per day, and preferably about 0.2 to about 80 mg/day in single or 2-3 divided doses.

In another preferred embodiment, the composition or treatment comprises the compound of Formula (IA) in combination with one or more peroxisome proliferator-activated receptor(s) activator(s). In this embodiment, preferably the peroxisome proliferator-activated receptor activator(s) is a fibric acid derivative such as gemfibrozil, clofibrate and/or fenofibrate.

In another alternative embodiment, the compositions, therapeutic combinations or methods of the present invention can further comprise one or more bile acid sequestrants (insoluble anion exchange resins), coadministered with or in combination with the compound of Formula (I) discussed above. Bile acid sequestrants bind hile acids in the intestine, interrupting the enterohepatic circulation of bile acids and causing an increase in the faecal excretion of steroids. Bile acid sequestrants can lower intrahepatic cholesterol and promote the synthesis of apo B/E (LDL) receptors that bind LDL from plasma to further reduce cholesterol levels in the blood. Non-limiting examples of suitable bile acid sequestrants include cholestyramine (a styrenedivinylbenzene copolymer containing quaternary ammonium cationic groups capable of binding bile acids, such as QUESTRAN® or QUESTRAN LIGHT® cholestyramine which are available from Bristol-Myers Squibb), colestipol (a copolymer of diethylenetriamine and 1-chloro-2,3-epoxypropane, such as COLESTID® tablets which are available from Pharmacia), and colesevelam hydrochloride (such as WelChol® Tablets (poly(allylamine hydrochloride) cross-linked with epichlorohydrin and alkylated with 1-bromodecane and (6-bromohexyl)-trimethylammonium bromide) which are available from Sankyo). Generally, a total daily dosage of bile acid sequestrant(s) can range from about 1 to about 50 grams per day, and preferably about 2 to about 16 grams per day in single or 2-4 divided doses.

In an alternative embodiment, the compositions or treatments of the present invention can further comprise one or more ileal bile acid transport ("IBAT") inhibitors (or apical sodium co-dependent bile acid transport ("ASBT") inhibitors) coadministered with or in combination with the compound of Formula (I) discussed above. The IBAT inhibitors can inhibit bile acid transport to reduce LDL cholesterol levels. Non-limiting examples of suitable IBAT inhibitors include benzothiepines such as therapeutic compounds comprising a 2,3,4,5-tetrahydro-1-benzothiepine 1,1-dioxide structure such as are disclosed in PCT Patent Application WO 00/38727 which is incorporated herein by reference. Generally, a total daily dosage of IBAT inhibitor(s) can range from about 0.01 to about 1000 mg/day, and preferably about 0.1 to about 50 mg/day in single or 2-4 divided doses.

In another alternative embodiment, the compositions or treatments of the present invention can further comprise nicotinic acid (niacin) and/or derivatives thereof coadministered with or in combination with the compound of Formula (I) discussed above. As used herein, "nicotinic acid derivative" means a compound comprising a pyridine-3-carboxylate structure or a pyrazine-2-carboxylate structure, including acid forms, salts, esters, zwitterions and tautomers, where available. Examples of nicotinic acid derivatives include niceritrol, nicofuranose and acipimox (5-methyl pyrazine-2-carboxylic acid 4-oxide). Nicotinic acid and its derivatives inhibit hepatic production of VLDL and its metabolite LDL and increases HDL and apo A-1 levels. An example of a suitable nicotinic acid product is NIASPAN® (niacin extended-release tablets) which are available from Kos. Generally, a total daily dosage of nicotinic acid or a derivative thereof can range from about 500 to about 10,000 mg/day, preferably about 1000 to about 8000 mg/day, and more preferably about 3000 to about 6000 mg/day in single or divided doses.

In another alternative embodiment, the compositions or treatments of the present invention can further comprise one or more AcylCoA:Cholesterol O-acyltransferase ("ACAT") Inhibitors, which can reduce LDL and VLDL levels, coadministered with or in combination with the compound of Formula (I) discussed above. ACAT is an enzyme responsible for esterifying excess intracellular cholesterol and may reduce the synthesis of VLDL, which is a product of cholesterol esterification, and overproduction of apo B-100-containing lipoproteins. Non-limiting examples of useful ACAT inhibitors include avasimibe, HL-004, lecimibide (DuP-128) and CL-277082 (*N-*(2,4-difluorophenyl)-*N-*[[4-(2,2-dimethylpropyl)phenyl]methyl]-*N-*heptylurea). See P. Chang et al., "Current, New and Future Treatments in Dyslipidaemia and Atherosclerosis", Drugs 2000 Jul;60(1); 55-93, which is incorporated by reference herein. Generally, a total daily dosage of ACAT inhibitor(s) can range from about 0.1 to about 1000 mg/day in single or 2-4 divided doses.

In another alternative embodiment, the compositions or treatments of the present invention can further comprise one or more Cholesteryl Ester Transfer Protein ("CETP") Inhibitors coadministered with or in combination with compound of Formula (I) discussed above. CETP is responsible for the exchange or transfer of cholesteryl ester carrying HDL and triglycerides in VLDL. Non-limiting examples of suitable CETP inhibitors are disclosed in PCT Patent Application No. WO 00/38721 and U.S. Patent No. 6,147,090, which are incorporated herein by reference. Pancreatic cholesteryl ester hydrolase (pCEH) inhibitors such as WAY-121898 also can be coadministered with or in combination with the compound of Formula (I) discussed above. Generally, a total daily dosage of CETP inhibitor(s) can range from about 0.01 to about 1000 mg/day, and preferably about 0.5 to about 20 mg/kg body weight/day in single or divided doses.

In another alternative embodiment, the compositions or treatments of the present invention can further comprise probucol or derivatives thereof (such as AGI-1067 and other derivatives disclosed in U.S. Patents Nos. 6,121,319 and 6,147,250), which can reduce LDL levels, coadministered with or in combination with the compound of Formula (I) discussed above. Generally; a total daily dosage of probucol or derivatives thereof can range from about 10 to about 2000 mg/day, and preferably about 500 to about 1500 mg/day in single or 2-4 divided doses.

In another alternative embodiment, the compositions or treatments of the present invention can further comprise low-density lipoprotein (LDL) receptor activators, coadministered with or in combination with the compound of Formula (I) discussed above. Non-limiting examples of suitable LDL-receptor activators include HOE-402, an imidazolidinyl-pyrimidine derivative that directly stimulates LDL receptor activity. See M. Huettinger et al., "Hypolipidemic activity of HOE-402 is Mediated by Stimulation of the LDL Receptor Pathway", Arterioscler. Thromb. 1993; 13:1005-12. Generally, a total daily dosage of LDL receptor activator(s) can range from about 1 to about 1000 mg/day in single or 2-4 divided doses.

In another alternative embodiment, the compositions or treatments of the present invention can further comprise fish oil, which contains Omega 3 fatty acids (3-PUFA), which can reduce VLDL and triglyceride levels, coadministered with or in combination with the compound of Formula (I) discussed above. Generally, a total daily dosage of fish oil or Omega 3 fatty acids can range from about 1 to about 30 grams per day in single or 2-4 divided doses.

In another alternative embodiment, the compositions or treatments of the present invention can further comprise natural water soluble fibers, such as psyllium, guar, oat and pectin, which can reduce cholesterol levels, coadministered with or in combination with the compound of Formula (I) discussed above. Generally, a total daily dosage of natural water soluble fibers can range from about 0.1 to about 10 grams per day in single or 2-4 divided doses.

In another alternative embodiment, the compositions or treatments of the present invention can further comprise plant sterols, plant stanols and/or fatty acid esters of plant stanols, such as sitostanol ester used in BENECOL® margarine, which can reduce cholesterol levels, coadministered with or in combination with the compound of Formula (I) discussed above. Generally, a total daily dosage of plant sterols, plant stanols and/or fatty acid esters of plant stanols can range from about 0.5 to about 20 grams per day in single or 2-4 divided doses.

In another alternative embodiment, the compositions or treatments of the present invention can further comprise antioxidants, such as probucol, tocopherol, ascorbic acid, β-carotene and selenium, or vitamins such as vitamin B₆ or vitamin B₁₂, coadministered with or in combination with the compound of Formula (I) discussed above. Generally, a total daily dosage of antioxidants or vitamins can range from about 0.05 to about 10 grams per day in single or 2-4 divided doses.

In another alternative embodiment, the compositions or treatments of the present invention can further comprise monocyte and macrophage inhibitors such as polyunsaturated fatty acids (PUFA), thyroid hormones including throxine analogues such as CGS-26214 (a thyroxine compound with a fluorinated ring), gene therapy and use of recombinant proteins such as recombinant apo E, coadministered with or in combination with the compound of Formula (I) discussed above. Generally, a total daily dosage of these agents can range from about 0.01 to about 1000 mg/day in single or 2-4 divided doses.

Also useful with the present invention are compositions or therapeutic combinations that further comprise hormone replacement agents and compositions. Useful hormone agents and compositions for hormone replacement therapy of the present invention include androgens, estrogens, progestins, their pharmaceutically acceptable salts and derivatives thereof. Combinations of these agents and compositions are also useful. The dosage of androgen and estrogen combinations vary, desirably from about 1 mg to about 4. mg androgen and from about 1 mg to about 3 mg estrogen.

The compositions, therapeutic combinations or methods of the present invention can further comprise one or more obesity control medications. Useful obesity control medications include, but are not limited to, drugs that reduce energy intake or suppress appetite, drugs that increase energy expenditure and nutrient-partitioning agents. Suitable obesity control medications include, but are not limited to, noradrenergic agents (such as diethylpropion, mazindol, phenylpropanolamine, phentermine, phendimetrazine, phendamine tartrate, methamphetamine, phendimetrazine and tartrate); serotonergic agents (such as sibutramine, fenfluramine, dexfenfluramine, fluoxetine, fluvoxamine and paroxtine); thermogenic agents (such as ephedrine, caffeine, theophylline, and selective β3-adrenergic agonists); alpha-blocking agents; kainite or AMPA receptor antagonists; leptin-lipolysis stimulated receptors; phosphodiesterase enzyme inhibitors; compounds having nucleotide sequences of the mahogany gene; fibroblast growth factor-10 polypeptides; monoamine oxidase inhibitors (such as befloxatone, moclobemide, brofaromine, phenoxathine, esuprone, befol, toloxatone, pirlindol, amiflamine, sercloremine, bazinaprine, lazabemide, milacemide and caroxazone); compounds for increasing lipid metabolism (such as evodiamine compounds); and lipase inhibitors (such as orlistat). Generally, a total dosage of the above-described obesity control medications can range from 1 to 3,000 mg/day, desirably from about 1 to 1,000 mg/day and more desirably from about 1 to 200 mg/day in single or 2-4 divided doses.

The compositions, therapeutic combinations or methods of the present invention can further comprise one or more blood modifiers which are chemically different from the compounds of Formula (I) discussed above, for example, they contain one or more different atoms, have a different arrangement of atoms or a different number of one or more atoms than the compounds of Formula (I) discussed above. Useful blood modifiers include but are not limited to anti-coagulants (argatroban, bivalirudin, dalteparin sodium, desirudin, dicumarol, lyapolate sodium, nafamostat mesylate, phenprocoumon, tinzaparin sodium, warfarin sodium); antithrombotic (anagrelide hydrochloride, bivalirudin, cilostazol, dalteparin sodium, danaparoid sodium, dazoxiben hydrochloride, efegatran sulfate, enoxaparin sodium, fluretofen, ifetroban, ifetroban sodium, lamifiban, lotrafiban hydrochloride, napsagatran, orbofiban acetate, roxifiban acetate, sibrafiban, tinzaparin sodium, trifenagrel, abciximab, zolimomab aritox); fibrinogen receptor antagonists (roxifiban acetate, fradafiban, orbofiban, lotrafiban hydrochloride, tirofiban, xemilofiban, monoclonal antibody 7E3, sibrafiban); platelet inhibitors (cilostazol, clopidogrel bisulfate, epoprostenol, epoprostenol sodium, ticlopidine hydrochloride, aspirin, ibuprofen, naproxen, sulindae, idomethacin, mefenamate, droxicam, diclofenac, sulfinpyrazone, piroxicam, dipyridamole); platelet aggregation inhibitors (acadesine, beraprost, beraprost sodium, ciprostene calcium, itazigrel, lifarizine, lotrafiban hydrochloride, orbofiban acetate, oxagrelate, fradafiban, orbofiban, tirofiban, xemilofiban); hemorrheologic agents (pentoxifylline); lipoprotein associated coagulation inhibitors; Factor VIIa inhibitors (4H-31-benzoxazin-4-ones, 4H-3,1-benzoxazin-4-thiones, quinazolin-4-ones, quinazolin-4-thiones, benzothiazin-4-ones, imidazolyl-boronic acid-derived peptide analogues TFPI-derived peptides, naphthalene-2-sulfonic acid {1-[3-(aminoiminomethyl)-benzyl]-2-oxo-pyrrolidin-3-(S)-yl} amide trifluoroacetate, dibenzofuran-2-sulfonic acid {1-[3-(aminomethyl)-benzyl]-5-, oxo-pyrrolidin-3-yl}-amide, tolulene-4-sulfonic acid {1-[3-(aminoiminomethyl)-benzyl]-2-oxo-pyrrolidin-3-(S)-yl}-amide trifluoroacetate, 3,4-dihydro-1 H-isoquinoline-2-sulfonic acid {1-[3-(aminoiminomethyl)-benzyl]-2-oxo-pyrrolin-3-(S)-yl}-amide trifluoroacetate); Factor Xa inhibitors (disubstituted pyrazolines, disubstituted triazolines, substituted n-[(aminoiminomethyl)phenyl] propylamides, substituted n-[(aminomethyl)phenyl] propylamides, tissue factor pathway inhibitor (TFPI), low molecular weight heparins, heparinoids, benzimidazolines, benzoxazolinones, benzopiperazinones, indanones, dibasic (amidinoaryl) propanoic acid derivatives, amidinophenyl-pyrrolidines, amidinophenyl-pyrrolines, amidinophenyl-isoxazolidines, amidinoindoles, amidinoazoles, bis-arlysulfonylaminobenzamide derivatives, peptidic Factor Xa inhibitors).

The compositions, therapeutic combinations or methods of the present invention can further comprise one or more cardiovascular agents which are chemically different from the compounds of Formula (I) discussed above, for example, they contain one or more different atoms, have a different arrangement of atoms or a different number of one or more atoms than the compounds of Formula (I) discussed above. Useful cardiovascular agents include but are not limited to calcium channel blockers (clentiazem maleate, amlodipine besylate, isradipine, nimodipine, felodipine, nilvadipine, nifedipine, teludipine hydrochloride, diltiazem hydrochloride, belfosdil, verapamil hydrochloride, fostedil); adrenergic blockers (fenspiride hydrochloride, labetalol hydrochloride, proroxan, alfuzosin hydrochloride, acebutolol, acebutolol hydrochloride, alprenolol hydrochloride, atenolol, bunolol hydrochloride, carteolol hydrochloride, celiprolol hydrochloride, cetamolol hydrochloride, cicloprolol hydrochloride, dexpropranolol hydrochloride, diacetolol hydrochloride, dilevalol hydrochloride, esmolol hydrochloride, exaprolol hydrochloride, flestolol sulfate, labetalol hydrochloride, levobetaxolol hydrochloride, levobunolol hydrochloride, metalol hydrochloride, metoprolol, metoprolol tartrate, nadolol, pamatolol sulfate, penbutolol sulfate, practolol, propranolol hydrochloride, sotalol hydrochloride, timolol, timolol maleate, tiprenolol hydrochloride, tolamolol, bisoprolol, bisoprolol fumarate, nebivolol); adrenergic stimulants; angiotensin converting enzyme (ACE) inhibitors (benazepril hydrochloride, benazeprilat, captopril, delapril hydrochloride, fosinopril sodium, libenzapril, moexipril hydrochloride, pentopril, perindopril, quinapril hydrochloride, quinaprilat, ramipril, spirapril hydrochloride; spiraprilat, teprotide, enalapril maleate, lisinopril, zofenopril calcium, perindopril erbumine); antihypertensive agents (althiazide, benzthiazide, captopril, carvedilol, chlorothiazide sodium, clonidine hydrochloride, cyclothiazide, delapril hydrochloride, dilevalol hydrochloride, doxazosin mesylate, fosinopril sodium, guanfacine hydrochloride, methyldopa, metoprolol succinate, moexipril hydrochloride, monatepil maleate, pelanserin hydrochloride, phenoxybenzamine hydrochloride, prazosin hydrochloride, primidolol, quinapril hydrochloride, quinaprilat, ramipril, terazosin hydrochloride, candesartan, candesartan cilexetil, telmisartan, amlodipine besylate, amlodipine maleate, bevantolol hydrochloride), for example HYZAAR® or COZAAR® antihypertensive agents available from Merck & Co., Inc.; angiotensin II receptor antagonists (candesartan, irbesartan, losartan potassium, candesartan cilexetil, telmisartan); anti-anginal agents (amlodipine besylate, amlodipine maleate, betaxolol hydrochloride, bevantolol hydrochloride, butoprozine hydrochloride, carvedilol, cinepazet maleate, metoprolol succinate, molsidomine, monatepil maleate, primidolol, ranolazine hydrochoride, tosifen, verapamil hydrochloride); coronary vasodilators (fostedil, azaclorzine hydrochloride, chromonar hydrochloride, clonitrate, diltiazem hydrochloride, dipyridamole, droprenilamine, erythrityl tetranitrate, isosorbide dinitrate, isosorbide mononitrate, lidoflazine, mioflazine hydrochloride, mixidine, molsidomine, nicorandil, nifedipine, nisoldipine, nitroglycerine, oxprenolol hydrochloride, pentrinitrol, perhexiline maleate, prenylamine, propatyl nitrate; terodiline hydrochloride, tolamolol, verapamil); diuretics (the combination product of hydrochlorothiazide and spironolactone and the combination product of hydrochlorothiazide and triamterene).

The compositions, therapeutic combinations or methods of the present invention can further comprise one or more antidiabetic medications for reducing blood glucose levels in a human. Useful antidiabetic medications include, but are not limited to, drugs that reduce energy intake or suppress appetite, drugs that increase energy expenditure and nutrient-partitioning agents. Suitable antidiabetic medications include, but are not limited to, sulfonylurea (such as acetohexamide, chlorpropamide, gliamilide, gliclazide, glimepiride, glipizide, glyburide, glibenclamide, tolazamide, and tolbutarnide), meglitinide (such as repaglinide and nateglinide), biguanide (such as metformin and buformin), alpha-glucosidase inhibitor (such as acarbose, miglitol, camiglibose, and voglibose), certain peptides (such as amlintide, pramlintide, exendin, and GLP-1 agonistic peptides), and orally administrable insulin or insulin composition for intestinal delivery thereof. Generally, a total dosage of the above-described antidiabetic medications can range from 0.1 to 1,000 mg/day in single or 2-4 divided doses.

The compositions, therapeutic combinations or methods of the present invention can further comprise one or more treatments for Alzheimer's Disease which are chemically different from the compounds of Formula (I). Non-limiting examples of suitable treatments which can be useful in treating Alzheimer's Disease include administration of one or more of the following: cholinesterase inhibitors, muscarinic receptor agonists, M2 muscarinic receptor antagonists, acetylcholine release stimulators, choline uptake stimulators, nicotinic cholinergic receptor agonists, anti-Aβ vaccines, γ-secretase inhibitors, β-secretase inhibitors, amyloid aggregation inhibitors, amyloid precursor protein antisense oligonucleotides, monoamine reuptake inhibitors, human stem cells, gene therapy, nootropic agents, AMPA receptor ligands, growth factors or growth factor receptor agonists, anti-inflammatory agents, free radical scavengers, antioxidants, superoxide dismutase stimulators, calcium channel blockers, apoptosis inhibitors, caspase inhibitors, monoamine oxidase inhibitors, estrogens and estrogen receptor ligands, NMDA receptor antagonists, Jun N-terminal kinase (JNK) inhibitors, copper/zinc chelators, 5-HT1 a receptor agonists, NGF stimulators, neuroprotective agents, H3 histamine receptor antagonists, calpain inhibitors, poly ADP ribose polymerase inhibitors, prolylendopeptidase inhibitors, calcium modulators, corticortropin releasing factor receptor antagonists, corticortropin releasing factor binding protein inhibitors, GABA modulators, GABA-A receptor antagonists, GABA-B receptor antagonists, neuroimmunophilin ligands, sigma receptor ligands, galanin receptor ligands, imidazoline/alpha adrenergic receptor antagonists, vasoactive intestinal peptide receptor agonists, benzodiazepine receptor inverse agonists, cannabinoid receptor agonists, thyrotropin releasing hormone receptor agonists, protein kinase C inhibitors, 5-HT3 receptor antagonists, prostaglandin receptor antagonists, topoisomerase II inhibitors, steroid receptor ligand, nitric oxide modulators, RAGE inhibitors, dopamine receptor agonists, and combinations thereof.

Mixtures of any of the pharmacological or therapeutic agents described above can be used in the compositions and therapeutic combinations of the present invention.

The pharmaceutical treatment compositions (formulations or medicaments) and therapeutic combinations of the present invention can further comprise one or more pharmaceutically acceptable carriers, one or more excipients and/or one or more additives. As used herein, the term "composition" is intended to encompass a product comprising the specified ingredients in the specified amounts, as well as any product which results, directly or indirectly, from combination of the specified ingredients in the specified amounts.

Non-limiting examples of pharmaceutically acceptable carriers include solids and/or liquids such as ethanol, glycerol, water and the like. The amount of carrier in the treatment composition can range from about 5 to about 99 weight percent of the total weight of the treatment composition or therapeutic combination. Non-limiting examples of suitable pharmaceutically acceptable excipients and additives include non-toxic compatible fillers, binders such as starch, disintegrants, buffers, preservatives, anti-oxidants, lubricants, flavorings, thickeners, coloring agents, emulsifiers and the like. The amount of excipient or additive can range from about 0.1 1 to about 90 weight percent of the total weight of the treatment composition or therapeutic combination. One skilled in the art would understand that the amount of carrier(s), excipients and additives (if present) can vary.

The treatment compositions of the present invention can be administered in any conventional dosage form, preferably an oral dosage form such as a capsule, tablet, powder, cachet, suspension or solution. The formulations and pharmaceutical compositions can be prepared using conventional pharmaceutically acceptable and conventional techniques. Several examples of preparation of dosage formulations are provided below.

The following formulation exemplifies a dosage form of this invention. In the formulation, the term "Active Compound I" designates a compound of Formula I described herein above.

### EXAMPLE

| No. | Tablets | |
|---|---|---|
| | Ingredient | mg/tablet |
| 1 | Active Compound I | 20 |
| 2 | Lactose monohydrate NF | 55 |
| 3 | Microcrystalline cellulose NF | 20 |
| 4 | Povidone (K29-32) USP | 4 |
| 5 | Croscarmellose sodium NF | 8 |
| 6 | Sodium lauryl sulfate | 2 |
| 7 | Magnesium stearate NF | 1 |
| | Total | 110 |

### Method of Manufacture

Mix Item No. 4 with purified water in suitable mixer to form binder solution. Spray the binder solution and then water over Items 1, 2, 6 and a portion of Item 5 in a fluidized bed processor to granulate the ingredients. Continue fluidization to dry the damp granules. Screen the dried granules and blend with Item No. 3 and the remainder of Item 5. Add Item No. 7 and mix. Compress the mixture to appropriate size and weight on a suitable tablet machine.

Since the present invention relates to treating conditions as discussed above, such as reducing the plasma sterol (especially cholesterol) concentrations or levels by treatment with a combination of active ingredients wherein the active ingredients may be administered separately, the invention also relates to combining separate pharmaceutical compositions in kit form. That is, a kit is contemplated wherein two separate units are combined: a pharmaceutical composition comprising at least one compound of Formula (I) and a separate pharmaceutical composition comprising at least one other therapeutic agent as described above. The kit will preferably include directions for the administration of the separate components. The kit form is particularly advantageous when the separate components must be administered in different dosage forms (e.g., oral and parenteral) or are administered at different dosage intervals.

The treatment compositions and therapeutic combinations of the present invention can inhibit the intestinal absorption of cholesterol in mammals, as shown in the Example below, and can be useful in the treatment and/or prevention of conditions, for example vascular conditions, such as atherosclerosis, hypercholesterolemia and sitosterolemia, stroke, obesity and lowering of plasma levels of cholesterol in mammals, in particular in mammals.

In another embodiment of the present invention, the compositions and therapeutic combinations of the present invention can inhibit sterol absorption or reduce plasma concentration of at least one sterol selected from the group consisting of phytosterols (such as sitosterol, campesterol, stigmasterol and avenosterol), 5α-stanols (such as cholestanol, 5α-campestanol, 5α-sitostanol), cholesterol and mixtures thereof. The plasma concentration can be reduced by administering to a mammal in need of such treatment an effective amount of at least one treatment composition or therapeutic combination comprising a compound of Formula (I) described above. The reduction in plasma concentration of sterols can range from about 1 to about 70 percent, and preferably about 10 to about 50 percent. Methods of measuring serum total blood cholesterol and total LDL cholesterol are well known to those skilled in the art and for example include those disclosed in PCT WO 99/38498 at page 11. Methods of determining levels of other sterols in serum are disclosed in H. Gylling et al., "Serum Sterols During Stanol Ester Feeding in a Mildly Hypercholesterolemic Population", J. Lipid Res. 40: 593-600 (1999).

Illustrating the invention are the following examples which, however, are not to be considered as limiting the invention to their details. Unless otherwise indicated, all parts and percentages in the following examples, as well as throughout the specification, are by weight.

### EXAMPLE

### Hypothetical In Vivo Evaluation

The hypercholesterolemic Golden Syrian hamster can be used as the in vivo model to evaluate the oral potency and in vivo efficacy of cholesterol absorption inhibitors. Hamsters would be fed a cholesterol-containing diet for 7 days, which results in an increase in hepatic cholesteryl esters. A compound which blocks intestinal cholesterol absorption would reduce the accumulation of hepatic cholesteryl ester levels.

Male Golden Syrian hamsters (Charles River Labs, Wilmington, MA.) would be fed Wayne rodent chow until study onset. At study onset (Day 1) animals would be separated into groups (n=4-6/group) and fed chow supplemented with 0.5% by weight of cholesterol (Research Diets Inc., New Brunswick, NJ). One group of hamsters would receive a dosage of 3 mg/kg of body weight of any one of the compounds of Formulae (II), (III), (IV) or (V) administered once daily for 7 days, starting on Day 1 via oral gavage in 0.2 ml corn oil. The control group of hamsters would receive placebo corn oil in the same amount on the same schedule. On Day 7 liver samples would be taken for neutral lipid analyses. Samples of liver would be lipid extracted. Lipid extracts would be dried under nitrogen into HPLC sample vials, resuspended in hexane and injected onto a Zorbax Sil (4.6 x 25 cm) silica column. Chromatography would be performed using an isocratic mobile phase containing 98.8% hexane and 1.2% isopropanol at a flow rate of 2 ml/min. Lipids can be detected by absorbance at 206 nm and quantitated by computer integration (System Gold, Beckman) of elution profiles. Cholesterol concentrations can be determined by the use of a response factor derived from a standard curve using known amounts of cholesterol. Cholesteryl ester content of liver-derived samples can be derived from a standard curve constructed using known amounts of cholesteryl oleate. Cholesteryl oleate can be used as the standard since this is the major cholesteryl ester species present in the liver and this specific cholesteryl ester has an extinction coefficient that approximates that of a weighted average for all the cholesteryl esters present in the liver.

The reduction of hepatic cholesteryl ester accumulation is utilized as a marker for cholesterol absorption inhibition.

It will be appreciated by those skilled in the art that changes could be made to the embodiments described above without departing from the broad inventive concept thereof. It is understood, therefore, that this invention is not limited to the particular embodiments disclosed, but it is intended to cover modifications that are within the scope of the invention, as defined by the appended claims.

## Claims

1. A compound represented by the structural formula (IA): 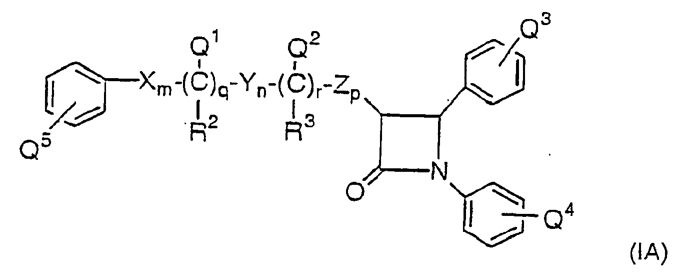 or pharmaceutically acceptable enantiomers, stereoisomers, rotamers, tautomers, racemates salts, solvates or esters of the compound of Formula (1A)
wherein in Formule (IA) above:
X, Y and Z can be the same or different and each is independently selected from the group consisting of -CH₂-, -CH(alkyl)- and -C(alkyl)₂-;
Q¹ and Q² can be the same or different and each is independently selected from the group consisting of H, -(C₀-C₃₀ alkylene)-G, -OR⁶, -OC(O)R⁶, -OC(O)OR⁹, -OC(O)NR⁶R⁷, and -L-M;
Q³ is 1 to 5 substituents independently selected from the group consisting of alkyl, alkenyl, alkynyl, -(C₀-C₃₀ alkylene)-G, -(C₀-C₁₀ alkylene)-OR⁶, -(C₀-C₁₀ alkylene)-C(O)R⁶, -(C₀-C₁₀ alkylene)-O(O)OR⁶, -(C₀-C₁₀ alkylene)-OC(O)R⁶; -(C₀-C₁₀ alkylene)-OC(O)OR⁹, -CH=CH-C(O)R⁶, -CH=CH-C(O)OR⁶, -C≡C-C(O)OR⁶, -C≡C-C(O)R⁶, -O-(C₁-C₁₀ alkylene)-OR⁶, -O-(C₁-C₁₀ alkylene)-C(O)R⁶, -O-(C₁-C₁₀ alkylene)-C(O)OR⁶, -CN, -O-(C₁-C₁₀ alkylene)-C(O)NR⁶R⁷, -O-(C₀-C₁₀ alkylene)-C(O)NR⁶NR⁷C(O)OR⁶, -O-(C₁-C₁₀ alkylene)-C(O)(aryl)-N-N=N⁻, -OC(O)-(C₁-C₁₀ alkylene)-C(O)OR⁵, -(C₀-C₁₀ alkylene)-C(O)NR⁶R⁷, -(C₀-C₁₀ alkylene)-OC(O)NR⁶R⁷, -NO₂, -(C₀-C₁₀ alkylene)-NR⁶R⁷, -O-(C₂-C₁₀ alkylene)-NR⁶R⁷, -NR⁶C(O)R⁷, -NR⁶C(O)OR⁹, -NR⁶C(O)NR⁷R⁸, -NR⁶S(O)_{c-2}R⁹, -N(S(O)₀₋₂R⁹)₂, -CHNOR⁶, -C(O)NR⁶R⁷, -C(O)NR⁶NR⁶R⁷, -S(O)₀₋₂NR⁶R⁷, -S(O)₀₋₂R⁹, -O-C(O)-(C₁-C₁₀ alkylene)-C(O)NR⁶R⁷, -OC(O)-(C₁-C₁₀ alkylene)-NR⁶C(O)O-(alkylaryl), -P(O)(OR¹⁰)₂, -(C₁-C₁₀ alkylene)-OSi(alkyl)₃, -CF₃, -OCF₃, halo, alkoxyalkoxy, alkoxyalkoxyalkoxy, alkoxycarbonylalkoxy, alkoxyarylalkoxy, alkoxyiminoalkyl, alkyldioyl, allyloxy, aryl, arylalkyl, aryloxy, arylalkoxy, aroyl, aroyloxy, aroylaroyloxy, arylalkoxycarbonyl, benzoylbenzoyloxy, heteroaryl, heteroarylalkyl, heteroarylalkoxy, dioxolanyl, heterocyclyl, heterocyclylalkyl, heterocyclylcarbonyl, heterocyclylcarbonylalkoxy and -L-M;
Q⁴ is 1 to 5 substituents independently selected from the group consisting of alkyl, alkenyl, alkynyl, -(C₀-C₃₀ alkylene)-G, -(C₀-C₁₀ alkylene)-OR⁶, -(C₀-C₁₀ alkylene)-C(O)R⁶, -(C₀-C₁₀ alkylene)-C(O)OR⁶, -(C₀-C₁₀ alkylene)-OC(O)R⁶, -(C₀-C₁₀ alkylene)-OC(O)OR⁹, -CH=CH-C(O)R⁶, -CH=CH-C(O)OR⁶, -C≡C-C(O)OR⁶, -C≡C-C(O)R⁶, -O-(C₁-C₁₀ alkylene)-OR⁶, -O-(C₁-C₁₀ alkylene)-C(O)R⁶, -O-(C₁-C₁₀ alkylene)-C(O)OR⁶, -CN, -O-(C₁-C₁₀ alkylene)-C(O)NR⁶R⁷, -O-(C₀-C₁₀ alkylene)-C(O)NR⁶NR⁷C(O)OR⁶, -O-(C₁-C₁₀ alkylene)-C(O)(aryl)-N-N=N⁻, -OC(O)-(C₁-C₁₀ alkylene)-C(O)OR⁶, -(C₀-C₁₀ alkylene)-C(O)NR⁶R⁷, -(C₀-C₁₀ alkylene)-OC(O)NR⁶R⁷, -NO₂, -(C₀-C₁₀ alkylene)-NR⁶R⁷, -O-(C₂-C₁₀ alkylene)-NR⁶R⁷, -NR⁶C(O)R⁷, -NR⁶C(O)OR⁹, -NR⁶C(O)NR⁷R⁸, -NR⁶S(O)₀₋₂R⁹, -N(S(O)₀₋₂R⁹)₂, -CHNOR⁶, -C(O)NR⁶R⁷, -C(O)NR⁶NR⁶R⁷, -S(O)₀₋₂NR⁶R⁷, -S(O)₀₋₂R⁹, -O-O(O)-(C₁-C10) alkylene)-C(O)NR⁶R⁷, -OC(O)-(C₁-C₁₀ alkylene)-NR⁶C(O)O-(alkylaryl), -P(O)(OR¹⁰)₂, -(C₁-C₁₀ alkylene)-OSi(alkyl)₃, -CF₃, -OCF₃, halo, alkoxyalkoxy, alkoxyalkoxyalkoxy, alkoxycarbonylalkoxy, alkoxyanylalkoxy, alkoxyiminoalkyl, alkyldioyl, allyloxy, aryl, arylalkyl, aryloxy, arylalkoxy, aroyl, aroyloxy, aroylaroyloxy, arylalkoxycarbonyl, benzoylbenzoyloxy, heteroaryl, heteroarylalkyl, heteroarylalkoxy, dioxolanyl, heterocyclyl, heterocyclylalkyl, heterocyclylcarbonyl, heterocyclylcarbonylalkoxy and -L-M;
Q⁵ is 1 to 5 substituents independently selected from the group consisting of alkyl, alkenyl, alkynyl, -(C₀-C₃₀ alkylene)-G, -(C₀-C₁₀ alkylene)-OR⁶, -(C₀-C₁₀ alkylene)-C(O)R⁶, -(C₀-C₁₀ alkylene)-C(O)OR⁶, -(C₀-C₁₀ alkylene)-OC(O)R⁶, -(C₀-C₁₀ alkylene)-OC(O)OR⁹, -CH=CH-C(O)R⁶, -CH=CH-C(O)OR⁶, -C≡C-C(O)OR⁶, -C≡C-C(O)R⁶, -O-(C₁-C₁₀ alkylene)-OR⁶, -O-(C₁-C₁₀ alkylene)-C(O)R⁶, -O-(C₁-C₁₀ alkylene)-C(O)OR⁶, -CN, -O-(C₁-C₁₀ alkylene)-C(O)NR⁶R⁷, -O-(C₀-C₁₀ alkylene)-C(O)NR⁶NR⁷C(O)OR⁶, -O-(C₁-C₁₀ alkylene)-C(O)(aryl)-N-N=N⁻, -OC(O)-(C₁-C₁₀ alkylene)-C(O)OR⁶, -(C₀-C₁₀ alkylene)-C(O)NR⁶R⁷, -(C₀-C₁₀ alkylene)-OC(O)NR⁶R⁷, -NO₂, -(C₀-C₁₀ alkylene)-NR⁶R⁷, -O-(C₂-C₁₀ alkylene)-NR⁶R⁷, -NR⁶C(O)R⁷, -NR⁶C(O)OR⁹, -NR⁶C(O)NR⁷R⁸, -NR⁶S(O)₀₋₂R⁹, -N(S(O)₀₋₂R⁹)₂, -CHNOR⁶, -C(O)NR⁶R⁷, -C(O)NR⁶NR⁶R⁷, -S(O)₀₋₂NR⁶R⁷, -S(O)₀₋₂R⁹, -O-C(O)-(C₇-C10 alkylene)-C(O)NR⁶R⁷, -OC(O)-(C₁-C₁₀ alkylene)-NR⁶C(O)C)-(alkylaryl), -P(O)(OR¹⁰)₂, -(C₁-C₁₀ alkylene)-OSi(alkyl)₃, -CF₃, -OCF₃, halo, alkoxyalkoxy, alkoxyalkoxyalkoxy, alkoxycarbonylalkoxy, alkoxyarylalkoxy, alkoxyiminoalkyl, alkyldioyl, alkyloxy, aryl, arylalkyl, aryloxy, arylalkoxy, aroyl, aroyloxy, aroylaroyloxy, arylalkoxycarbonyl, benzoylbenzoyloxy, heteroaryl, heteroarylalkyl, heteroarylalkoxy, dioxolanyl, heterocyclyl, heterocyclylalkyl, heterocyclylcarbonyl, heterocyclylcarbonylalkoxy and -L-M;
wherein optionally one or more carbon atoms of the -(C₀-C₃₀ alkylene)- radical of Q¹, Q², Q³, Q⁴ and Q⁵ is independently replaced by -O-, -C(O)-, -CH=CH-, -C≡C-, -N(alkyl)-, -N(alkylary)- or -NH-;
G. is setected from the group consisting of a sugar residus, disugar residue, trisugar residue, tetrasugar residue, sugar acid, amino sugar, amino acid residue, oligopeptide residue comprising 2 to 9 amino acids, trialkylammoniumalkyl radical and -S(O)₂-OH, wherein optionally the sugar residue, disugar resiclue, trisugar residue, tetrasugar residue, sugar acid, amino sugar, amino acid residue or oligopeptide residue of G. is substituted with -L-M;
L is selected from the group consisting of 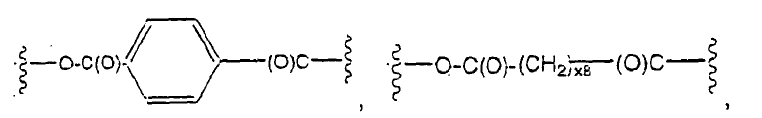 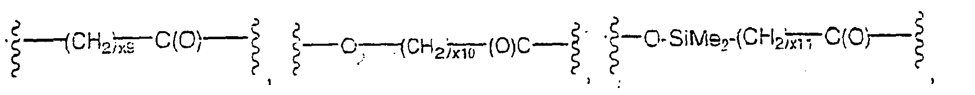 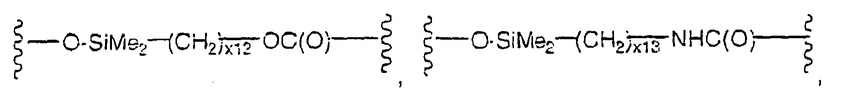 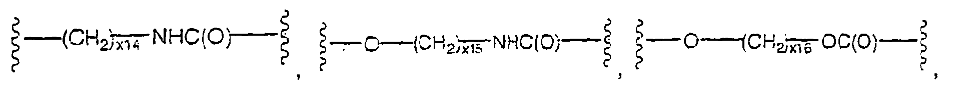 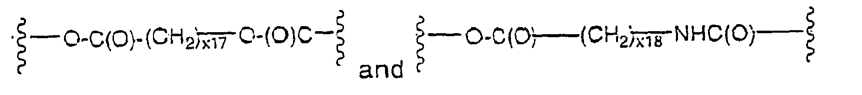 wherein Me is methyl;
M is selected from the group of moieties consisting of 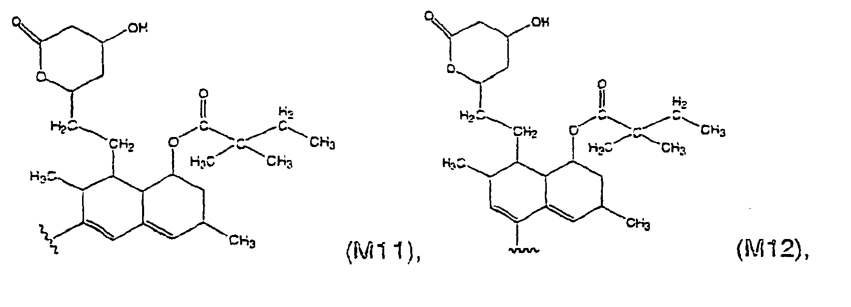 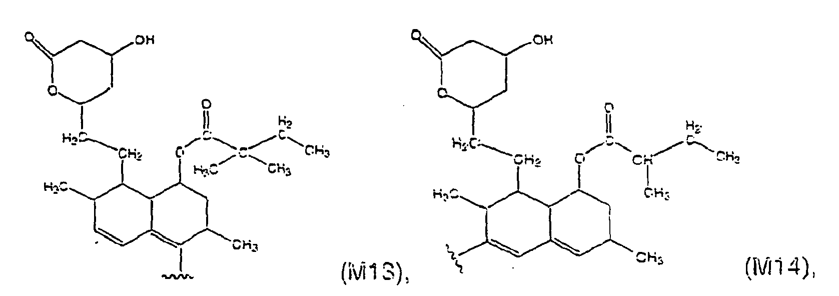 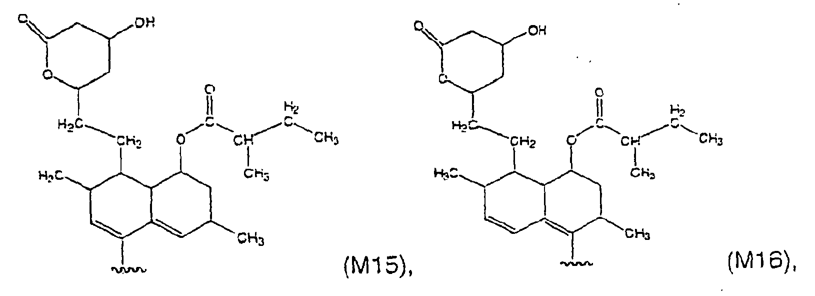 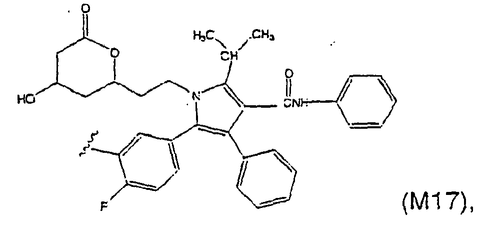 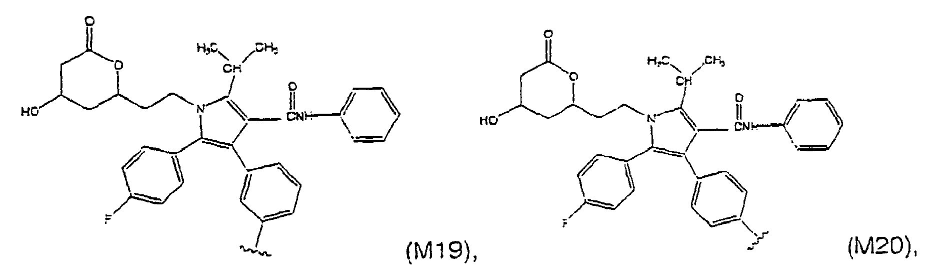 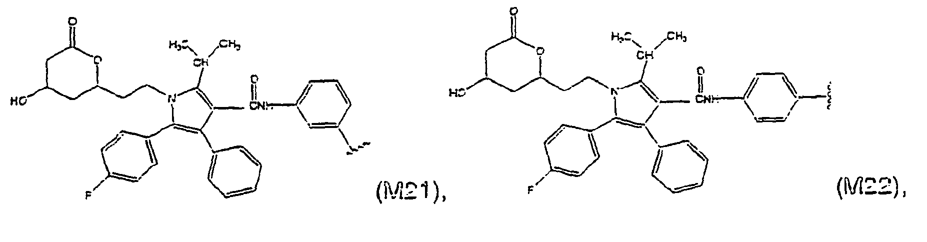 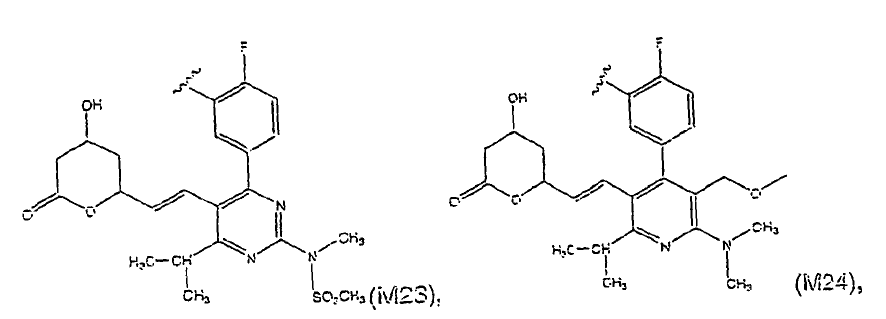 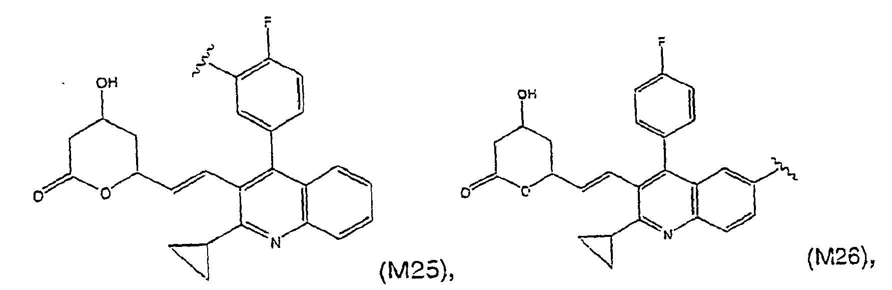 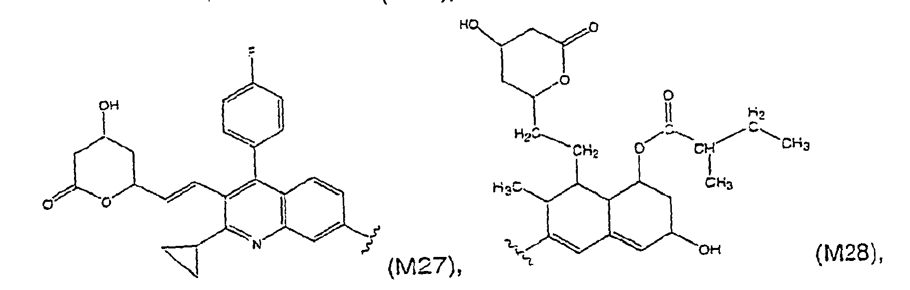 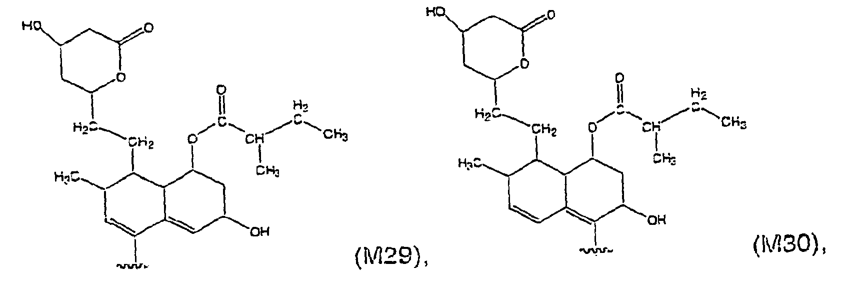 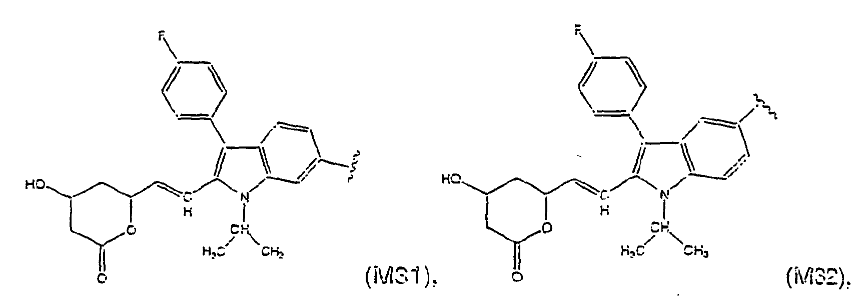 and 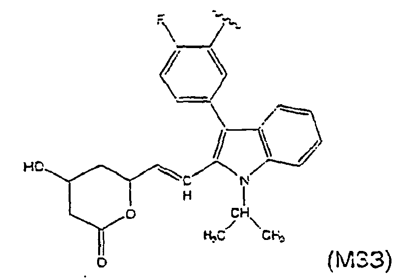 and pharmaceutically acceptable salts of moieties (M1) to (M33);
R² and R³ can be the same or different and each is independently selected from the group consisting of hydrogen, alkyl and aryl;
R⁶, R⁷ and R⁸ can be the same or different and each is independently selected from the group consisting of hydrogen, alkyl, aryl and arylalkyl; and
each R⁹ is independently alkyl, aryl or arylalkyl.
each R¹⁰ is independently H or alkyl;
q is 0 or 1;
r is 0 or 1;
m, n and p are independently selected from 0, 1, 2, 3 or 4; provided that at least one of q and r is 1, and the sum of m, n, p, q and r is 1, 2, 3, 4, 5 or 6; and provided that when p is 0 and r is 1, the sum of m, q and n is 1, 2, 3, T or 5:
x8 is 1 to 10;
x9 is 1 to 10;
x10 is 1 to 10;
x11 is 1 to 10;
x12 ts to 10;
a:?3a'1 tolC;
x 14 is 1 to 10;
x15 is 1 to 10; and
x16 is i to 10;
x17 is i to 10; and
x18 is 1 to 10;
with the proviso that at least one of Q¹, Q², Q³, Q⁴ and Q⁵ is -L-M or the sugar residue, disugar residue, trisugar residue, tetrasugar residue, sugar acid, amino sugar, amino acid residue or oligopeptide residue of G is substituted with -L-M,
wherein the following definitions apply:
The term "alkyl" means an aliphatic hydrocarbon group that can be straight or branched and comprises 1 to about 20 carbon atoms in the chain, optionally substituted by one or more substituents independently selected from the group consisting of halo, aryl, cycloalkyl, cyano, hydroxy, alkoxy, alkylthio, amino, -NH(alkyl), -NH(cycloalkyl), -N(alkyl)₂ (which alkyls can be the same or different), carboxy and - C(O)O-alkyl. Non-limiting examples of suitable alkyl groups include methyl, ethyl, n-propyl, isopropyl, n-butyl, t-butyl, n-pentyl, heptyl, nonyl, decyl, fluoromethyl, trifluoromethyl and cyclopropylmethyl;
"Alkenyl" means an aliphatic hydrocarbon group (straight or branched carbon chain) comprising one or more double bonds in the chain and which can be conjugated or unconjugated, optionally substituted by one or more substituents independently selected from the group consisting of halo, alkyl, aryl, cycloalkyl, cyano and alkoxy;
"Alkoxy" means an alkyl-O- group in which the alkyl group is as previously described;
"Alkoxyarylalkoxy" means an alkyl-O-aryl-alkylene-O- group in which the alkyl, alkylene and aryl groups are as previously described;
"Alkoxycarbonylalkoxy" means an alkyl-O-C(O)-alkylene-O- group in which the alkyl and alkylene groups are as previously described;
"Alkoxyiminoalkyl" means an alkyl-O-N=CH-alkylene- group in which the alkyl and alkylene groups are as previously described;
"Alkyldioyl" means an ROC(O)-alkylene-C(O)-O- group in which R is alkyl or H and the alkylene group is as previously described.
"Alkynyl" means an aliphatic hydrocarbon group comprising at least one carbon-carbon triple bond and which may be straight or branched and comprising about 2 to about 15 carbon atoms in the chain, optionally substituted by one or more substituents which may be the same or different, each substituent being independently selected from the group consisting of alkyl, aryl and cycloalkyl;
"Allyloxy" means H₂C=CH-O-.
"Aryl" means an aromatic monocyclic or multicyclic ring system comprising about 5 to about 14 carbon atoms, optionally substituted with one or more "ring system substituents" which may be the same or different, and are as defined below; "Arylene" means a bivalent phenyl group, including ortho, meta and para-substitution;
"Aralkyl" or "arylalkyl" means an aryl-alkylene- group in which the aryl and alkylene are as previously described;
"Aryloxy" means an aryl-O- group in which the aryl group is as previously described;
"Aralkoxy" or "arylalkyloxy" means an aralkyl-O- group in which the aralkyl group is as previously described;
"Aroyl" means an aryl-C(O)- group in which the aryl group is as previously described;
"Aroyloxy" means an aroyl-O- group in which the aroyl group is as previously described;
"Cycloalkyl" means a non-aromatic mono- or multicyclic ring system comprising about 3 to about 10 carbon atoms, optionally substituted with one or more "ring system substituents" which may be the same or different, and are as defined below; "Cycloalkylene" refers to a corresponding bivalent ring, wherein the points of attachment to other groups include all positional isomers;
"Dioxolanyl" means 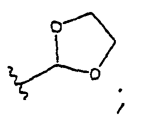
"Halo" refers to fluorine, chlorine, bromine or iodine radicals;
"Heteroaryl" means a monocyclic or multicyclic aromatic ring system of about 5 to about 14 ring atoms, in which one or more of the atoms in the ring system is/are atoms other than carbon, and the heteroatom(s) interrupt a carbocyclic ring structure and have a sufficient number of delocalized pi electrons to provide aromatic character, provided that the rings do not contain adjacent oxygen and/or sulfur atoms, the heteroaryl group being optionally substituted by one or more "ring system substituents" which may be the same or different, and are as defined below; the prefix aza, oxa or thia before the heteroaryl root name means that at least a nitrogen, oxygen or sulfur atom respectively, is present as a ring atom; a nitrogen atom of a heteroaryl can be oxidized to form the corresponding N-oxide;
"Heteroarylalkyl" or "heteroaralkyl" means a heteroaryl-alkylene- group in which the heteroaryl and alkyl are as previously described; "Heteroarylalkoxy" means a heteroaryl-alkylene-O-group in which the heteroaryl and alkylene are as previously described;
"Heterocyclyl" means a non-aromatic saturated monocyciic or multicyclic ring system comprising about 3 to about 10 ring atoms, in which one or more of the atoms in the ring system is an element other than carbon, provided that there are no adjacent oxygen and/or sulfur atoms present in the ring system; the prefix aza, oxa or thia before the heterocyclyl root name means that at least a nitrogen, oxygen or sulfur atom respectively is present as a ring atom; the heterocyclyl is optionally substituted by one or more "ring system substituents" which may be the same or different, and are as defined below; the nitrogen or sulfur atom of the heterocyclyl can be optionally oxidized to the corresponding N-oxide, S-oxide or S,S-dioxide;
"Heterocyclylalkyl" means a heterocyoyl-alkylene- group in which the hetarocyciyl and alkylene groups are as previously described; "Heterocyclylcarbonyl" means a heterocyclyl-C(O)- group in which the hsterocyclyl is as previously described; "Heterocyclylcarbonylalkoxy" means a heterocyciyl-C(O)-alkoxy- group in which the heterocyclyl and alkoxy are as previously described;
"Ring system substituent" means a substituent attached to an aromatic or nonaromatic ring system that, for example, replaces an available hydrogen on the ring system; ring system substituents may be the same or different, each being independently selected from the group consisting of aryl, heteroaryl, aralkyl, alkylaryl, aralkenyl, heteroaralkyl, alkylheteroaryl, heteroaralkenyl, hydroxy, hydroxyalkyl, alkoxy, aryloxy, aralkoxy, acyl, aroyl, halo, nitro, cyano, carboxy, alkoxycarbonyl, aryloxycarbonyl, aralkoxycarbonyl, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio, cycloalkyl, cycloalkenyl, heterocyclyl, heterocyclenyl, Y₁Y₂N-, Y₁Y₂N-alkyl-, Y₁Y₂NC(O)- and Y₁Y₂NSO₂-, wherein Y₁ and Y₂ may be the same or different and are independently selected from the group consisting of hydrogen, alkyl, aryl, and aralkyl;
"Sugar residue" means a moiety derived from an aldose or ketose that has 3 to 7 carbon atoms and may belong to the D or L series;
"Disugar residue" means a moiety derived from a sugar that can be hydrolyzed to two monosaccharide molecules;
"Trisugar residue" means a moiety derived from a sugar that can be hydrolyzed to three monosaccharide molecules; "Tetrasugar residue" means a moiety derived from a sugar that can be hydrolyzed to four monosaccharide molecules;
"Sugar acid" means an sugar residue, such as can be formed from glucuronic acid, galacturonic acid, gluconic acid, galactonic acid, mannonic acid, glucaric acid and galactaric acid;
"Amino sugar" means an amino-substituted sugar residue such as can be formed from glucosamine, galactosamine, glucamine or 3-amino-1,2-propanediol; and
"Amino acid residue" means a moiety derived from an amino acid. The amino acid moiety can be prepared from the D or L forms of the amino acid.

2. The compound according to claim 1, wherein m, n and r are each zero, q is 1, p is 2, and Z is -CH₂-.

3. The compound according to claim 1, wherein m, n and r are each zero, q is 1, p is 2, and Z is -CH₂-, Q¹ is -OR⁶, wherein R⁶ is hydrogen and Q⁵ is fluorine.

4. The compound according to claim 1, wherein R² and R³ are each preferably hydrogen.

5. The compound according to claim 1, wherein Q¹ and Q² are each independently selected from the group consisting of -OR⁶, -O(CO)R⁶, -O(CO)OR⁹ and -O(CO)NR⁶R⁷.

6. The compound according to claim 1, wherein Q⁴ is halo or -OR⁶.

7. The compound according to claim 1, wherein Q¹ is -OR⁶ wherein R⁶ is H.

8. The compound according to claim 1, wherein Q¹, Q², Q³, Q⁴ or Q⁵ is-L-M.

9. The compound according to claim 1 wherein Q¹, Q², Q³, Q⁴ or Q⁵ is -(C₀-C₃₀ alkylene)-G.

10. The compound according to claim 1, wherein G is selected from the group consisting of: 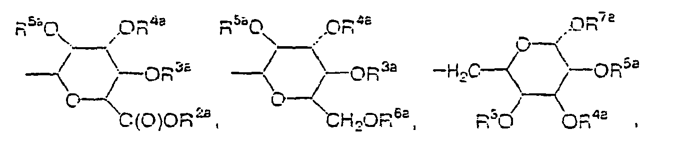 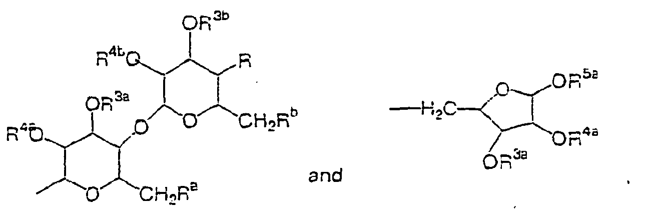
wherein R, R^{a} and R^{b} can be the same or different and each is independently selected from the group consisting of H, -OH, halo, -NH₂, azido, alkoxyalkoxy or -W-R³⁰;
W is independently selected from the group consisting of -NH-C(O)-, -O-C(O)-, -O-C(O)-N(R³¹)-, -NH-C(O)-N(R³¹)- and -O-C(S)-N(R³¹)-;
R^{2a} and R^{6a} can be the same or different and each is independently selected from the group consisting of H, alkyl, acetyl, aryl and arylalkyl;
R^{3a}, R^{4a}, R^{5a}, R^{7a}, R^{3b} and R^{4b} can be the same or different and each is independently, selected from the group consisting of H, alkyl, acetyl, arylalkyl,-C(O)alkyl and -C(O)aryl;
R³⁰ is independently selected from the group consisting of R³²-substituted T, R³²-substituted-T-alkyl, R³²-substituted-alkenyl, R³²-substituted-alkyl, R³²-substituted-cycloalkyl and R³²-substituted-cycloalkylalkyl;
R³¹ is independently selected from the group consisting of H and alkyl;
T is independently selected from the group consisting of phenyl, furyl, thianyl, pyrrolyl, oxazolyl, isoxazolyl, thiazolyl, isothiazolyl, benzothiazolyl, thiadiazolyl, pyrazolyl, imidazolyl and pyridyl;
R³² is 1 to 3 substituents which are each independently selected from the group consisting of H, halo, alkyl, -OH, phenoxy, -CF₃, -NO₂, alkoxy, methylenedioxy, oxo, alkylsulfanyl, alkylsulfinyl, alkylsulfonyl, -N(CH₃)₂, -C(O)-NHalkyl, -C(O)-N(alkyl)₂, -C(O)-alkyl, -C(O)-alkoxy and pyrrolidinylcarbonyl; or R³² is a covalent bond and R³¹, the nitrogen to which it is attached and R³² form a pyrrolidinyl, piperidinyl, N-methyl-piperazinyl, indolinyl or morpholinyl group, or a alkoxycarbonyl-substituted pyrrolidinyl, piperidinyl, N-methylpiperazinyl, indolinyl or morpholinyl group.

11. The compound according to claim 10 wherein G is selected from: 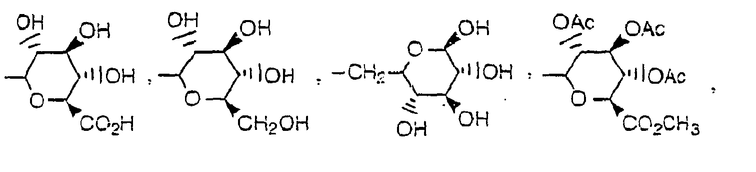 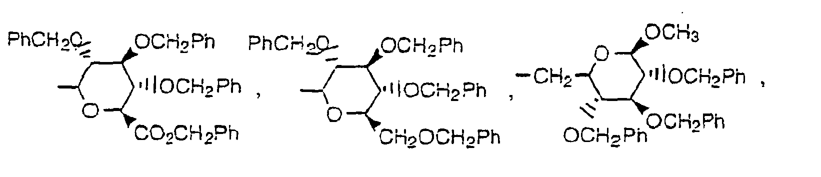 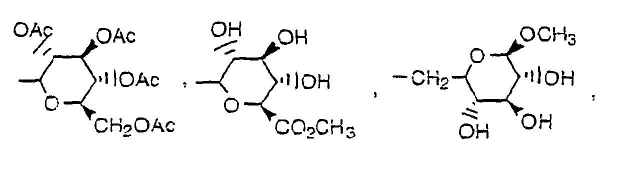 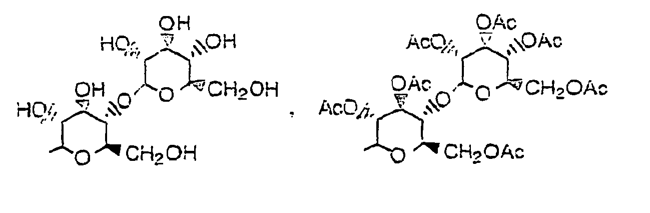 and 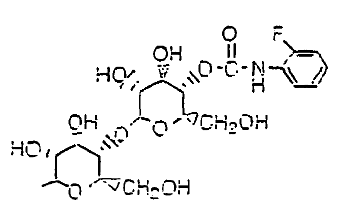 wherein Ac is acetyl and Ph is phenyl.

12. The compound according to claim 1 wherein optionally one or more carbon atoms of the -(C₀-C₃₀ alkylene)- radical oi Q¹, Q², Q³, Q⁴ and Q⁵ is independently replaced by -O-.

13. A pharmaceutical composition for use in the treatment or prevention of a vascular condition, diabetes, obesity, stroke, lowering a concentration of a sterol or stanol in plasma of a mammal, preventing demyelination or treating Alzheimer's disease and/or regulating levels of amyloid β peptides in a subject comprising a therapeutically effective amount of a compound of claim 1 in a pharmaceutical acceptable carrier.

14. A pharmaceutical composition comprising a cholesterol-lowering effective amount of a compound of claim 1 in a pharmaceutical acceptable carrier.

15. Use of a compound as defined in claim 1 for the manufacture of a medicament for use in treating or preventing a vascular condition, diabetes, obesity, stroke, lowering a concentration of a sterol or stanol in plasma of a mammal, preventing demyelination or treating Alzheimer's disease or regulating a level of an amyloid β peptide in a subject.

16. Use of a compound as defined in claim 1 for the manufacture of a medicament for use in lowering cholesterol level in plasma of a mammal in need of such treatment.

## Patentansprüche

1. Eine Verbindung, dargestellt durch die Strukturformel (IA): 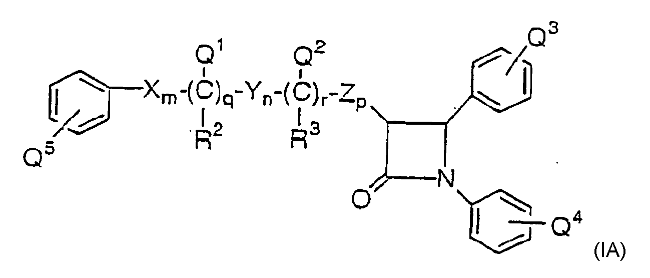 oder pharmazeutisch annehmbare Enantiomere, Stereoisomere, Rotamere, Tautomere, Racemate, Salze, Solvate oder Ester der Verbindung der Formel (1A),
wobei in der obigen Formel (IA):
X, Y und Z gleich oder verschieden sein können und jedes unabhängig ausgewählt ist aus der Gruppe, bestehend aus -CH₂-, -CH(Alkyl)- und -C(Alkyl)₂-,
Q¹ und Q² gleich oder verschieden sein können und jedes unabhängig ausgewählt ist aus der Gruppe, bestehend aus H, -(C₀-C₃₀-Alkylen)-G, -OR⁶, -OC(O)R⁶, -OC(O)OR⁹, -OC(O)NR⁶R⁷ und -L-M,
Q³ 1 bis 5 Substituenten ist, unabhängig ausgewählt aus der Gruppe, bestehend aus Alkyl, Alkenyl, Alkinyl, -(C₀-C₃₀-Alkylen)-G, -(C₀-C₁₀-Alkylen)-OR⁶, -(C₀-C₁₀-Alkylen)-C(O)R⁶, -(C₀-C₁₀-Alkylen)-C(O)OR⁶, -(C₀-C₁₀-Alkylen)-OC(O)R⁶, -(C₀-C₁₀-Alkylen)-OC(O)OR⁹, -CH=CH-C(O)R⁶, -CH=CH-C(O)OR⁶, -C≡C-C(O)OR⁶, -C≡C-C(O)R⁶, -O-(C₁-C₁₀-Alkylen)-OR⁶, -O-(C₁-C₁₀-Alkylen)-C(O)R⁶, -O-(C₁-C₁₀-Alkylen)-C(O)OR⁶, -CN, -O-(C₁-C₁₀-Alkylen)-C(O)NR⁶R⁷, -O-(C₀-C₁₀-Alkylen)-C(O)NR⁶NR⁷C(O)OR⁶, -O-(C₁-C₁₀-Alkylen)-C(O)(aryl)-N-N=N⁻, -OC(O)-(C₁-C₁₀-Alkylen)-C(O)OR⁶, -(C₀-C₁₀-Alkylen)-C(O)NR⁶R⁷, -(C₀-C₁₀-Alkylen)-OC(O)NR⁶R⁷, -NO₂, -(C₀-C₁₀-Alkylen)-NR⁶R⁷, -O-(C₂-C₁₀-Alkylen)-NR⁶R⁷, -NR⁶C(O)R⁷, -NR⁶C(O)OR⁹, -NR⁶C(O)NR⁷R⁸, -NR⁶S(O)₀₋₂R⁹, -N(S(O)₀₋₂R⁹)₂, -CHNOR⁶, -C(O)NR⁶R⁷, -C(O)NR⁶NR⁶R⁷, -S(O)₀₋₂NR⁶R⁷, -S(O)₀₋₂R⁹, -O-C(O)-(C₁-C₁₀-Alkylen)-C(O)NR⁶R⁷, -OC(O)-(C₁-C₁₀-Alkylen)-NR⁶C(O)O-(alkylaryl), -P(O)(OR¹⁰)₂, -(C₁-C₁₀-Alkylen)-OSi(alkyl)₃, -CF₃, -OCF₃, Halogen, Alkoxyalkoxy, Alkoxyalkoxyalkoxy, Alkoxycarbonylalkoxy, Alkoxyarylalkoxy, Alkoxyiminoalkyl, Alkyldioyl, Allyloxy, Aryl, Arylalkyl, Aryloxy, Arylalkoxy, Aroyl, Aroyloxy, Aroylaroyloxy, Arylalkoxycarbonyl, Benzoylbenzoyloxy, Heteroaryl, Heteroarylalkyl, Heteroarylalkoxy, Dioxolanyl, Heterocyclyl, Heterocyclylalkyl, Heterocyclylcarbonyl, Heterocyclylcarbonylalkoxy und -L-M,
Q⁴ 1 bis 5 Substituenten ist, unabhängig ausgewählt aus der Gruppe, bestehend aus Alkyl, Alkenyl, Alkinyl, -(C₀-C₃₀-Alkylen)-G, -(C₀-C₁₀-Alkylen)-OR⁶, -(C₀-C₁₀-Alkylen)-C(O)R⁶, -(C₀-C₁₀-Alkylen)-C(O)OR⁶, -(C₀-C₁₀-Alkylen)-OC(O)R⁶, -(C₀-C₁₀-Alkylen)-OC(O)OR⁹, -CH=CH-C(O)R⁶, -CH=CH-C(O)OR⁶, -C≡C-C(O)OR⁶, -C≡C-C(O)R⁶, -O-(C₁-C₁₀-Alkylen)-OR⁶, -O-(C₁-C₁₀-Alkylen)-C(O)R⁶, -O-(C₁-C₁₀-Alkylen)-C(O)OR⁶, -CN, -O-(C₁-C₁₀-Alkylen)-C(O)NR⁶R⁷, -O-(C₀-C₁₀-Alkylen)-C(O)NR⁶NR⁷C(O)OR⁶, -O-(C₁-C₁₀-Alkylen)-C(O)(aryl)-N-N=N⁻, -OC(O)-(C₁-C₁₀-Alkylen)-C(O)OR⁶, -(C₀-C₁₀-Alkylen)-C(O)NR⁶R⁷, -(C₀-C₁₀-Alkylen)-OC(O)NR⁶R⁷, -NO₂, -(C₀-C₁₀-Alkylen)-NR⁶R⁷, -O-(C₂-C₁₀-Alkylen)-NR⁶R⁷, -NR⁶C(O)R⁷, -NR⁶C(O)OR⁹, -NR⁶C(O)NR⁷R⁸, -NR⁶S(O)₀₋₂R⁹, -N(S(O)₀₋₂R⁹)₂, -CHNOR⁶, -C(O)NR⁶R⁷, -C(O)NR⁶NR⁶R⁷, -S(O)₀₋₂NR⁶R⁷, -S(O)₀₋₂R⁹, -O-C(O)-(C₁-C₁₀-Alkylen)-C(O)NR⁶R⁷, -OC(O)-(C₁-C₁₀-Alkylen)-NR⁶C(O)O-(alkylaryl), -P(O)(OR¹⁰)₂, -(C₁-C₁₀-Alkylen)-OSi(alkyl)₃, -CF₃, -OCF₃, Halogen, Alkoxyalkoxy, Alkoxyalkoxyalkoxy, Alkoxycarbonylalkoxy, Alkoxyarylalkoxy, Alkoxyiminoalkyl, Alkyldioyl, Allyloxy, Aryl, Arylalkyl, Aryloxy, Arylalkoxy, Aroyl, Aroyloxy, Aroylaroyloxy, Arylalkoxycarbonyl, Benzoylbenzoyloxy, Heteroaryl, Heteroarylalkyl, Heteroarylalkoxy, Dioxolanyl, Heterocyclyl, Heterocyclylalkyl, Heterocyclylcarbonyl, Heterocyclylcarbonylalkoxy und -L-M,
Q⁵ 1 bis 5 Substituenten ist, unabhängig ausgewählt aus der Gruppe, bestehend aus Alkyl, Alkenyl, Alkinyl, -(C₀-C₃₀-Alkylen)-G, -(C₀-C₁₀-Alkylen)-OR⁶, -(C₀-C₁₀-Alkylen)-C(O)R⁸, -(C₀-C₁₀-Alkylen)-C(O)OR⁶, -(C₀-C₁₀-Alkylen)-OC(O)R⁶, -(C₀-C₁₀-Alkylen)-OC(O)OR⁹, -CH=CH-C(O)R⁶, -CH=CH-C(O)OR⁶, -C≡C-C(O)OR⁶, -C≡C-C(O)R⁶, -O-(C₁-C₁₀-Alkylen)-OR⁶, -O-(C₁-C₁₀-Alkylen)-C(O)R⁶, -O-(C₁-C₁₀-Alkylen)-C(O)OR⁶, -CN, -O-(C₁-C₁₀-Alkylen)-C(O)NR⁶R⁷, -O-(C₀-C₁₀-Alkylen)-C(O)NR⁶NR⁷C(O)OR⁶, -O-(C₁-C₁₀-Alkylen)-C(O)(aryl)-N-N=N⁻, -OC(O)-(C₁-C₁₀-Alkylen)-C(O)OR⁶, -(C₀-C₁₀-Alkylen)-C(O)NR⁶R⁷, -(C₀-C₁₀-Alkylen)-OC(O)NR⁶R⁷, -NO₂, -(C₀-C₁₀-Alkylen)-NR⁶R⁷, -O-(C₂-C₁₀-Alkylen)-NR⁶R⁷, -NR⁶C(O)R⁷, -NR⁶C(O)OR⁹, -NR⁶C(O)NR⁷R⁸, -NR⁶S(O)₀₋₂R⁹, -N(S(O)₀₋₂R⁹)₂, -CHNOR⁶, -C(O)NR⁶R⁷, -C(O)NR⁶NR⁶R⁷, -S(O)₀₋₂NR⁶R⁷, -S(O)₀₋₂R⁹, -O-C(O)-(C₁-C₁₀-Alkylen)-C(O)NR⁶R⁷, -OC(O)-(C₁-C₁₀-Alkylen)-NR⁶C(O)O-(alkylaryl), -P(O)(OR¹⁰)₂, -(C₁-C₁₀-Alkylen)-OSi(alkyl)₃, -CF₃, -OCF₃, Halogen, Alkoxyalkoxy, Alkoxyalkoxyalkoxy, Alkoxycarbonylalkoxy, Alkoxyarylalkoxy, Alkoxyiminoalkyl, Alkyldioyl, Allyloxy, Aryl, Arylalkyl, Aryloxy, Arylalkoxy, Aroyl, Aroyloxy, Aroylaroyloxy, Arylalkoxycarbonyl, Benzoylbenzoyloxy, Heteroaryl, Heteroarylalkyl, Heteroarylalkoxy, Dioxolanyl, Heterocyclyl, Heterocyclylalkyl, Heterocyclylcarbonyl, Heterocyclylcarbonylalkoxy und -L-M,
wobei gegebenenfalls ein oder mehrere Kohlenstoffatome des -(C₀-C₃₀-Alkylen)-Rests von Q¹, Q², Q³, Q⁴ und Q⁵ unabhängig durch -O-, -C(O)-, -CH=CH-, -C≡C-, -N(Alkyl)-, -N(Alkylaryl)- oder -NH- ersetzt sind,
G ausgewählt ist aus der Gruppe, bestehend aus einem Zuckerrest, Zweifachzuckerrest, Dreifachzuckerrest, Vierfachzuckerrest, einer Zuckersäure, einem Aminozucker, Aminosäurerest, Oligopeptidrest mit 2 bis 9 Aminosäuren, Trialkylammoniumalkylrest und -S(O)₂-OH, wobei gegebenenfalls der Zuckerrest, Zweifachzuckerrest, Dreifachzuckerrest, Vierfachzuckerrest, die Zuckersäure, der Aminozucker, Aminosäurerest oder Oligopeptidrest von G mit -L-M substituiert ist,
L ausgewählt ist aus der Gruppe, bestehend aus 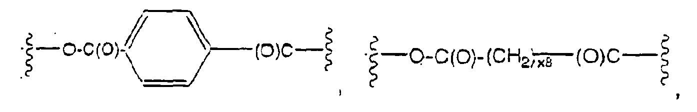 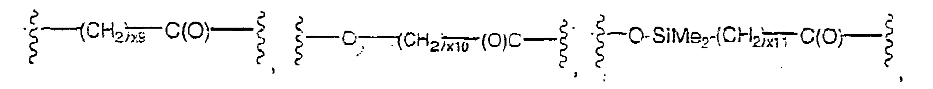 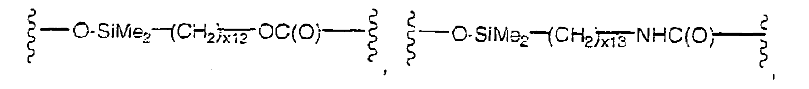 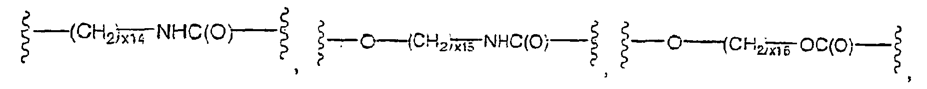 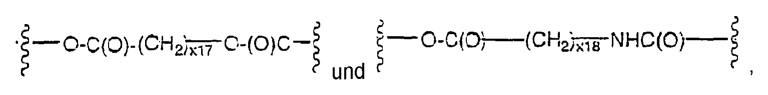 wobei Me Methyl ist,
M ausgewählt ist aus der Gruppe von Resten, bestehend aus 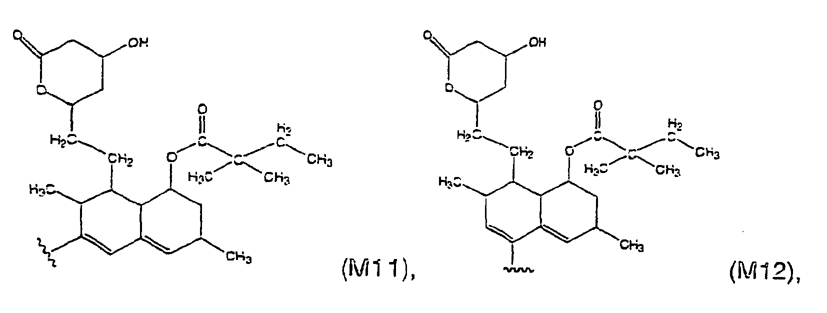 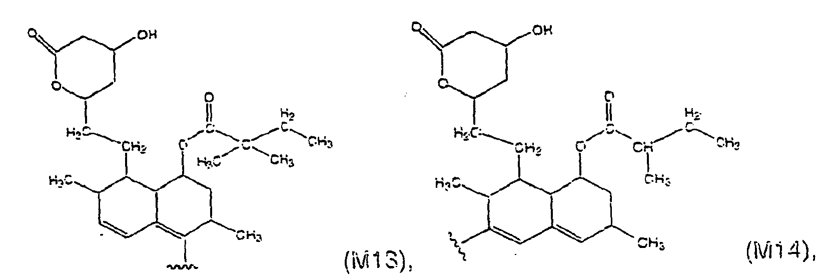 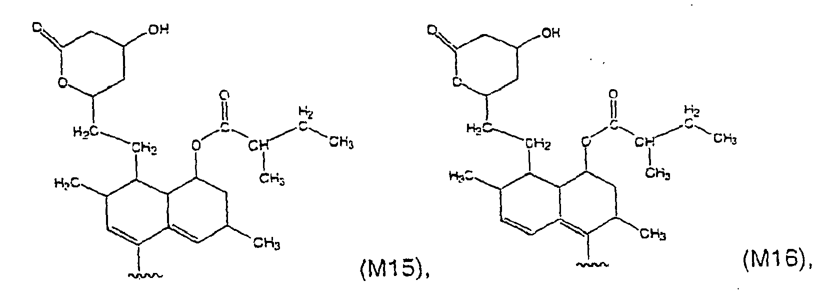 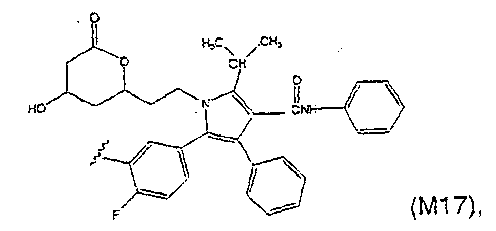 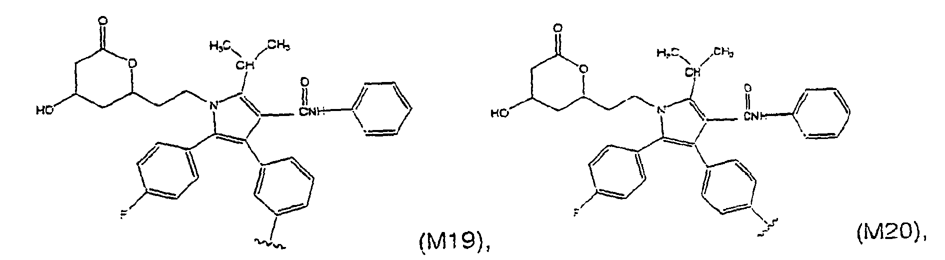 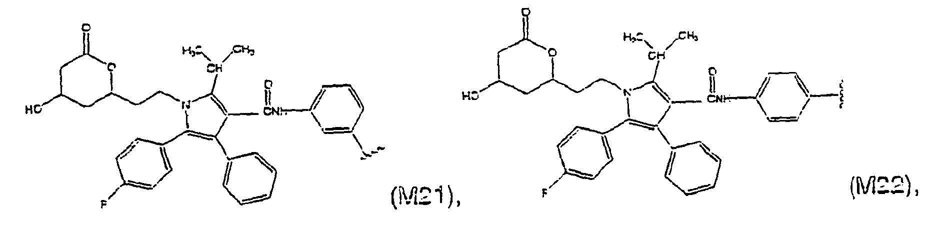 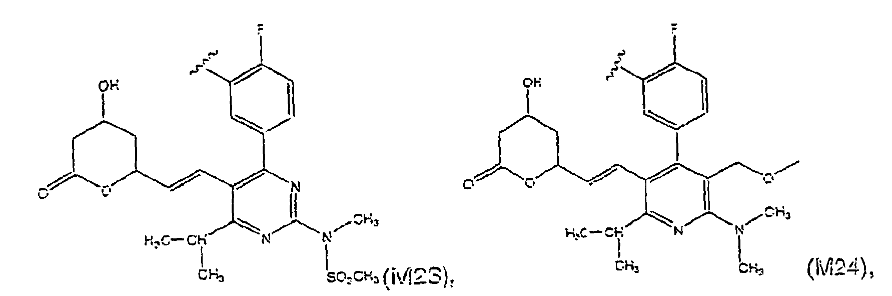 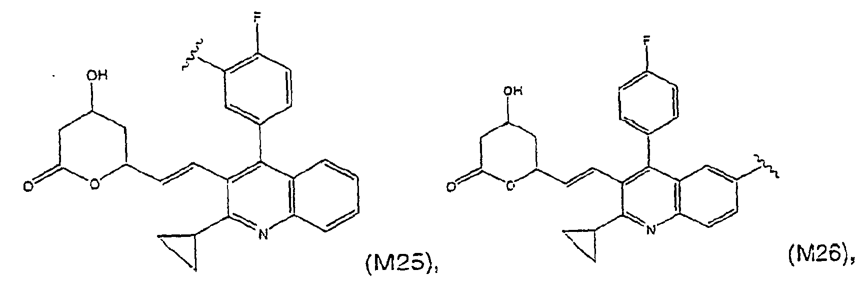 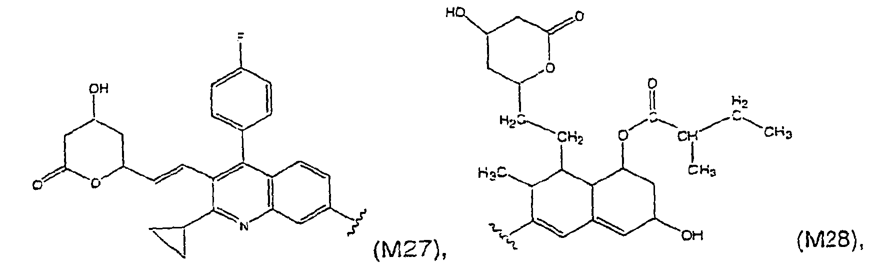 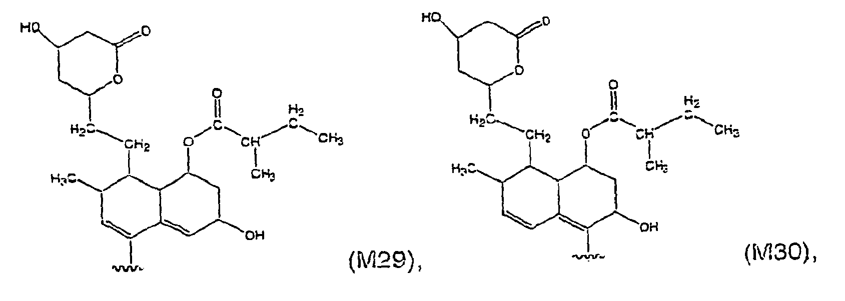 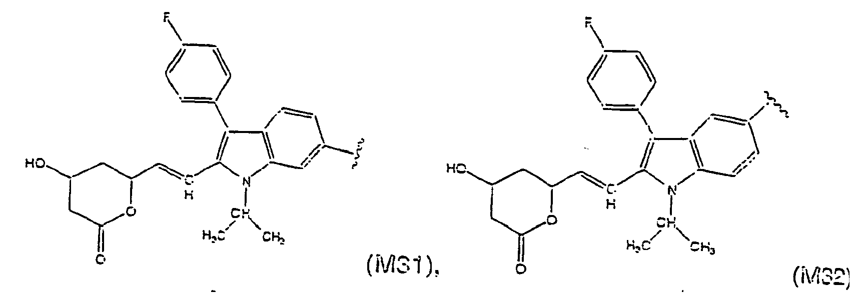 und 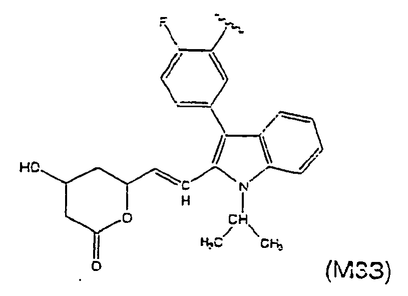 und pharmazeutisch annehmbaren Salzen der Reste (M1) bis (M33),
R² und R³ gleich oder verschieden sein können und jedes unabhängig ausgewählt ist aus der Gruppe, bestehend aus Wasserstoff, Alkyl und Aryl,
R⁶, R⁷ und R⁸ gleich oder verschieden sein können und jedes unabhängig ausgewählt ist aus der Gruppe, bestehend aus Wasserstoff, Alkyl, Aryl und Arylalkyl, und
jedes R⁹ unabhängig Alkyl, Aryl oder Arylalkyl ist,
jedes R¹⁰ unabhängig H oder Alkyl ist,
q 0 oder 1 ist,
r 0 oder 1 ist,
m, n und p unabhängig ausgewählt sind aus 0, 1, 2, 3 oder 4, mit der Maßgabe, dass wenigstens eines von q und r 1 ist und die Summe aus m, n, p, q und r 1, 2, 3, 4, 5 oder 6 ist, und mit der Maßgabe, dass, wenn p 0 ist und r 1 ist, die Summe aus m, q und n 1, 2, 3, 4 oder 5 ist,
x8 1 bis 10 ist,
x9 1 bis 10 ist,
x10 1 bis 10 ist,
x11 1 bis 10 ist,
x12 1 bis 10 ist,
x13 1 bis 10 ist,
x14 1 bis 10 ist,
x15 1 bis 10 ist und
x16 1 bis 10 ist,
x17 1 bis 10 ist und
x18 1 bis 10 ist,
mit der Maßgabe, dass wenigstens eines von Q¹, Q², Q³, Q⁴ und Q⁵ -L-M ist oder der Zuckerrest, Zweifachzuckerrest, Dreifachzuckerrest, Vierfachzuckerrest, die Zuckersäure, der Aminozucker, Aminosäurerest oder Oligopeptidrest von G mit -L-M substituiert ist,
wobei die folgenden Definitionen gelten:
Die Bezeichnung "Alkyl" bedeutet eine aliphatische Kohlenwasserstoffgruppe, die gerade oder verzweigt sein kann und 1 bis etwa 20 Kohlenstoffatome in der Kette umfasst, gegebenenfalls substituiert durch einen oder mehrere Substituenten, unabhängig ausgewählt aus der Gruppe, bestehend aus Halogen, Aryl, Cycloalkyl, Cyano, Hydroxy, Alkoxy, Alkylthio, Amino, -NH(Alkyl), -NH(Cycloalkyl), -N(Alkyl)₂ (wobei die Alkyle gleich oder verschieden sein können), Carboxy und -C(O)O-Alkyl. Nichtlimitierende Beispiele für geeignete Alkylgruppen sind u.a. Methyl, Ethyl, n-Propyl, Isopropyl, n-Butyl, t-Butyl, n-Pentyl, Heptyl, Nonyl, Decyl, Fluormethyl, Trifluormethyl und Cyclopropylmethyl,
"Alkenyl" bedeutet eine aliphatische Kohlenwasserstoffgruppe (gerade oder verzweigte Kohlenstoffkette), die eine oder mehrere Doppelbindungen in der Kette umfasst, die konjugiert oder nicht konjugiert sein können, gegebenenfalls substituiert durch einen oder mehrere Substituenten, unabhängig ausgewählt aus der Gruppe, bestehend aus Halogen, Alkyl, Aryl, Cycloalkyl, Cyano und Alkoxy,
"Alkoxy" bedeutet eine Alkyl-O-Gruppe, wobei die Alkylgruppe wie zuvor beschrieben ist,
"Alkoxyarylalkoxy" bedeutet eine Alkyl-O-arylalkylen-O-Gruppe, wobei die Alkyl-, Alkylenund Arylgruppen wie zuvor beschrieben sind,
"Alkoxycarbonylalkoxy" bedeutet eine Alkyl-O-C(O)-alkylen-O-Gruppe, wobei die Alkyl-, und Alkylengruppen wie zuvor beschrieben sind,
"Alkoxyiminoalkyl" bedeutet eine Alkyl-O-N=CH-alkylengruppe, wobei die Alkyl- und Alkylengruppen wie zuvor beschrieben sind,
"Alkyldioyl" bedeutet eine ROC(O)-Alkylen-C(O)-O-Gruppe, wobei R Alkyl oder H ist und die Alkylengruppe wie zuvor beschrieben ist,
"Alkinyl" bedeutet eine aliphatische Kohlenwasserstoffgruppe, die wenigstens eine Kohlenstoff-Kohlenstoff-Dreifachbindung umfasst und die gerade oder verzweigt sein kann und etwa 2 bis etwa 15 Kohlenstoffatome in der Kette umfasst, gegebenenfalls substituiert durch einen oder mehrere Substituenten, die gleich oder verschieden sein können, wobei jeder Substituent unabhängig ausgewählt ist aus der Gruppe, bestehend aus Alkyl, Aryl und Cycloalkyl,
"Allyloxy" bedeutet H₂C=CH-O-,
"Aryl" bedeutet ein aromatisches monocyclisches oder multicyclisches Ringsystem, das etwa 5 bis etwa 14 Kohlenstoffatome umfasst, gegebenenfalls substituiert mit einem oder mehreren "Ringsystemsubstituenten", die gleich oder verschieden sein können und wie nachstehend definiert sind,
"Aralkyl" oder "Arylalkyl" bedeutet eine Arylalkylengruppe, wobei das Aryl und das Alkylen wie zuvor beschrieben sind,
"Aryloxy" bedeutet eine Aryl-O-Gruppe, wobei die Arylgruppe wie zuvor beschrieben ist, "Aralkoxy" oder "Arylalkyloxy" bedeutet eine Aralkyl-O-Gruppe, wobei die Aralkylgruppe wie zuvor beschrieben ist,
"Aroyl bedeutet eine Aryl-C(O)-Gruppe, wobei die Arylgruppe wie zuvor beschrieben ist,
"Aroyloxy" bedeutet eine Aroyl-O-Gruppe, wobei die Aroylgruppe wie zuvor beschrieben ist,
"Cycloalkyl" bedeutet ein nichtaromatisches mono- oder multicyclisches Ringsystem, das etwa 3 bis etwa 10 Kohlenstoffatome umfasst, gegebenenfalls substituiert mit einem oder mehreren "Ringsystemsubstituenten", die gleich oder verschieden sein können und wie nachstehend definiert sind,
"Cycloalkylen bedeutet einen korrespondierenden bivalenten Ring, wobei die Verknüpfungspunkte zu anderen Gruppen alle Stellungsisomere umfassen,
"Dioxolanyl" bedeutet 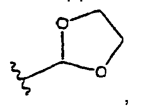
"Halogen" bedeutet einen Fluor-, Chlor-, Brom- oder Iodrest,
"Heteroaryl" bedeutet ein monocyclisches oder multicyclisches aromatisches Ringsystem aus etwa 5 bis etwa 14 Ringatomen, wobei eines oder mehrere der Atome im Ringsystem anders als Kohlenstoff ist/sind und die/das Heteroatom(e) eine carbocyclische Ringstruktur unterbricht/unterbrechen und eine ausreichende Anzahl von delokalisierten pi-Elektronen besitzt/besitzen, um aromatischen Charakter zu verleihen, mit der Maßgabe, dass die Ringe keine benachbarten Sauerstoff- und/oder Schwefelatome enthalten, wobei die Heteroarylgruppe gegebenenfalls substituiert ist durch einen oder mehrere "Ringsystemsubstituenten", die gleich oder verschieden sein können und wie nachstehend definiert sind, die Vorsilbe Aza, Oxa oder Thia vor dem Namen des Heteroaryl-Stamms bedeutet, dass wenigstens ein Stickstoff-, Sauerstoff- bzw. Schwefelatom als ein Ringatom vorliegt, ein Stickstoffatom eines Heteroaryls oxidiert sein kann unter Bildung des entsprechenden N-Oxids,
"Heteroarylalkyl" oder "Heteroaralkyl" bedeutet eine Heteroarylalkylengruppe, wobei das Heteroaryl und das Alkyl wie zuvor beschrieben sind,
"Heteroarylalkoxy" bedeutet eine Heteroarylalkylen-O-Gruppe, wobei das Heteroaryl und das Alkylen wie zuvor beschrieben sind,
"Heterocyclyl" bedeutet ein nichtaromatisches gesättigtes monocyclisches oder multicyclisches Ringsystem, das etwa 3 bis etwa 10 Ringatome umfasst, wobei eines oder mehrere der Atome in dem Ringsystem ein Element anders als Kohlenstoff ist, mit der Maßgabe, dass keine benachbarten Sauerstoff- und/oder Schwefelatome im Ringsystem vorliegen, wobei die Vorsilbe Aza, Oxa oder Thia vor dem Namen des Heterocyclyl-Stamms bedeutet, dass wenigstens ein Stickstoff-, Sauerstoff- bzw. Schwefelatom als ein Ringatom vorliegt, das Heterocyclyl gegebenenfalls substituiert ist durch einen oder mehrere "Ringsystemsubstituenten", die gleich oder verschieden sind und wie nachstehend definiert sind, das Stickstoff- oder Schwefelatom des Heterocyclyls gegebenenfalls zum entsprechenden N-Oxid, S-Oxid oder S,S-Dioxid oxidiert sein kann,
"Heterocyclylalkyl" bedeutet eine Heterocyclylalkylengruppe, wobei die Heterocyclyl- und Alkylengruppen wie zuvor beschrieben sind,
"Heterocyclylcarbonyl" bedeutet eine Heterocyclyl-C(O)-Gruppe, wobei das Heterocyclyl wie zuvor beschrieben ist,
"Heterocyclylcarbonylalkoxy" bedeutet eine Heterocyclyl-C(O)-alkoxygruppe, wobei das Heterocyclyl und das Alkoxy wie zuvor beschrieben sind,
"Ringsystemsubstituent" bedeutet einen Substituenten, der an ein aromatisches oder nichtaromatisches Ringsystem gebunden ist und zum Beispiel ein zur Verfügung stehendes Wasserstoff am Ringsystem ersetzen kann, wobei die Ringsystemsubstituenten gleich oder verschieden sind und jeder unabhängig ausgewählt ist aus der Gruppe, bestehend aus Aryl, Heteroaryl, Aralkyl, Alkylaryl, Aralkenyl, Heteroaralkyl, Alkylheteroaryl, Heteroaralkenyl, Hydroxy, Hydroxyalkyl, Alkoxy, Aryloxy, Aralkoxy, Acyl, Aroyl, Halogen, Nitro, Cyano, Carboxy, Alkoxycarbonyl, Aryloxycarbonyl, Aralkoxycarbonyl, Alkylsulfonyl, Arylsulfonyl, Heteroarylsulfonyl, Alkylsulfinyl, Arylsulfinyl, Heteroarylsulfinyl, Alkylthio, Arylthio, Heteroarylthio, Aralkylthio, Heteroaralkylthio, Cycloalkyl, Cycloalkenyl, Heterocyclyl, Heterocyclenyl, Y₁Y₂N-, Y₁Y₂N-Alkyl-, Y₁Y₂NC(O)- und Y₁Y₂NSO₂-, wobei Y₁ und Y₂ gleich oder verschieden sein können und unabhängig ausgewählt sind aus der Gruppe, bestehend aus Wasserstoff, Alkyl, Aryl und Aralkyl,
"Zuckerrest bedeutet einen Rest, der von einer Aldose oder Ketose stammt, die 3 bis 7 Kohlenstoffatome besitzt und zur D- oder L-Reihe gehören kann,
"Zweifachzuckerrest" bedeutet einen Rest, der von einem Zucker stammt, der zu zwei Monosaccharidmolekülen hydrolysiert werden kann,
"Dreifachzuckerrest" bedeutet einen Rest, der von einem Zucker stammt, der zu drei Monosaccharidmolekülen hydrolysiert werden kann,
"Vierfachzuckerrest" bedeutet einen Rest, der von einem Zucker stammt, der zu vier Monosaccharidmolekülen hydrolysiert werden kann,
"Zuckersäure" bedeutet einen Zuckerrest, wie er zum Beispiel aus Glucuronsäure, Galacturonsäure, Gluconsäure, Galactonsäure, Mannonsäure, Glucarsäure und Galactarsäure gebildet werden kann,
"Aminozucker" bedeutet einen aminosubstituierten Zuckerrest, wie er zum Beispiel aus Glucosamin, Galactosamin, Glucamin oder 3-Amino-1,2-propandiol gebildet werden kann, und
"Aminosäurerest" bedeutet einen Rest, der von einer Aminosäure stammt. Der Aminosäurerest kann aus der D- oder L-Form der Aminosäure hergestellt werden.

2. Die Verbindung gemäß Anspruch 1, wobei m, n und r jeweils null sind, q 1 ist, p 2 ist und Z -CH₂- ist.

3. Die Verbindung gemäß Anspruch 1, wobei m, n und r jeweils null sind, q 1 ist, p 2 ist und Z -CH₂- ist, Q¹ ist -OR⁶, wobei R⁶ Wasserstoff ist, und Q⁵ ist Fluor.

4. Die Verbindung gemäß Anspruch 1, wobei R² und R³ jeweils vorzugsweise Wasserstoff sind.

5. Die Verbindung gemäß Anspruch 1, wobei Q¹ und Q² jeweils unabhängig ausgewählt sind aus der Gruppe, bestehend aus -OR⁶, -O(CO)R⁶, -O(CO)OR⁹ und -O(CO)NR⁶R⁷.

6. Die Verbindung gemäß Anspruch 1, wobei Q⁴ Halogen oder -OR⁶ ist.

7. Die Verbindung gemäß Anspruch 1, wobei Q¹ -OR⁶ ist, wobei R⁶ H ist.

8. Die Verbindung gemäß Anspruch 1, wobei Q¹, Q², Q³, Q⁴ oder Q⁵ -L-M ist.

9. Die Verbindung gemäß Anspruch 1, wobei Q¹, Q², Q³, Q⁴ oder Q⁵ -(C₀-C₃₀-Alkylen)-G ist.

10. Die Verbindung gemäß Anspruch 1, wobei G ausgewählt ist aus der Gruppe, bestehend aus: 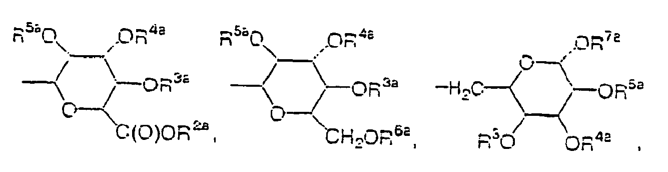 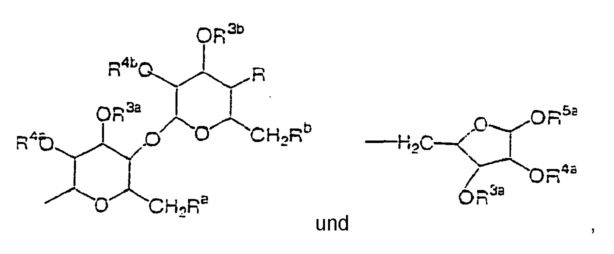
wobei R, R^{a} und R^{b} gleich oder verschieden sein können und jedes unabhängig ausgewählt ist aus der Gruppe, bestehend aus H, -OH, Halogen, -NH₂, Azido, Alkoxyalkoxy oder -W-R³⁰,
W unabhängig ausgewählt ist aus der Gruppe, bestehend aus -NH-C(O)-, -O-C(O)-, -O-C(O)-N(R³¹)-, -NH-C(O)-N(R³¹)- und -O-C(S)-N(R³¹)-,
R^{2a} und R^{6a} gleich oder verschieden sein können und jedes unabhängig ausgewählt ist aus der Gruppe, bestehend aus H, Alkyl, Acetyl, Aryl und Arylalkyl,
R^{3a}, R^{4a}, R^{5a}, R^{7a}, R^{3b} und R^{4b} gleich oder verschieden sein können und jedes unabhängig ausgewählt ist aus der Gruppe, bestehend aus H, Alkyl, Acetyl, Arylalkyl, -C(O)-Alkyl und -C(O)Aryl,
R³⁰ unabhängig ausgewählt ist aus der Gruppe, bestehend aus R³²-substituertem T, R³²-substituiertem T-Alkyl, R³²-substituiertem Alkenyl, R³²-substituiertem Alkyl, R³²-substituiertem Cycloalkyl und R³²-substituiertem Cycloalkylalkyl,
R³¹ unabhängig ausgewählt ist aus der Gruppe, bestehend aus H und Alkyl,
T unabhängig ausgewählt ist aus der Gruppe, bestehend aus Phenyl, Furyl, Thienyl, Pyrrolyl, Oxazolyl, Isoxazolyl, Thiazolyl, Isothiazolyl, Benzothiazolyl, Thiadiazolyl, Pyrazolyl, Imidazolyl und Pyridyl,
R³² 1 bis 3 Substituenten ist, die jeweils unabhängig ausgewählt sind aus der Gruppe, bestehend aus H, Halogen, Alkyl, -OH, Phenoxy, -CF₃, -NO₂, Alkoxy, Methylendioxy, Oxo, Alkylsulfanyl, Alkylsulfinyl, Alkylsulfonyl, -N(CH₃)₂, -C(O)-NH-Alkyl, -C(O)-N(Alkyl)₂, -C(O)-Alkyl, -C(O)-Alkoxy und Pyrrolidinylcarbonyl, oder R³² eine kovalente Bindung ist und R³¹, das Stickstoffatom, an das es gebunden ist, und R³² eine Pyrrolidinyl-, Piperidinyl-, N-Methylpiperazinyl-, Indolinyl- oder Morpholinylgruppe oder eine mit Alkoxycarbonyl substituierte Pyrrolidinyl-, Piperidinyl-, N-Methylpiperazinyl-, Indolinyl- oder Morpholinylgruppe bilden.

11. Die Verbindung gemäß Anspruch 10, wobei G ausgewählt ist aus: 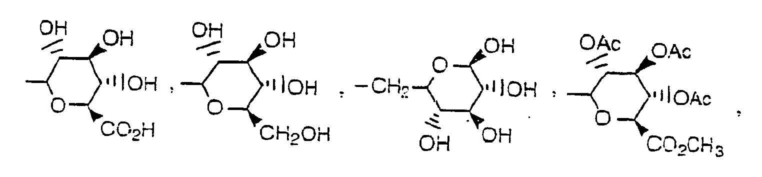 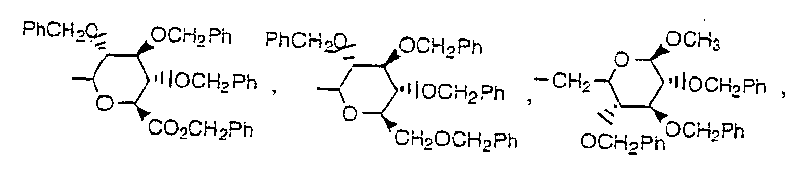 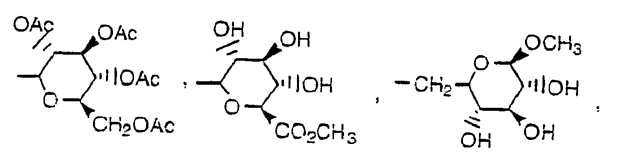 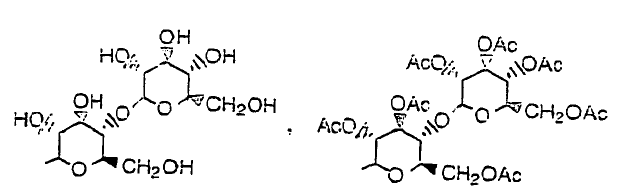 und 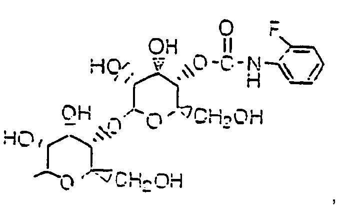 wobei Ac Acetyl ist und Ph Phenyl ist.

12. Die Verbindung gemäß Anspruch 1, wobei gegebenenfalls ein oder mehrere Kohlenstoffatome des -(C₀-C₃₀-Alkylen)-Rests von Q¹, Q², Q³, Q⁴ und Q⁵ unabhängig durch -O-ersetzt sind.

13. Eine pharmazeutische Zusammensetzung zur Verwendung bei der Behandlung oder Prävention von einem vaskulären Zustand, Diabetes, Adipositas, Apoplexie, zur Verringerung einer Konzentration eines Sterols oder Stanols im Plasma eines Säugers, zur Prävention von Demyelinisierung oder zur Behandlung von Alzheimer-Krankheit und/oder zur Steuerung der Amyloid-β-Peptid-Spiegel bei einem Subjekt, umfassend eine therapeutisch wirksame Menge einer Verbindung nach Anspruch 1 in einem pharmazeutisch annehmbaren Träger.

14. Eine pharmazeutische Zusammensetzung, umfassend eine zur Cholesterinsenkung wirksame Menge einer Verbindung nach Anspruch 1 in einem pharmazeutisch annehmbaren Träger.

15. Verwendung einer wie in Anspruch 1 definierten Verbindung zur Herstellung eines Medikaments zur Verwendung bei der Behandlung oder Prävention von einem vaskulären Zustand, Diabetes, Adipositas, Apoplexie, zur Verringerung einer Konzentration eines Sterols oder Stanols im Plasma eines Säugers, zur Prävention von Demyelinisierung oder zur Behandlung von Alzheimer-Krankheit oder zur Steuerung eines Amyloid-β-Peptid-Spiegels bei einem Subjekt.

16. Verwendung einer wie in Anspruch 1 definierten Verbindung zur Herstellung eines Medikaments zur Verwendung bei der Senkung des Cholesterinspiegels im Plasma eines Säugers, der eine solche Behandlung benötigt.

## Revendications

1. Composé représenté par la formule développée (IA): 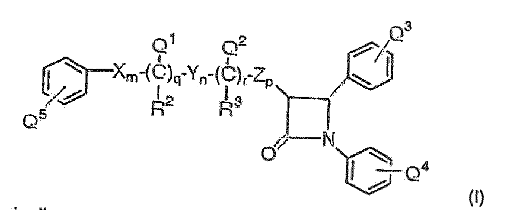 ou des énantiomères, stéréoisomères, rotamères, tautomères, racémates, sels, solvates ou esters pharmaceutiquement acceptables du composé de la Formule (IA),
où dans la Formule (IA) ci-dessus:
X, Y et Z peuvent être les mêmes ou différents et chacun est sélectionné indépendamment parmi le groupe consistant en -CH₂-, -CH(alkyle)- et -C(alkyle)₂-;
Q¹ et Q² peuvent être les mêmes ou différents et chacun est sélectionné indépendamment parmi le groupe consistant en: H, -(alkylène C₀-C₃₀)-G, -OR⁶,-OC(O)R⁶, -OC(O)OR⁹, -OC(O)NR⁶R⁷ et -L-M;
Q³ est 1 à 5 substituants sélectionnés indépendamment parmi le groupe consistant en alkyle, alcényle, alcynyle, -(alkylène C₀-C₃₀)-G, -(alkylène C₀-C₁₀)-OR⁶, -(alkylène C₀-C₁₀)-C(O)R⁶, -(alkylène C₀-C₁₀)-C(O)OR⁶, -(alkylène C₀-C₁₀)-OC(O)R⁶, -(alkylène C₀-C₁₀)-OC(O)OR⁹, CH=CH-C(O)R⁶, CH=CH-C(O)OR⁶, -C=C-C(O)OR⁶, -C=C-C(O)R⁶, -O-(alkylène C₁-C₁₀)-OR⁶, -O-(alkylène C₁-C₁₀)-C(O)R⁶, -O-(alkylène C₁-C₁₀)-C(O)OR⁶, -CN, -O-(alkylène C₁-C₁₀)-C(O)NR⁶R⁷, -O-(alkylène C₀-C₁₀)-C(O)NR⁶NR⁷C(O)OR⁶, -O-(alkylène C₁-C₁₀)-C(O)(aryle)-N-N=N-, OC(O)-(alkylène C₁-C₁₀)-C(O)OR⁶, -(alkylène C₀-C₁₀)-C(O)NR⁶R⁷, -(alkylène C₀-C₁₀)-OC(O)NR⁶R⁷, -NO₂, - (alkylène C₀-C₁₀)-NR⁶R⁷, -O-(alkylène C₂-C₁₀)-NR⁶R⁷, -NR⁶C(O)R⁷, -NR⁶C(O)OR⁹, - NR⁶C(O)NR⁷R⁸, -NR⁶S(O)₀₋₂R⁹, -N(S(O)₀₋₂R⁹)₂, -CHNOR⁶, -C(O)NR⁶R⁷, - C(O)NR⁶NR⁶R⁷, -S(O)₀₋₂NR⁶R⁷, -S(O)₀₋₂R⁹, -O-C(O)-(alkylène C₁-C₁₀)-C(O)NR⁶R⁷,-OC(O)-(alkylène C₁-C₁₀)-NR⁶C(O)O-(alkylaryle), -P(O)(OR¹⁰)₂, -(alkylène C₁-C₁₀)-OSi(alkyle)₃, -CF₃, -OCF₃, halo, alcoxyalcoxy, alcoxyalcoxyalcoxy, alcoxycarbonylalcoxy, alcoxyarylalcoxy, alcoxyiminoalkyle, alkyldioyle, allyloxy, aryle, arylalkyle, aryloxy, arylalcoxy, aroyle, aroyloxy, aroylaroyloxy, arylalcoxycarbonyle, benzoylbenzoyloxy, hétéroaryle, hétéroarylalkyle, hétéroarylalcoxy, dioxolanyle, hétérocycyle, hétérocyclylalkyle, hétérocyclylcarbonyle, hétérocyclylcarbonylalcoxy et-L-M;
Q⁴ est 1 à 5 substituants sélectionnés indépendamment parmi le groupe consistant en alkyle, alcényle, alcynyle, -(alkylène C₀-C₃₀)-G, -(alkylène C₀-C₁₀)-OR⁶, -(alkylène C₀-C₁₀)-C(O)R⁶, -(alkylène C₀-C₁₀)-C(O)OR⁶, -(alkylène C₀-C₁₀)-OC(O)R⁶, -(alkylène C₀-C₁₀)-OC(O)OR⁹, -CH=CH-C(O)R⁶, -CH=CH-C(O)OR⁶, -C≡C-C(O)OR⁶, -C≡C-C(O)R⁶, -O-(alkylène C₁-C₁₀)-OR⁶, -O-(alkylène C₁-C₁₀)-C(O)R⁶, -O-(alkylène C₁-C₁₀)-C(O)OR⁶, -CN, -O-(alkylène C₁-C₁₀)-C(O)NR⁶R⁷, -O-(alkylène C₀-C₁₀)-C(O)NR⁶NR⁷C(O)OR⁶, -O-(alkylène C₁-C₁₀)-C(O)(aryle)-N-N=N⁻, -OC(O)-(alkylène C₁-C₁₀)-C(O)OR⁶, -(alkylène C₀-C₁₀)-C(O)NR⁶R⁷, -(alkylène C₀-C₁₀)-OC(O)NR⁶R⁷, -NO₂,-(alkylène C₀-C₁₀)-NR⁶R⁷, -O-(alkylène C₂-C₁₀)-NR⁶R⁷, -NR⁶C(O)R⁷, -NR⁶C(O)OR⁹,-NR⁶C(O)NR⁷R⁸, -NR⁶S(O)₀₋₂R⁹, -N(S(O)₀₋₂R⁹)₂, -CHNOR⁶, -C(O)NR⁶R⁷,-C(O)NR⁶NR⁶R⁷, -S(O)₀₋₂NR⁶R⁷, -S(O)₀₋₂R⁹, -O-C(O)-(alkylène C₁-C₁₀)-C(O)NR⁶R⁷,-OC(O)-(alkylène C₁-C₁₀)-NR⁶C(O)O-(alkylaryle), -P(O)(OR¹⁰)₂, -(alkylène C₁-C₁₀)-OSi(alkyle)₃, -CF₃, -OCF₃, halo, alcoxyalcoxy, alcoxyalcoxyalcoxy, alcoxycarbonylalcoxy, alcoxyarylalcoxy, alcoxyiminoalkyle, alkyldioyle, allyloxy, aryle, arylalkyle, aryloxy, arylalcoxy, aroyle, aroyloxy, aroylaroyloxy, arylalcoxycarbonyle, benzoylbenzoyloxy, hétéroaryle, hétéroarylalkyle, hétéroarylalcoxy, dioxolanyle, hétérocyclyle, hétérocyclylalkyle, hétérocyclylcarbonyle, hétérocyclylcarbonylalcoxy et-L-M;
Q⁵ est 1 à 5 substituants sélectionnés indépendamment parmi le groupe consistant en alkyle, alcényle, alcynyle, -(alkylène C₀-C₃₀)-G, -(alkylène C₀-C₁₀)-OR⁶, -(alkylène C₀-C₁₀)-C(O)R⁶, -(alkylène C₀-C₁₀)-C(O)OR⁶, -(alkylène C₀-C₁₀)-OC(O)R⁶, -(alkylène C₀-C₁₀)-OC(O)OR⁹, -CH=CH-C(O)R⁶, -CH=CH-C(O)OR⁶, -C≡C-C(O)OR⁶, -C≡C-C(O)R⁶, -O-(alkylène C₁-C₁₀)-OR⁶, -O-(alkylène C₁-C₁₀)-C(O)R⁶, -O-(alkylène C₁-C₁₀)-C(O)OR⁶, -CN, -O-(alkylène C₁-C₁₀)-C(O)NR⁶R⁷, -O-(alkylène C₀-C₁₀)-C(O)NR⁶NR⁷C(O)OR⁶, -O-(alkylène C₁-C₁₀)-C(O)(aryle)-N-N=N⁻, -OC(O)-(alkylène C₁-C₁₀)-C(O)OR⁶, -(alkylène C₀-C₁₀)-C(O)NR⁶R⁷, -(alkylène C₀-C₁₀)-OC(O)NR⁶R⁷, -NO₂,-(alkylène C₀-C₁₀)-NR⁶R⁷, -O-(alkylène C₂-C₁₀)-NR⁶R⁷, -NR⁶C(O)R⁷, -NR⁶C(O)OR⁹,-NR⁶C(O)NR⁷R⁸, -NR⁶S(O)₀₋₂R⁹, -N(S(O)₀₋₂R⁹)₂, -CHNOR⁶, -C(O)NR⁶R⁷,-C(O)NR⁶NR⁶R⁷, -S(O)₀₋₂NR⁶R⁷, -S(O)₀₋₂R⁹, -O-C(O)-(alkylène C₁-C₁₀)-C(O)NR⁶R⁷,-OC(O)-(alkylène C₁-C₁₀)-NR⁶C(O)O-(alkylaryle), -P(O)(OR¹⁰)₂, -(alkylène C₁-C₁₀)-OSi(alkyle)₃, -CF₃, -OCF₃, halo, alcoxyalcoxy, alcoxyalcoxyalcoxy, alcoxycarbonylalcoxy, alcoxyarylalcoxy, alcoxyiminoalkyle, alkyldioyle, allyloxy, aryle, arylalkyle, aryloxy, arylalcoxy, aroyle, aroyloxy, aroylaroyloxy, arylalcoxycarbonyle, benzoylbenzoyloxy, hétéroaryle, hétéroarylalkyle, hétéroarylalcoxy, dioxolanyle, hétérocyclyle, hétérocyclylalkyle, hétérocyclylcarbonyle, hétérocyclylcarbonylalcoxy et-L-M;
où optionnellement un ou plusieurs atomes de carbone du radical -(alkylène C₀-C₃₀)- de Q¹, Q², Q³, Q⁴ et Q⁵ sont remplacés indépendamment par -O-, -C(O)-, -CH=CH-, -C≡C-, -N(alkyle)-, -N(alkylaryle)- ou -NH-;
G est sélectionné parmi le groupe consistant en résidu sucre, résidu disucre, résidu trisucre, résidu tétrasucre, acide sucre, sucre aminé, résidu d'acide aminé, résidu d'oligopeptide comprenant 2 à 9 acides aminés, le radical trialkylammoniumalkyle et-S(O)₂-OH, où optionnellement le résidu sucre, le résidu disucre, le résidu trisucre, le résidu tétrasucre, l'acide sucre, le sucre aminé, le résidu d'acide aminé ou le résidu d'oligopeptide de G est substitué par -L-M;
L est sélectionné parmi le groupe consistant en 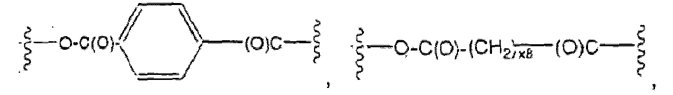 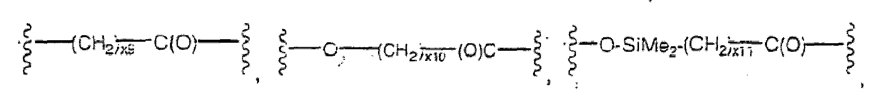 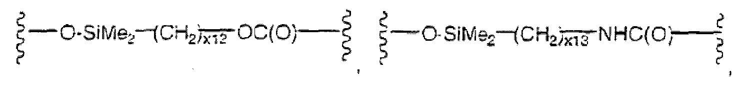 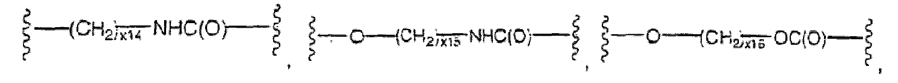 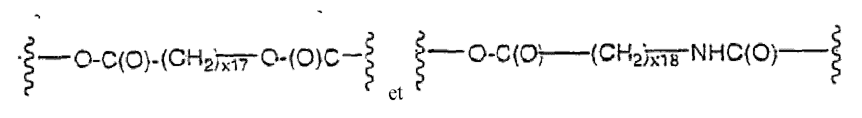
où Me est un méthyle;
M est sélectionné parmi le groupe de fractions consistant en: 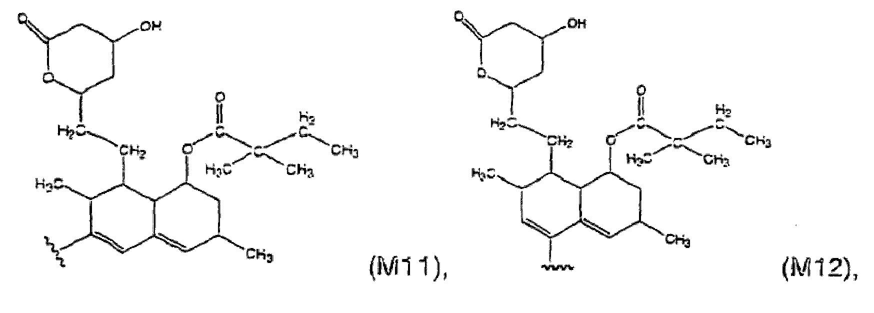 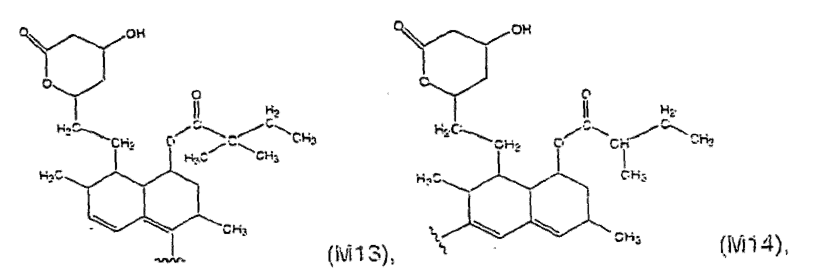 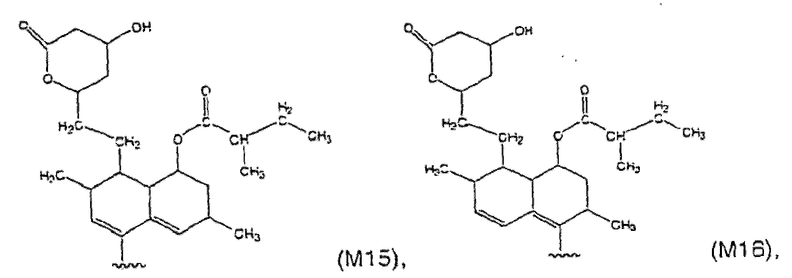 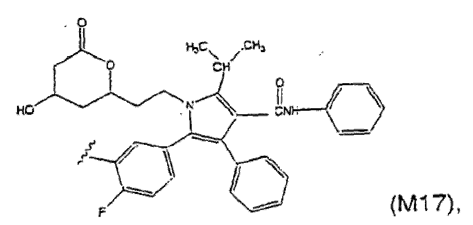 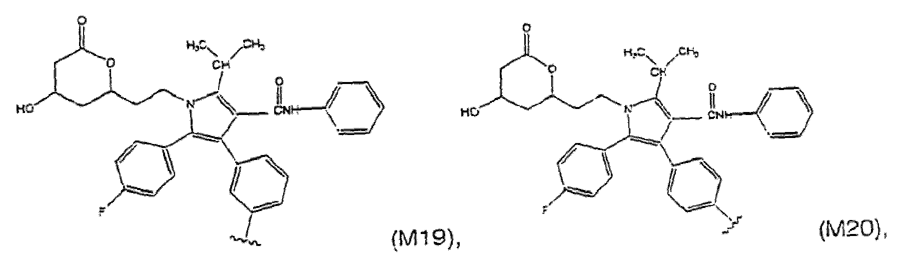 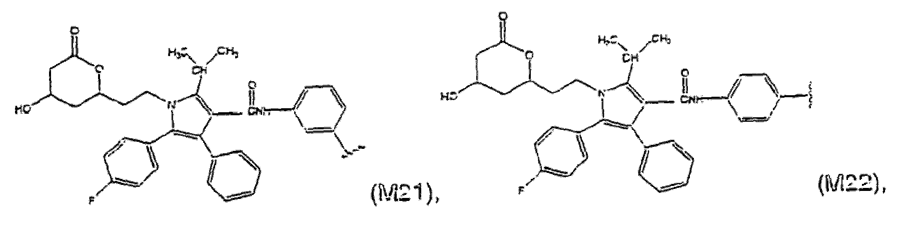 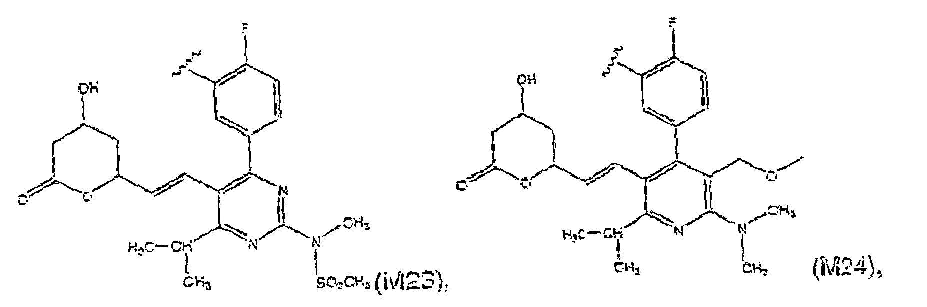 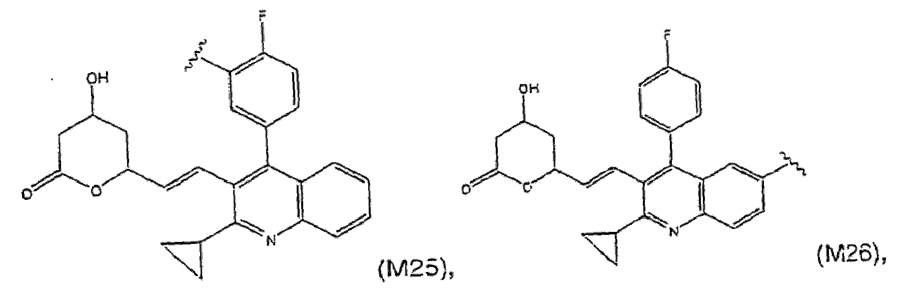 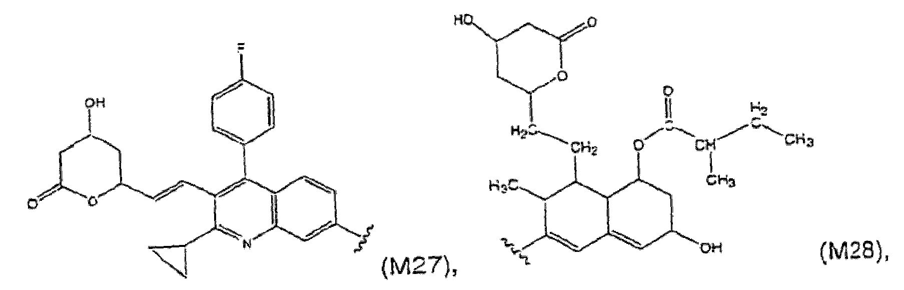 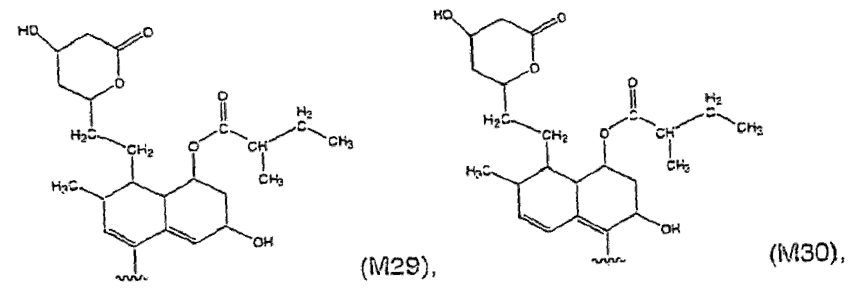 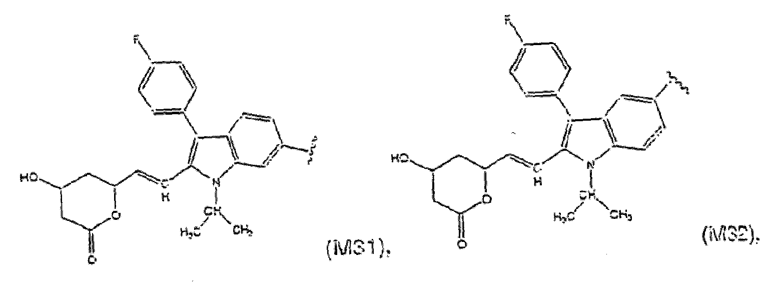 et 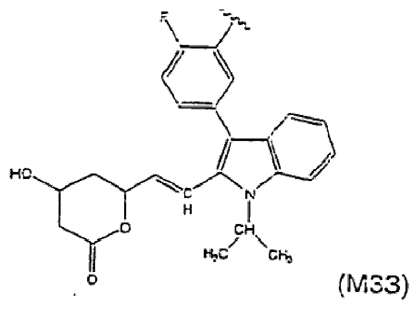 des sels pharmaceutiquement acceptables des fractions (M1) à (M33) ;
R² et R³ peuvent être les mêmes ou différents et chacun est sélectionné indépendamment parmi le groupe consistant en hydrogène, alkyle et aryle;
R⁶ R⁷ et R⁸ peuvent être les mêmes ou différents et chacun est sélectionné indépendamment parmi le groupe consistant en hydrogène, alkyle, aryle et arylalkyle; et
chaque R⁹ est indépendamment un alkyle, aryle ou arylalkyle;
chaque R¹⁰ est indépendamment H ou alkyle;
q est 0 ou 1 ;
r est 0 ou 1 ;
m, n et p sont sélectionnés indépendamment parmi 0, 1, 2, 3 ou 4; à condition qu'au moins l'un de q et de r soit 1 et que la somme de m, n, q et r soit 1, 2, 3, 4, 5 ou 6; et à condition que lorsque p est 0 et que r est 1, la somme de m, q et n soit 1, 2, 3, 4 ou 5;
x8 est 1 à 10;
x9 est 1 à 10;
x10 est 1 à 10;
x11 est 1 à 10;
x12 est 1 à 10;
x13 est 1 à 10;
x14 est 1 à 10;
x15 est 1 à 10 ; et
x16 est 1 à 10;
x17 est 1 à 10 ; et
x18 est 1 à 10;
à condition qu'au moins l'un de Q¹, Q², Q³, Q⁴ et Q⁵ soit -L-M ou que le résidu sucre, le résidu disucre, le résidu trisucre, le résidu tétrasucre, l'acide sucre, le sucre aminé, le résidu d'acide aminé ou le résidu d'oligopeptide de G soit substitué par -L-M;
où les définitions suivantes s'appliquent:
Le terme "alkyle" signifie un groupe d'hydrocarbure aliphatique qui peut être linéaire ou ramifié et comprend 1 à environ 20 atomes de carbone dans la chaîne, optionnellement substitué par un ou plusieurs substituants sélectionnés indépendamment parmi le groupe consistant en halo, aryle, cycloalkyle, cyano, hydroxy, alcoxy, alkylthio, amino, -NH(alkyle), -NH(cycloalkyle), -N(alkyle)₂ (lesquels alkyles peuvent être les mêmes ou différents), carboxy et -C(O)O-alkyle. Des exemples non limitatifs de groupes alkyle qui conviennent englobent méthyle, éthyle, n-propyle, isopropyle, n-butyle, t-butyle, n-pentyle, heptyle, nonyle, décyle, fluorométhyle, trifluorométhyle et cyclopropylméthyle;
"Alcényle" signifie un groupe d'hydrocarbure aliphatique (chaîne carbonée linéaire ou ramifiée) comprenant une ou plusieurs liaisons doubles dans la chaîne et qui peut être conjugué ou non conjugué, optionnellement substitué par un ou plusieurs substituants sélectionnés indépendamment parmi le groupe consistant en halo, alkyle, aryle, cycloalkyle, cyano et alcoxy;
"Alcoxy" signifie un groupe alkyle-O- dans lequel le groupe alkyle est tel que décrit précédemment;
"Alcoxyarylalcoxy" signifie un groupe alkyle-O-aryle-alkylène-O- dans lequel les groupes alkyle, alkylène et aryle sont tels que décrits précédemment;
"Alcoxycarbonylalcoxy" signifie un groupe alkyle-O-(C(O)-alkylène-O- dans lequel les groupes alkyle et alkylène sont tels que décrits précédemment;
"Alcoxyiminoalkyle" signifie un groupe alkyle-O-N=CH-alkylène- dans lequel les groupes alkyle et alkylène sont tels que décrits précédemment;
"Alkyldiol" signifie un groupe ROC-(O)-alkylène-C(O)-O- dans lequel R est alkyle ou H et le groupe alkylène est tel que décrit précédemment;
"Alcynyle" signifie un groupe d'hydrocarbure aliphatique comprenant au moins une liaison triple carbone-carbone et qui peut être linéaire ou ramifié et comprenant d'environ 2 à environ 15 atomes de carbone dans la chaîne, optionnellement substitué par un ou plusieurs substituants qui peuvent être les mêmes ou différents, chaque substituant étant sélectionné indépendamment parmi le groupe consistant en alkyle, aryle et cycloalkyle;
"Allyloxy" signifie H₂C=CH-O-;
"Aryle" signifie un système de cycle aromatique monocyclique ou multicyclique comprenant d'environ 5 à environ 14 atomes de carbone, optionnellement substitué par un ou plusieurs "substituants de système cyclique" qui peuvent être les mêmes ou différents et qui sont définis ci-dessous;
"Arylène" signifie un groupe phényle bivalent, comprenant des substitutions en ortho, méta et para;
"Aralkyle" ou "arylalkyle" signifie un groupe aryle-alkylène dans lequel l'aryle et falkylène sont tels que décrits précédemment;
"Aryloxy" signifie un groupe -aryle-O- où le groupe aryle est tel que décrit précédemment;
"Aralcoxy" ou "arylalkyloxy" signifie un groupe aralkyle-O-, où le groupe aralkyle est tel que décrit précédemment;
"Aroyle" signifie un groupe aryle-C(O)-, où le groupe aryle est tel que décrit précédemment;
"Aroyloxy" signifie un groupe aroyle-O-, où le groupe aroyle est tel que décrit précédemment;
"Cycloalkyle" signifie un système de cycle non aromatique monocyclique ou multicyclique comprenant d'environ 3 à environ 10 atomes de carbone, optionnellement substitué par un ou plusieurs "substituants de système cyclique" qui peuvent être les mêmes ou différents et qui sont tels que définis ci-dessous;
"Cycloalkylène" se rapporte à un cycle bivalent correspondant, où les points d'attache à d'autres groupes comprennent tous les isomères positionnels;
"Dioxolanyle" signifie 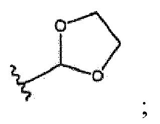
"Halo" se rapporte à des radicaux fluor, chlore, brome et iode;
"Hétéroaryle" signifie un système de cycle aromatique monocyclique ou multicyclique d'environ 5 à environ 14 atomes cycliques, dans lequel un ou plusieurs des atomes dans le système de cycle est/sont des atomes autres que carbone et le/les hétéroatome(s) interrompt/interrompent une structure de cycle carbocyclique et a/ont un nombre suffisant d'électrons pi délocalisés pour donner un caractère aromatique, à condition que les cycles ne contiennent pas d'atomes d'oxygène et/ou de soufre adjacents, le groupe hétéroaryle étant optionnellement substitué par un ou plusieurs "substituants de système cyclique" qui peuvent être les mêmes ou différents et qui sont tels que définis ci-dessous; le préfixe aza, oxa ou thia devant le nom de la racine de l'hétéroaryle signifie qu'au moins un atome d'azote, d'oxygène ou de soufre est respectivement présent comme un atome cyclique; un atome d'azote d'un hétéroaryle peut être oxydé pour former le N-oxyde correspondant;
"Hétéroarylalkyle" ou "hétéroaralkyle" signifie un groupe hétéroaryle-alkylène-dans lequel l'hétéroaryle et l'alkylène sont tels que décrits précédemment;
"Hétéroarylalcoxy" signifie un groupe hétéroaryle-alkylène-O- dans lequel l'hétéroaryle et l'alkylène sont tels que décrits précédemment;
"Hétérocyclyle" signifie un système de cycle non aromatique saturé monocyclique ou multicyclique comprenant d'environ 3 à environ 10 atomes cycliques, dans lequel un ou plusieurs des atomes dans le système de cycle est/sont un élément autre que carbone, à condition qu'il n'y ait pas d'atomes d'oxygène et/ou de soufre adjacents présents dans le système de cycle; le préfixe aza, oxa ou thia devant le nom de la racine de l'hétérocyclyle signifie qu'au moins un atome d'azote, d'oxygène ou de soufre est respectivement présent comme un atome cyclique; l'hétérocycle est optionnellement substitué par un ou plusieurs "substituants de système cyclique" qui peuvent être les mêmes ou différents et qui sont tels que définis ci-dessous; l'atome d'azote ou de soufre de l'hétérocyclyle peut être optionnellement oxydé en les N-oxyde, S-oxyde ou S,S-dioxyde correspondants;
"Hétérocyclylalkyle" signifie un groupe hétérocyclyle-alkylène- dans lequel les groupes hétérocyclyle et alkylène sont tels que décrits précédemment;
"Hétérocyclylcarbonyle" signifie un groupe hétérocyclyle-C(O)- dans lequel l'hétérocyclyle est tel que décrit précédemment;
"Hétérocyclylcarbonylalcoxy" signifie un groupe hétérocyclyle-C(O)-alcoxy- dans lequel l'hétérocyclyle et l'alcoxy sont tels que décrits précédemment;
"Substituant de système de cycle" signifie un substituant attaché à un système de cycle aromatique ou non aromatique qui remplace, par exemple, un hydrogène disponible sur le système de cycle; les substituants de système de cycle peuvent être les mêmes ou différents, chacun étant sélectionné indépendamment parmi le groupe consistant en aryle, hétéroaryle, aralkyle, alkylaryle, aralcényle, hétéroaralkyle, alkylhétéroaryle, hétéroaralcényle, hydroxy, hydroxyalkyle, alcoxy, aryloxy, aralcoxy, acyle, aroyle, halo, nitro, cyano, carboxy, alcoxycarbonyle, aryloxycarbonyle, aralcoxycarbonyle, alkylsulfonyle, arylsulfonyle, hétéroarylsulfonyle, alkylsulfinyle, arylsulfinyle, hétéroarylsulfinyle, alkylthio, arylthio, hétéroarylthio, aralkylthio, hétéroaralkylthio, cycloalkyle, cycloalcényle, hétérocyclyle, hétérocyclényle, Y₁Y₂N-, Y₁Y₂N-alkyle, Y₁Y₂NC(O)- et Y₁Y₂NSO₂-, où Y₁ et Y₂ peuvent être les mêmes ou différents et sont sélectionnés indépendamment parmi le groupe consistant en hydrogène, alkyle, aryle et aralkyle;
"Résidu sucre" signifie une fraction dérivée d'un aldose ou d'un kétose qui a 3 à 7 atomes de carbone et qui peut appartenir à la série D ou L;
"Résidu disucre" signifie une fraction dérivée d'un sucre qui peut être hydrolysée en deux molécules de monosaccharide;
"Résidu trisucre" signifie une fraction dérivée d'un sucre qui peut être hydrolysée en trois molécules de monosaccharide;
"Résidu tétrasucre" signifie une fraction dérivée d'un sucre qui peut être hydrolysée en quatre molécules de monosaccharide;
"Acide sucre" signifie un résidu sucre tel qu'il peut être formé à partir de l'acide glucuronique, de l'acide galacturonique, de l'acide gluconique, de l'acide galactonique, de l'acide mannonique, de l'acide glucarique et de l'acide galactarique;
"Sucre aminé" signifie un résidu sucre amino-substitué qui peut être formé à partir d'une glucosamine, d'une galactosamine, d'une glucamine ou de 3-amino-1,2-propanediol; et
"Résidu d'acide aminé" signifie une fraction dérivée d'un acide aminé. La fraction d'acide aminé peut être préparée à partir des formes D ou L de l'acide aminé.

2. Composé selon la revendication 1, dans lequel m, n et r sont chacun zéro, q est 1, p est 2 et Z est -CH₂-.

3. Composé selon la revendication 1, dans lequel m, n et r sont chacun zéro, q est 1, p est 2 et Z est -CH₂-, Q¹ est -OR⁶; où R⁶ est un hydrogène et Q⁵ est un fluor.

4. Composé selon la revendication 1, dans lequel R² et R³ sont chacun de préférence un hydrogène.

5. Composé selon la revendication 1, dans lequel Q¹ et Q² sont sélectionnés chacun indépendamment parmi le groupe consistant en -OR⁶, -O(CO)R⁶, -O(CO)OR⁹ et-O(CO)NR⁶R⁷.

6. Composé selon la revendication 1, dans lequel Q⁴ est un halo ou -OR⁶.

7. Composé selon la revendication 1, dans lequel Q¹ est -OR⁶ où R⁶ est H.

8. Composé selon la revendication 1, dans lequel Q¹, Q², Q³, Q⁴ ou Q⁵ est -L-M.

9. Composé selon la revendication 1, dans lequel Q¹, Q², Q³, Q⁴ ou Q⁵ est -(alkylène C₀-C₃₀)-G.

10. Composé selon la revendication 1, dans lequel G est sélectionné parmi le groupe consistant en: 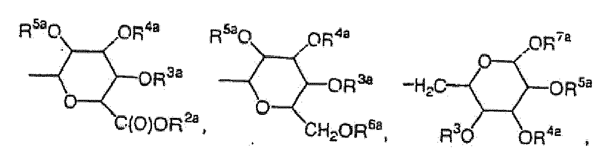 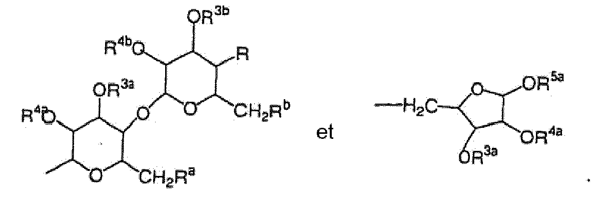
où R, R^{a} et R^{b} peuvent être les mêmes ou différents et chacun est sélectionné indépendamment parmi le groupe consistant en H, -OH, halo, -NH₂, azido, alcoxyalcoxy ou -W-R³⁰ ;
W est sélectionné indépendamment parmi le groupe consistant en -NH-C(O)-, -O-C(O)-, -O-C(O)-N(R³¹)-, -NH-C(O)-N(R³¹)- et -O-C(S)-N(R³¹)-;
R^{2a} et R^{6a} peuvent être les mêmes ou différents et chacun est sélectionné indépendamment parmi le groupe consistant en H, alkyle, acétyle, aryle et arylalkyle;
R^{3a}, R^{4a}, R^{5a}, R^{7a}, R^{3b} et R^{4b} peuvent être les mêmes ou différents et chacun est sélectionné indépendamment parmi le groupe consistant en H, alkyle, acétyle, arylalkyle, -C(O)alkyle et -C(O)aryle;
R³⁰ est sélectionné indépendamment parmi le groupe consistant en T substitué R³², T-alkyle substitué R³², alcényle substitué R³², alkyle substitué R³², cycloalkyle substitué R³² et cycloalkylalkyle substitué R³²;
R³¹ est sélectionné indépendamment parmi le groupe consistant en H et alkyle;
T est sélectionné indépendamment parmi le groupe consistant en phényle, furyle, thiényle, pyrrolyle, oxazolyle, isoxazolyle, thiazolyle, isothiazolyle, benzothiazolyle, thiadiazolyle, pyrazolyle, imidazolyle et pyridyle;
R³² est 1 à 3 substituants qui sont chacun sélectionnés indépendamment parmi le groupe consistant en H, halo, alkyle, -OH, phénoxy, -CF₃, -NO₂, alcoxy, méthylènedioxy, oxo, alkylsulfanyle, alkylsulfinyle, alkylsulfonyle, -N(CH₃)₂, -C(O)-NHalkyle, -C(O)-N(alkyle)₂, -C(O)-alkyle, -C(O)-alcoxy et pyrrolidinylcarbonyle; ou bien R³² est une liaison covalente et R³¹, l'azote auquel il est attaché et R³² forment un groupe pyrrolidinyle, pipéridinyle, N-méthylpipérazinyle, indolinyle ou morpholinyle ou un groupe pyrrolidinyle, pipéridinyle, N-méthylpipérazinyle, indolinyle ou morpholinyle substitué alcoxycarbonyle.

11. Composé selon la revendication 10, dans lequel G est sélectionné parmi: 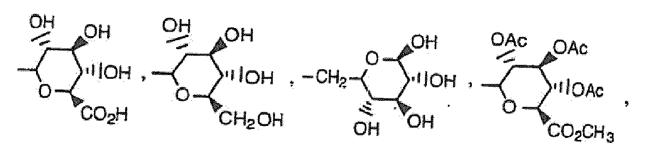 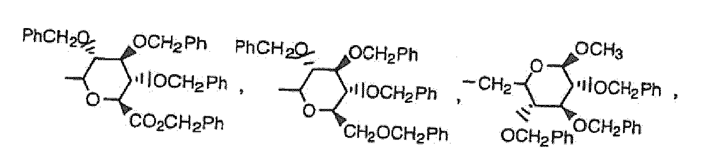 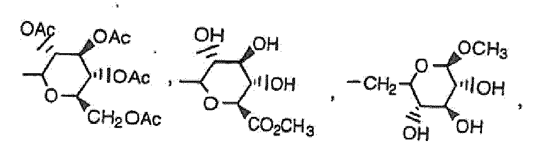 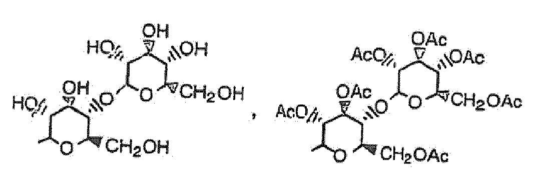 et 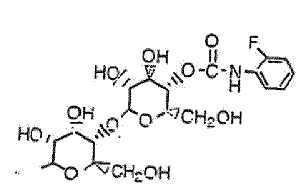 dans lequel Ac est acétyle et Ph est phényle.

12. Composé selon la revendication 1, dans lequel optionnellement un ou plusieurs atomes de carbone du radical -(alkylène C₀-C₃₀) de Q¹, Q², Q³, Q⁴ et Q⁵, sont indépendamment remplacés par -O-.

13. Composition pharmaceutique destinée à être utilisée dans le traitement ou à la prévention d'une condition vasculaire, du diabète, de l'obésité, d'un accident vasculaire cérébral, de l'abaissement d'une concentration d'un stérol ou d'un stanol dans le plasma d'un mammifère, à la prévention de la démyélinisation ou au traitement de la maladie d'Alzheimer et/ou à la régulation des taux de peptides β-amyloïdes chez un sujet, comprenant une quantité thérapeutiquement efficace d'un composé selon la revendication 1 dans un véhicule pharmaceutiquement acceptable.

14. Composition pharmaceutique comprenant un composé selon la revendication 1 en une quantité efficace pour abaisser le cholestérol, dans un véhicule pharmaceutiquement acceptable.

15. Utilisation d'un composé selon la revendication 1, dans la fabrication d'un médicament destiné à être utilisé dans le traitement ou la prévention d'une condition vasculaire, du diabète, de l'obésité, d'un accident vasculaire cérébral, de l'abaissement d'une concentration d'un stérol ou d'un stanol dans le plasma d'un mammifère, dans la prévention de la démyélinisation ou dans le traitement de la maladie d'Alzheimer et/ou dans la régulation d'un taux d'un peptide β-amyloïde chez un sujet.

16. Utilisation d'un composé selon la revendication 1, dans la fabrication d'un médicament destiné à être utilisé pour abaisser le taux de cholestérol dans le plasma d'un mammifère ayant besoin d'un tel traitement.
